(19) Europäisches Patentamt
European Patent Office
Office européen des brevets

(11) **EP 3 067 424 A1**

(12) **EUROPEAN PATENT APPLICATION**

(43) Date of publication:
**14.09.2016 Bulletin 2016/37**

(21) Application number: **16160084.6**

(22) Date of filing: **14.03.2016**

(51) Int Cl.:
***C12N 15/82*** *(2006.01)* ***C12N 15/113*** *(2010.01)*
***C12N 9/12*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.03.2015 US 201562133214 P**

(71) Applicants:
- **Dow AgroSciences LLC**
  **Indianapolis IN 46268-1054 (US)**
- **Fraunhofer-Gesellschaft zur Förderung der angewandten Forschung e.V.**
  **80686 München (DE)**

(72) Inventors:
- **Narva, Kenneth E.**
  **Indianapolis, IN 46268 (US)**
- **Worden, Sarah E.**
  **Indianapolis, IN 46268 (US)**
- **Frey, Meghan L.**
  **Indianapolis, IN 46268 (US)**
- **Rangasamy, Murugesam**
  **Indianapolis, IN 46268 (US)**
- **Gandra, Premchand**
  **Indianapolis, IN 46268 (US)**
- **Veeramani, Balaji**
  **Indianapolis, IN 46268 (US)**
- **Lo, Wendy**
  **Indianapolis, IN 46268 (US)**
- **Vilcinskas, Andreas**
  **35394 Giessen (DE)**
- **Knorr, Eileen**
  **35392 Giessen (DE)**
- **Fishilevich, Elane**
  **Indianapolis, IN 46268 (US)**

(74) Representative: **Zwicker, Jörk**
**ZSP Patentanwälte PartG mbB**
**Radlkoferstrasse 2**
**81373 München (DE)**

# (54) RNA POLYMERASE I1 NUCLEIC ACID MOLECULES TO CONTROL INSECT PESTS

(57) This disclosure concerns nucleic acid molecules and methods of use thereof for control of insect pests through RNA interference-mediated inhibition of target coding and transcribed noncoding sequences in insect pests, including coleopteran pests. In specific examples RNA polymerase I may be selected as the target. The disclosure also concerns methods for making transgenic plants that express nucleic acid molecules useful for the control of insect pests, and the plant cells and plants obtained thereby.

EP 3 067 424 A1

## Description

### PRIORITY CLAIM

This application claims the benefit of the filing date of United States Provisional Patent Application Serial No. 62/133,214, filed March 13, 2015 which is incorporated herein in its entirety.

### TECHNICAL FIELD OF THE DISCLOSURE

The present invention relates generally to genetic control of plant damage caused by insect pests (*e.g.*, coleopteran pests). In particular embodiments, the present invention relates to identification of target coding and non-coding polynucleotides, and the use of recombinant DNA technologies for post-transcriptionally repressing or inhibiting expression of target coding and non-coding polynucleotides in the cells of an insect pest to provide a plant protective effect.

### BACKGROUND

The western corn rootworm (WCR), *Diabrotica virgifera virgifera* LeConte, is one of the most devastating corn rootworm species in North America and is a particular concern in corn-growing areas of the Midwestern United States. The northern corn rootworm (NCR), *Diabrotica barberi* Smith and Lawrence, is a closely-related species that co-inhabits much of the same range as WCR. There are several other related subspecies of *Diabrotica* that are significant pests in the Americas: the Mexican corn rootworm (MCR), *D. virgifera zeae* Krysan and Smith; the southern corn rootworm (SCR), *D. undecimpunctata howardi* Barber; *D. balteata* LeConte; *D. undecimpunctata tenella; D. speciosa* Germar; and *D. u. undecimpunctata* Mannerheim. The United States Department of Agriculture has estimated that corn rootworms cause $1 billion in lost revenue each year, including $800 million in yield loss and $200 million in treatment costs.

Both WCR and NCR are deposited in the soil as eggs during the summer. The insects remain in the egg stage throughout the winter. The eggs are oblong, white, and less than 0.004 inches in length. The larvae hatch in late May or early June, with the precise timing of egg hatching varying from year to year due to temperature differences and location. The newly hatched larvae are white worms that are less than 0.125 inches in length. Once hatched, the larvae begin to feed on corn roots. Corn rootworms go through three larval instars. After feeding for several weeks, the larvae molt into the pupal stage. They pupate in the soil, and then emerge from the soil as adults in July and August. Adult rootworms are about 0.25 inches in length.

Corn rootworm larvae complete development on corn and several other species of grasses. Larvae reared on yellow foxtail emerge later and have a smaller head capsule size as adults than larvae reared on corn. Ellsbury et al. (2005) Environ. Entomol. 34:627-34. WCR adults feed on corn silk, pollen, and kernels on exposed ear tips. If WCR adults emerge before corn reproductive tissues are present, they may feed on leaf tissue, thereby slowing plant growth and occasionally killing the host plant. However, the adults will quickly shift to preferred silks and pollen when they become available. NCR adults also feed on reproductive tissues of the corn plant, but in contrast rarely feed on corn leaves.

Most of the rootworm damage in corn is caused by larval feeding. Newly hatched rootworms initially feed on fine corn root hairs and burrow into root tips. As the larvae grow larger, they feed on and burrow into primary roots. When corn rootworms are abundant, larval feeding often results in the pruning of roots all the way to the base of the corn stalk. Severe root injury interferes with the roots' ability to transport water and nutrients into the plant, reduces plant growth, and results in reduced grain production, thereby often drastically reducing overall yield. Severe root injury also often results in lodging of corn plants, which makes harvest more difficult and further decreases yield. Furthermore, feeding by adults on the corn reproductive tissues can result in pruning of silks at the ear tip. If this "silk clipping" is severe enough during pollen shed, pollination may be disrupted.

Control of corn rootworms may be attempted by crop rotation, chemical insecticides, biopesticides (*e.g.,* the spore-forming gram-positive bacterium, *Bacillus thuringiensis* (*Bt*)), transgenic plants that express *Bt* toxins, or a combination thereof. Crop rotation suffers from the disadvantage of placing unwanted restrictions upon the use of farmland. Moreover, oviposition of some rootworm species may occur in soybean fields, thereby mitigating the effectiveness of crop rotation practiced with corn and soybean.

Chemical insecticides are the most heavily relied upon strategy for achieving corn rootworm control. Chemical insecticide use, though, is an imperfect corn rootworm control strategy; over $1 billion may be lost in the United States each year due to corn rootworm when the costs of the chemical insecticides are added to the costs of the rootworm damage that may occur despite the use of the insecticides. High populations of larvae, heavy rains, and improper application of the insecticide(s) may all result in inadequate corn rootworm control. Furthermore, the continual use of insecticides may select for insecticide-resistant rootworm strains, as well as raise significant environmental concerns due to their toxicity to non-target species.

RNA interference (RNAi) is a process utilizing endogenous cellular pathways, whereby an interfering RNA (iRNA) molecule *(e.g.,* a dsRNA molecule) that is specific for all, or any portion of adequate size, of a target gene results in the degradation of the mRNA encoded thereby. In recent years, RNAi has been used to perform gene "knockdown" in a number of species and experimental systems; for example, *Caenorhabditis elegans,* plants, insect embryos, and cells in tissue culture. *See, e.g.*, Fire et al. (1998) Nature 391:806-11; Martinez et al. (2002) Cell 110:563-74; McManus and Sharp (2002) Nature Rev. Genetics 3:737-47.

RNAi accomplishes degradation of mRNA through an endogenous pathway including the DICER protein complex. DICER cleaves long dsRNA molecules into short fragments of approximately 20 nucleotides, termed small interfering RNA (siRNA). The siRNA is unwound into two single-stranded RNAs: the passenger strand and the guide strand. The passenger strand is degraded, and the guide strand is incorporated into the RNA-induced silencing complex (RISC). Micro ribonucleic acids (miRNAs) are structurally very similar molecules that are cleaved from precursor molecules containing a polynucleotide "loop" connecting the hybridized passenger and guide strands, and they may be similarly incorporated into RISC. Post-transcriptional gene silencing occurs when the guide strand binds specifically to a complementary mRNA molecule and induces cleavage by Argonaute, the catalytic component of the RISC complex. This process is known to spread systemically throughout the organism despite initially limited concentrations of siRNA and/or miRNA in some eukaryotes such as plants, nematodes, and some insects.

Only transcripts complementary to the siRNA and/or miRNA are cleaved and degraded, and thus the knockdown of mRNA expression is sequence-specific. In plants, several functional groups of DICER genes exist. The gene silencing effect of RNAi persists for days and, under experimental conditions, can lead to a decline in abundance of the targeted transcript of 90% or more, with consequent reduction in levels of the corresponding protein. In insects, there are at least two DICER genes, where DICER1 facilitates miRNA-directed degradation by Argonaute1. Lee et al. (2004) Cell 117 (1):69-81. DICER2 facilitates siRNA-directed degradation by Argonaute2.

U.S. Patent 7,612,194 and U.S. Patent Publication Nos. 2007/0050860, 2010/0192265, and 2011/0154545 disclose a library of 9112 expressed sequence tag (EST) sequences isolated from *D. v. virgifera* LeConte pupae. It is suggested in U.S. Patent 7,612,194 and U.S. Patent Publication No. 2007/0050860 to operably link to a promoter a nucleic acid molecule that is complementary to one of several particular partial sequences of *D. v. virgifera* vacuolar-type $H^+$-ATPase (V-ATPase) disclosed therein for the expression of anti-sense RNA in plant cells. U.S. Patent Publication No. 2010/0192265 suggests operably linking a promoter to a nucleic acid molecule that is complementary to a particular partial sequence of a *D. v. virgifera* gene of unknown and undisclosed function (the partial sequence is stated to be 58% identical to C56C10.3 gene product in *C. elegans*) for the expression of anti-sense RNA in plant cells. U.S. Patent Publication No. 2011/0154545 suggests operably linking a promoter to a nucleic acid molecule that is complementary to two particular partial sequences of *D. v. virgifera* coatomer beta subunit genes for the expression of anti-sense RNA in plant cells. Further, U.S. Patent 7,943,819 discloses a library of 906 expressed sequence tag (EST) sequences isolated from *D. v. virgifera* LeConte larvae, pupae, and dissected midguts, and suggests operably linking a promoter to a nucleic acid molecule that is complementary to a particular partial sequence of a *D. v. virgifera* charged multivesicular body protein 4b gene for the expression of double-stranded RNA in plant cells.

No further suggestion is provided in U.S. Patent 7,612,194, and U.S. Patent Publication Nos. 2007/0050860, 2010/0192265, and 2011/0154545 to use any particular sequence of the more than nine thousand sequences listed therein for RNA interference, other than the several particular partial sequences of V-ATPase and the particular partial sequences of genes of unknown function. Furthermore, none of U.S. Patent 7,612,194, and U.S. Patent Publication Nos. 2007/0050860, 2010/0192265, and 2011/0154545 provides any guidance as to which other of the over nine thousand sequences provided would be lethal, or even otherwise useful, in species of corn rootworm when used as dsRNA or siRNA. U.S. Patent 7,943,819 provides no suggestion to use any particular sequence of the more than nine hundred sequences listed therein for RNA interference, other than the particular partial sequence of a charged multivesicular body protein 4b gene. Furthermore, U.S. Patent 7,943,819 provides no guidance as to which other of the over nine hundred sequences provided would be lethal, or even otherwise useful, in species of corn rootworm when used as dsRNA or siRNA. U.S. Patent Application Publication No. U.S. 2013/040173 and PCT Application Publication No. WO 2013/169923 describe the use of a sequence derived from a *Diabrotica virgifera* Snf7 gene for RNA interference in maize. (Also disclosed in Bolognesi et al. (2012) PLoS ONE 7(10): e47534. doi:10.1371/journal.pone.0047534).

The overwhelming majority of sequences complementary to corn rootworm DNAs (such as the foregoing) do not provide a plant protective effect from species of corn rootworm when used as dsRNA or siRNA. For example, Baum et al. (2007) Nature Biotechnology 25:1322-1326, describe the effects of inhibiting several WCR gene targets by RNAi. These authors reported that 8 of the 26 target genes they tested were not able to provide experimentally significant coleopteran pest mortality at a very high iRNA (*e.g.*, dsRNA) concentration of more than 520 ng/$cm^2$.

The authors of U.S. Patent 7,612,194 and U.S. Patent Publication No. 2007/0050860 made the first report of *in planta* RNAi in corn plants targeting the western corn rootworm. Baum et al. (2007) Nat. Biotechnol. 25(11): 1322-6. These authors describe a high-throughput *in vivo* dietary RNAi system to screen potential target genes for developing transgenic RNAi maize. Of an initial gene pool of 290 targets, only 14 exhibited larval control potential. One of the most effective double-stranded RNAs (dsRNA) targeted a gene encoding vacuolar ATPase subunit A (V-ATPase), resulting in a rapid suppression of corresponding endogenous mRNA and triggering a specific RNAi response with low concentrations of dsRNA. Thus, these authors documented for the first time the potential for *in planta* RNAi as a possible pest management tool, while simultaneously demonstrating that effective targets could not be accurately identified *a priori*, even from a relatively small set of candidate genes.

## SUMMARY OF THE DISCLOSURE

Disclosed herein are nucleic acid molecules (*e.g.*, target genes, DNAs, dsRNAs, siRNAs, miRNAs, and hpRNAs), and methods of use thereof, for the control of insect pests, including, for example, coleopteran pests, such as *D. v. virgifera* LeConte (western corn rootworm, "WCR"); *D. barberi* Smith and Lawrence (northern corn rootworm, "NCR"); *D. u. howardi* Barber (southern corn rootworm, "SCR"); *D. v. zeae* Krysan and Smith (Mexican corn rootworm, "MCR"); *D. balteata* LeConte; *D. u. tenella; D. u. undecimpunctata* Mannerheim; and *D. speciosa* Germar. In particular examples, exemplary nucleic acid molecules are disclosed that may be homologous to at least a portion of one or more native nucleic acids in an insect pest.

In these and further examples, the native nucleic acid sequence may be a target gene, the product of which may be, for example and without limitation: involved in a metabolic process or involved in larval development. In some examples, post-transcriptional inhibition of the expression of a target gene by a nucleic acid molecule comprising a polynucleotide homologous thereto may be lethal to an insect pest or result in reduced growth and/or viability of an insect pest. In specific examples, *RNA polymerase I* subunit (referred to herein as, for example, *rpI1*, *rpI1-1*, or *rpI1-2*) may be selected as a target gene for post-transcriptional silencing. In particular examples, a target gene useful for post-transcriptional inhibition is an *rpI1* gene is the gene referred to herein as *Diabrotica rpI1-1 (e.g.,* SEQ ID NO:1) or the gene referred to herein as *Diabrotica rpI1-2 (e.g.,* SEQ ID NO:3). An isolated nucleic acid molecule comprising the polynucleotide of SEQ ID NO: 1; the complement of SEQ ID NO:1; SEQ ID NO:3; the complement of SEQ ID NO:3; and/or fragments of any of the foregoing *(e.g.,* SEQ ID NOs:5-8) is therefore disclosed herein.

Also disclosed are nucleic acid molecules comprising a polynucleotide that encodes a polypeptide that is at least about 85% identical to an amino acid sequence within a target gene product (for example, the product of a *rpI1-1* gene). For example, a nucleic acid molecule may comprise a polynucleotide encoding a polypeptide that is at least 85% identical to SEQ ID NO:2 (*Diabrotica* RPI1-1), or SEQ ID NO:4 (*Diabrotica* RPI1-2), and/or an amino acid sequence within a product of *Diabrotica rpI1-1* or *Diabrotica rpI1-2.* Further disclosed are nucleic acid molecules comprising a polynucleotide that is the reverse complement of a polynucleotide that encodes a polypeptide at least 85% identical to an amino acid sequence within a target gene product.

Also disclosed are cDNA polynucleotides that may be used for the production of iRNA (*e.g.*, dsRNA, siRNA, shRNA, miRNA, and hpRNA) molecules that are complementary to all or part of an insect pest target gene, for example, a *rpI1-1* or *rpI1-2* gene. In particular embodiments, dsRNAs, siRNAs, shRNAs, miRNAs, and/or hpRNAs may be produced *in vitro* or *in vivo* by a genetically-modified organism, such as a plant or bacterium. In particular examples, cDNA molecules are disclosed that may be used to produce iRNA molecules that are complementary to all or part of an *rpI1-1* or *rpI1-2* gene *(e.g.,* SEQ ID NO: 1 and SEQ ID NO:3).

Further disclosed are means for inhibiting expression of an essential gene in a coleopteran pest, and means for providing coleopteran pest protection to a plant. A means for inhibiting expression of an essential gene in a coleopteran pest is a single- or double-stranded RNA molecule consisting of a polynucleotide selected from the group consisting of SEQ ID NOs:77-80; and the complements thereof. Functional equivalents of means for inhibiting expression of an essential gene in a coleopteran pest include single- or double-stranded RNA molecules that are substantially homologous to all or part of a coleopteran *rpI1* gene comprising SEQ ID NO:5, SEQ ID NO:6, SEQ ID NO:7, and/or SEQ ID NO:8. A means for providing coleopteran pest protection to a plant is a DNA molecule comprising a polynucleotide encoding a means for inhibiting expression of an essential gene in a coleopteran pest operably linked to a promoter, wherein the DNA molecule is capable of being integrated into the genome of a plant.

Disclosed are methods for controlling a population of an insect pest *(e.g.,* a coleopteran pest), comprising providing to an insect pest *(e.g.,* a coleopteran pest) an iRNA *(e.g.,* dsRNA, siRNA, shRNA, miRNA, and hpRNA) molecule that functions upon being taken up by the pest to inhibit a biological function within the pest, wherein the iRNA molecule comprises all or part of a polynucleotide selected from the group consisting of: SEQ ID NO:75; the complement of SEQ ID NO:75; SEQ ID NO:76; the complement of SEQ ID NO:76; SEQ ID NO:77; the complement of SEQ ID NO:77; SEQ ID NO:78; the complement of SEQ ID NO:78; SEQ ID NO:79; the complement of SEQ ID NO:79; SEQ ID NO:80; the complement of SEQ ID NO:80; a polynucleotide that hybridizes to a native *rpI1* polynucleotide of an insect *(e.g.,* WCR); the complement of a polynucleotide that hybridizes to a native *rpI1* polynucleotide of an insect; a polynucleotide that hybridizes to a native coding polynucleotide of a *Diabrotica* organism *(e.g.,* WCR) comprising all or part of any of SEQ ID NOs:1, 3, and 5-8; the complement of a polynucleotide that hybridizes to a native coding polynucleotide of a *Diabrotica* organism comprising all or part of any of SEQ ID NOs:1, 3, and 5-8.

In particular embodiments, an iRNA that functions upon being taken up by an insect pest to inhibit a biological function within the pest is transcribed from a DNA comprising all or part of a polynucleotide selected from the group consisting of: SEQ ID NO: 1; the complement of SEQ ID NO: 1; SEQ ID NO:3; the complement of SEQ ID NO:3; SEQ ID NO:5; the complement of SEQ ID NO:5; SEQ ID NO:6; the complement of SEQ ID NO:6; SEQ ID NO:7; the complement of SEQ ID NO:7; SEQ ID NO:8; the complement of SEQ ID NO:8; a native coding polynucleotide of a *Diabrotica* organism *(e.g.,* WCR) comprising all or part of any of SEQ ID NOs:1, 3, and 5-8; the complement of a native coding polynucleotide of a *Diabrotica* organism comprising all or part of any of SEQ ID NOs:1, 3, and 5-8.

Also disclosed herein are methods wherein dsRNAs, siRNAs, shRNAs, miRNAs, and/or hpRNAs may be provided to an insect pest in a diet-based assay, or in genetically-modified plant cells expressing the dsRNAs, siRNAs, shRNAs, miRNAs, and/or hpRNAs. In these and further examples, the dsRNAs, siRNAs, shRNAs, miRNAs, and/or hpRNAs may be ingested by the pest. Ingestion of dsRNAs, siRNAs, shRNAs, miRNAs, and/or hpRNAs of the invention may then result in RNAi in the pest, which in turn may result in silencing of a gene essential for viability of the pest and leading ultimately to mortality. Thus, methods are disclosed wherein nucleic acid molecules comprising exemplary polynucleotide(s) useful for control of insect pests are provided to an insect pest. In particular examples, a coleopteran pest controlled by use of nucleic acid molecules of the invention may be WCR, NCR, SCR, *D. undecimpunctata howardi*, *D. balteata*, *D. undecimpunctata tenella*, *D. speciosa*, or *D. u. undecimpunctata.*

The foregoing and other features will become more apparent from the following Detailed Description of several embodiments, which proceeds with reference to the accompanying **FIGs. 1-2.**

## BRIEF DESCRIPTION OF THE FIGURES

**FIG. 1** includes a depiction of a strategy used to provide dsRNA from a single transcription template with a single pair of primers.

**FIG. 2** includes a depiction of a strategy used to provide dsRNA from two transcription templates.

## SEQUENCE LISTING

The nucleic acid sequences listed in the accompanying sequence listing are shown using standard letter abbreviations for nucleotide bases, as defined in 37 C.F.R. § 1.822. The nucleic acid and amino acid sequences listed define molecules (*i.e.*, polynucleotides and polypeptides, respectively) having the nucleotide and amino acid monomers arranged in the manner described. The nucleic acid and amino acid sequences listed also each define a genus of polynucleotides or polypeptides that comprise the nucleotide and amino acid monomers arranged in the manner described. In view of the redundancy of the genetic code, it will be understood that a nucleotide sequence including a coding sequence also describes the genus of polynucleotides encoding the same polypeptide as a polynucleotide consisting of the reference sequence. It will further be understood that an amino acid sequence describes the genus of polynucleotide ORFs encoding that polypeptide.

Only one strand of each nucleic acid sequence is shown, but the complementary strand is understood as included by any reference to the displayed strand. As the complement and reverse complement of a primary nucleic acid sequence are necessarily disclosed by the primary sequence, the complementary sequence and reverse complementary sequence of a nucleic acid sequence are included by any reference to the nucleic acid sequence, unless it is explicitly stated to be otherwise (or it is clear to be otherwise from the context in which the sequence appears). Furthermore, as it is understood in the art that the nucleotide sequence of a RNA strand is determined by the sequence of the DNA from which it was transcribed (but for the substitution of uracil (U) nucleobases for thymine (T)), a RNA sequence is included by any reference to the DNA sequence encoding it. In the accompanying sequence listing:

SEQ ID NO:1 shows a contig containing an exemplary *Diabrotica rpl1* DNA, also referred to herein in some places as *rpl1-1*:

TTCAAATGAAGAGAAGAAGAAGAAGAATCTTCAAAGCATGTTTTTGTTAGGTTAAATTTCAAATTT
TTTAATTTGATGTTTGTTATTGTGTTTAAAAACAAGATTATTAAATAAACGGAAACATGAAGATTC
ACTTTATACCCGATAAAGTGAGCTTTTCACTTTTTACCACAGAAGAAATCAAGAAAATGTGTGTTA
CTCAAATTATTACCCCACTTATGTTAGATCCCTTAGGTCATCCTCTACAAGGAGGTTTATATGATC
GTAAATTAGGACCATACTCAGCCAAAGATACTCTATGTGAATCTTGTAACCAAACATTCACCAATT
GTCCTGGCCATTTTGGATACATTGAACTTCCTTTACCAGTAGTAAATCCTCTGTTTCATAAAATTA
TTGGAACCATTATAAAAATATCATGTTTGTCATGTTTCCACATACAACTACCAACTCATATAAAAA
AAGTTTTATGTATTCAAATGAAACTTTTAAATTGTGGACTGATTACAGAAGCCTTATCAGTAGAAA
CAGCATTAGCGGAATTAATTTCGAAATATGAAAAGTTTGAGAATATTCCAACTGAAAGTATAAAAA
GTGTATTACGATATGAGGAATTAGCTGATGAAACATTAAAGAATCTAGAAGGTAAAAATGTAACCA
GCCAAAACACCGAAACACTCCGCAACAACTTTGTGTCTAAAATGTTGAAAGAGGTAAAATCTAGAC
AACTATGTATATTTTGTAAAAACAGAATTAATAGGATTCAGGCACTAAAGAACAGAATTATTTTAA
CAAAAAAGAAAGGTGATCTAGATGATTCTAACGTGGTTATGGGACAAAAAGTGACAGGTATGGAGT
CTCAATACATTACGCCGGAAGAGTCAAGGCAATATTTAAGAAATATATGGAGACAAGAAAAAGAAT
TTCTTCAACAACTTGTATCTGTACTCGCAAATGTCGACTGTGAACACCCAACTGATGCATTTTATT
TTGAAGTGATTCCTGTTCCACCACCAAATGTTAGACCTGTTAACTTCGTGAATGGTAGAATATTAG
AGAATAAACAATCGATTGGTTACAAAAATATCATACAAAATGTCATTCTTTTAAAAACTATAATAC
AAGTAGTACAAAGTAAGAGCGATATTAATAGCTTGTCATCTGTGGAAGCTAAGAGTGCCTATAGCA
TTGCCAGAGGAAATTCTCCTGTAGAAAAATTAAATTATTGCTGGGAAGAATTGCAGAGTGACGTAA
ACGGGTTACTTGATAATGAAAATGTTCGACAAGAGGGACAGGGATTGAAACAGATCATTGAAAAGA
AAGAAGGTGTTATACGTATGTGTATGATGGGTAAACGTGTTAATTTTTCCGCTAGATCAGTTATAA
CTCCTGACCCCAACCTTAATATCGACGAAATCGGAATCCCGGAAGAATTTGCAAAGAAGCTGACTT
ATCCAGTAGCTGTAACTCCTTGGAATGTTGAAGAACTAAGGAAAATGATTTTAAATGGACCTAATG
TTCACCCAGGGGCAATTATGATAGAAAACAATGGGATTCTAAAACGAATTAATCCCTATAATGAAG
TTCAACAAAAAAGCATATTGAAATGTTTGTTAACTCCTGAAGCCACTAAAGGACCAAAAGGACAAG
GAATTCAAATCGTGCATAGGCATTTATGCAACGGTGACGTTCTGTTACTTAATCGCCAACCAACTT
TGCACAAACCTAGTATAATGGCGCACACAGCGCGAATTTTAAAGGGAGAAAAAACTCTTCGATTGC
ATTATGCTAATTGTAAAGCCTATAATGCTGATTTTGACGGGGATGAAATGAACGCACATTACCCCC
AAAATGAACTTGCAAGAAGTGAAGGCTATAATATTGTTAACGTTTCTAACCAATATCTCGTTCCAA
AAGATGGCACTCCTCTCAGTGGTTTAATTCAGGACCACATGATTTCTGGTGTGCGACTGTCTTTAA
GAGGAAGATTTTTTGATAAACAAGATTATGAGCATCTAGTTTACCAAGCTCTCTCGTTCAAAACAG
GTCGCATAAAGCTCTTACCACCAACAATTATAAAACCACAAGTGTTATGGTCTGGGAAACAAATTT
TATCTACAGTTATAATTAACGTCATACCCGACGAAAGAGAATTAATCAATCTTACGTCAACAGCTA
AGATTTCTTCCAAAGCTTGGCAGAGAAGACCCTCGAGAAGATGGAGAGCAGGTGGTACTATATTTC
TTGATGACAAGGTCATGAGTGAAGCTGAGGTCATAATTAGAGGTGGTGAACTTCTTGTTGGGGTTC
TAGATAAAACTCACTACGGTTCTACTCCTTATGGTTTAGTACACTGTATTTATGAGTTATATGGGG
GTACCTATGCAATCAGATTACTTTCCTCGTTGACAAAACTTTTCATGAGATTTTTGCAACAAGAAG
GGTTTACACTTGGAGTACATGATATACTTACAGTAGAAAGAGCTGATGTTAGGAGAAGGGAAATTA
TAAAAGACTGTAGACAAGTAGGAAAAGAAGCCGTAACTAAAGCTTTAGATGTACCTTTAGACACTC
CTGATGCTGAAGTTGTTGAAACAATAGAAAAACTAAGTGCTGCTGATCCCAAAATTAGAGCTACAA
TCGACAGGTCCTACAAGTCTTCGATGGATATTTTTACCAATGAAATTAATAGAACTTGTTTGCCTG
CTGGTCTGGTTTGTAAATTTCCTGAAAATAATCTTCAATTGATGGTACAATCTGGAGCGAAAGGTT
CAACAGTAAATACTATGCAAATTTCCTGTCTTCTTGGTCAAATAGAATTGGAAGGAAAACGGCCAC
CTGTAATGATATCCGGAAAATCTCTACCTAGTTTTCCATCATTCGAGTTTACTCCAAGGGCGGGAG
GATTTATCGATGGACGATTCATGACTGGTATCCAACCGCAAGAATTCTTCTTCCATTGTATGGCAG
GACGTGAAGGTCTTATTGATACAGCTGTTAAAACTAGTCGTAGTGGATATTTACAAAGATGTCTCA

TCAAACATTTGGAAGGTCTACGTGTTGGTTATGATATGACCGTGAGAAACAGTGATAAAAGTGTAA
TACAGTTTTTGTATGGAGAGGATGGAATGGATATTTCAAAAGCTCAGTTTTTCAATGAAAAACAGA
TGAGCTTCTTGGCCGAAAATATCAAGGTGTTGGGTAATTCTGATACGCTTAAACAGTTGAAGAATG
AAGAAGATCAAGAGGCTGTAAAAGAACATGGAAAAGCGGTAAAAGAATGGAAGAAACATCATGGGA
ATCCATTAAATCACAGAAGGAATAGTCCATTTTCCCTGTTTGCTAAATATGTTCAAAATAGGACTG
GAGATAACAACCTTTTAACCAAGGAGAAGTTAATGAAACTTTGGTATGAAATGGACAAGGACATAA
AAACAAACTTCACCGACCAGTGCGAGAAATGCCCAGATCCCATAGAAGCCGTGTATCAACCTGATG
CAAATTTTGGAGCGATAAATGAAACAGTGCAGAAACTTATCAAGAACTATAAAGGATTCGACAATA
AGAAAAGTAAGAAAAAATTTGAAGATGTCATAAAATTGAAAGTAATGGAATCGATGTGTTCTGCTG
GAGAACCAGTTGGACTTCTTGCGGCACAATCAATAGGAGAACCATCTACCCAGATGACACTCAATA
CTTTCCATTTTGCTGGAAGAGGTGAAATGAACGTTACTCTCGGTATTCCCCGTTTAAGAGAAATCT
TAATGATGGCTTCAAAGAATATAAAAACGCCATCGATGGAGATTCCATTCTTGCAGGTTCCAGATT
TAGAATATAAGGCGAACGAGTTAAGGAAACTTCTGACTCGAGTGGTGGTAGCCGACGTTTTAGAAA
CTATTGATGTTACTGTTGAACTTCAATTTAAACCCATTAGGCAATATAAGTATACCTTGAAATTCC
AATTTTTACCGAAGAAATATTACAGTATGGATTATTGTGTGAATCCCACAAAAATACTAAGGCATA
TGAAGGGGAAATATTTTGGTGAAATGTTTGCGTCCATCAAGAAAGTCAGTAAAATTAATTCAAACA
TAGTAATGATGGAGGAAGAAAGAAACAAGAAACGTACAACTAATAACGAAGAAGATGAAGATCGAC
CAGAAACAAATGAAAGAGAAGGTGACAATCAAATTGATTCCTCAGATGACGAAGTGGAAGATAATG
AAGATGCTAAGCAGAGTCATAAGTACCAAGAAACAAGGGATGATTTAGAACCAGAAGAAGAAGAGA
AAGAAAAATCTGACGATGAGGATGATGAAAGCGATAACGAAACCCAAGCGAACCAAAAAGAAACTG
ACAATCAAGAACAAGATAATGAAGTAGTTGATAGTTACAATTTTGCACAAAGTTATTATGAAGACC
AACAAAAACAATTGTGGTGTGAAATAACATTTGGTTTGCCCTTGTCGTTCAAAAAATTGGATCTTA
CTGCAATTTTAAAGGAGACTGCCGGCAAATCTGTTCTTTGGGAAACGCCCCAAATTAAAAGAGCCA
TTACTTATGTGAAGGATGATAAATTAATGCTTAGAACGGATGGTATTAATATTGTTGAAATGTTTA
AATACAATACCCTTTTAGACTTGCCACAACTTTATTGTAATGATATCCATAAAGTGGCAGAAACAT
ACGGCATTGAAGCAGCATCTAAAGTAATAGTAAAGGAGGTTAAAGACGTATTTAATGTGTACGGAA
TTAAAGTAGATCCTCGTCATTTGTCCCTAGTAGCCGACTACATGACATTTAATGGTACATTTGAAC
CACTCAGCAGAAGAGGAATGGAAAACAGCGCTTCCCCTCTGCAACAGATGTCATTTGAATCATCTT
TAGTATTTTTAAGGAATGCAGCAATTAGAGGCCGAGAAGATGATTTACAAAACCCTTCGAGTAGTC
TTATGTTAGGAAAACCATGTGGAACCGGCACAGGAAGCTTTACCCTTTTACATAAGTCCTTTGTAA
CATGTTAATAAATAAATTGTTATAGATAAAAAAAAAAA

SEQ ID NO:2 shows the amino acid sequence of a *Diabrotica* RPI1 polypeptide, also referred to herein in some places as RPI1-1 encoded by an exemplary *Diabrotica rpl1-1* DNA:

MKIHFIPDKVSFSLFTTEEIKKMCVTQIITPLMLDPLGHPLQGGLYDRKLGPYSAKDTLCESCNQT
FTNCPGHFGYIELPLPVVNPLFHKIIGTIIKISCLSCFHIQLPTHIKKVLCIQMKLLNCGLITEAL
SVETALAELISKYEKFENIPTESIKSVLRYEELADETLKNLEGKNVTSQNTETLRNNFVSKMLKEV
KSRQLCIFCKNRINRIQALKNRIILTKKKGDLDDSNVVMGQKVTGMESQYITPEESRQYLRNIWRQ
EKEFLQQLVSVLANVDCEHPTDAFYFEVIPVPPPNVRPVNFVNGRILENKQSIGYKNIIQNVILLK
TIIQVVQSKSDINSLSSVEAKSAYSIARGNSPVEKLNYCWEELQSDVNGLLDNENVRQEGQGLKQI
IEKKEGVIRMCMMGKRVNFSARSVITPDPNLNIDEIGIPEEFAKKLTYPVAVTPWNVEELRKMILN
GPNVHPGAIMIENNGILKRINPYNEVQQKSILKCLLTPEATKGPKGQGIQIVHRHLCNGDVLLLNR
QPTLHKPSIMAHTARILKGEKTLRLHYANCKAYNADFDGDEMNAHYPQNELARSEGYNIVNVSNQY
LVPKDGTPLSGLIQDHMISGVRLSLRGRFFDKQDYEHLVYQALSFKTGRIKLLPPTIIKPQVLWSG
KQILSTVIINVIPDERELINLTSTAKISSKAWQRRPSRRWRAGGTIFLDDKVMSEAEVIIRGGELL
VGVLDKTHYGSTPYGLVHCIYELYGGTYAIRLLSSLTKLFMRFLQQEGFTLGVHDILTVERADVRR
REIIKDCRQVGKEAVTKALDVPLDTPDAEVVETIEKLSAADPKIRATIDRSYKSSMDIFTNEINRT
CLPAGLVCKFPENNLQLMVQSGAKGSTVNTMQISCLLGQIELEGKRPPVMISGKSLPSFPSFEFTP
RAGGFIDGRFMTGIQPQEFFFHCMAGREGLIDTAVKTSRSGYLQRCLIKHLEGLRVGYDMTVRNSD

KSVIQFLYGEDGMDISKAQFFNEKQMSFLAENIKVLGNSDTLKQLKNEEDQEAVKEHGKAVKEWKK
HHGNPLNHRRNSPFSLFAKYVQNRTGDNNLLTKEKLMKLWYEMDKDIKTNFTDQCEKCPDPIEAVY
QPDANFGAINETVQKLIKNYKGFDNKKSKKKFEDVIKLKVMESMCSAGEPVGLLAAQSIGEPSTQM
TLNTFHFAGRGEMNVTLGIPRLREILMMASKNIKTPSMEIPFLQVPDLEYKANELRKLLTRVVVAD
VLETIDVTVELQFKPIRQYKYTLKFQFLPKKYYSMDYCVNPTKILRHMKGKYFGEMFASIKKVSKI
NSNIVMMEEERNKKRTTNNEEDEDRPETNEREGDNQIDSSDDEVEDNEDAKQSHKYQETRDDLEPE
EEEKEKSDDEDDESDNETQANQKETDNQEQDNEVVDSYNFAQSYYEDQQKQLWCEITFGLPLSFKK
LDLTAILKETAGKSVLWETPQIKRAITYVKDDKLMLRTDGINIVEMFKYNTLLDLPQLYCNDIHKV
AETYGIEAASKVIVKEVKDVFNVYGIKVDPRHLSLVADYMTFNGTFEPLSRRGMENSASPLQQMSF
ESSLVFLRNAAIRGREDDLQNPSSSLMLGKPCGTGTGSFTLLHKSFVTC

SEQ ID NO:3 shows a contig comprising a further exemplary *Diabrotica rpl1* DNA, also referred to herein in some places as *rpl1-2*:

TGCTCGACCTGTAGATTCTTGTAACGGATTTCGGAGAGTTCGATTCGTTGTCGAGCCTTCAAAATG
GCTACCAACGATAGTAAAGCTCCGTTGAGGACAGTTAAAAGAGTGCAATTTGGAATACTTAGTCCA
GATGAAATTAGACGAATGTCAGTCACAGAAGGGGGCATCCGCTTCCCAGAAACCATGGAAGCAGGC
CGCCCCAAACTATGCGGTCTTATGGACCCCAGACAAGGTGTCATAGACAGAAGCTCAAGATGCCAG
ACATGTGCCGGAAATATGACAGAATGTCCTGGACATTTCGGACATATCGAGCTGGCAAAACCAGTT
TTCCACGTAGGATTCGTAACAAAAACAATAAAGATCTTGAGATGCGTTTGCTTCTTTTGCAGTAAA
TTATTAGTCAGTCCAAATAATCCGAAAATTAAAGAAGTTGTAATGAAATCAAAGGGACAGCCACGT
AAAAGATTAGCTTTCGTTTATGATCTGTGTAAAGGTAAAAATATTTGTGAAGGTGGAGATGAAATG
GATGTGGGTAAAGAAAGCGAAGATCCCAATAAAAAAGCAGGCCATGGTGGTTGTGGTCGATATCAA
CCAAATATCAGACGTGCCGGTTTAGATTTAACAGCAGAATGGAAACACGTCAATGAAGACACACAA
GAAAAGAAAATCGCACTATCTGCCGAACGTGTCTGGGAAATCCTAAAACATATCACAGATGAAGAA
TGTTTCATTCTTGGTATGGATCCCAAATTTGCTAGACCAGATTGGATGATAGTAACGGTACTTCCT
GTTCCTCCCCTAGCAGTACGACCTGCTGTAGTTATGCACGGATCTGCAAGGAATCAGGATGATATC
ACTCACAAATTGGCCGACATTATCAAGGCGAATAACGAATTACAGAAGAACGAGTCTGCAGGTGCA
GCCGCTCATATAATCACAGAAAATATTAAGATGTTGCAATTTCACGTCGCCACTTTAGTTGACAAC
GATATGCCGGGAATGCCGAGAGCAATGCAAAAATCTGGAAAACCCCTAAAAGCTATCAAAGCTCGG
CTGAAAGGTAAAGAAGGAAGGATTCGAGGTAACCTTATGGGAAAGCGTGTGGACTTTTCTGCACGT
ACTGTCATCACACCAGATCCCAATTTACGTATCGACCAAGTAGGAGTGCCTAGAAGTATTGCTCAA
AACATGACGTTTCCAGAAATCGTCACACCTTTCAATTTTGACAAAATGTTGGAATTGGTACAGAGA
GGTAATTCTCAGTATCCAGGAGCTAAGTATATCATCAGAGACAATGGAGAGAGGATTGATTTACGT
TTCCACCCAAAACCGTCAGATTTACATTTGCAGTGTGGTTATAAGGTAGAAAGACACATCAGAGAC
GGCGATCTAGTAATCTTCAACCGTCAACCAACCCTCCACAAGATGAGTATGATGGGCCACAGAGTC
AAAGTCTTACCCTGGTCGACGTTCCGTATGAATCTCTCGTGCACCTCTCCCTACAACGCCGATTTT
GACGGCGACGAAATGAACCTCCATGTGCCCCAAAGTATGGAAACTCGAGCTGAAGTCGAAAACCTC
CACATCACTCCCAGGCAAATCATTACTCCGCAAGCTAACCAACCCGTCATGGGTATTGTACAAGAT
ACGTTGACAGCTGTTAGGAAGATGACAAAAAGGGATGTATTCATCGAGAAGGAACAAATGATGAAT
ATATTGATGTTCTTGCCAATTTGGGATGGTAAAATGCCCCGTCCAGCCATCCTCAAACCCAAACCG
TTGTGGACAGGAAAACAGATATTTTCCCTGATCATTCCTGGCAATGTAAATATGATACGTACCCAT
TCTACGCATCCAGACGACGAGGACGACGGTCCCTATAAATGGATATCGCCAGGAGATACGAAAGTT
ATGGTAGAACATGGAGAATTGGTCATGGGTATATTGTGTAAGAAAAGTCTTGGAACATCAGCAGGT
TCCCTGCTGCATATTTGTATGTTGGAATTAGGACACGAAGTGTGTGGTAGATTTTATGGTAACATT
CAAACTGTAATCAACAACTGGTTGTTGTTAGAAGGTCACAGCATCGGTATTGGAGACACCATTGCC
GATCCTCAGACTTACACAGAAATTCAGAGAGCCATCAGGAAAGCCAAAGAAGATGTAATAGAAGTC
ATCCAGAAAGCTCACAACATGGAACTGGAACCGACTCCCGGTAATACGTTGCGTCAGACTTTCGAA
AATCAAGTAAACAGAATTCTAAACGACGCTCGTGACAAAACTGGTGGTTCCGCTAAGAAATCTTTG
ACTGAATACAATAACCTAAAGGCTATGGTCGTATCGGGATCCAAGGATCCAACATTAATATTTCC

CAGGTTATTGCTTGCGTGGGTCAACAGAACGTAGAAGGTAAACGTATTCCATTTGGCTTCAGAAAA
CGCACGTTGCCGCACTTCATCAAGGACGATTACGGTCCTGAATCCAGAGGTTTCGTAGAAAATTCG
TATCTTGCCGGTCTCACTCCTTCGGAGTTCTATTTCCACGCTATGGGAGGTCGTGAAGGTCTTATC
GATACTGCTGTAAAAACTGCCGAAACTGGTTACATCCAACGTCGTCTGATAAAGGCTATGGAGAGT
GTAATGGTACACTACGACGGTACCGTAAGAAATTCTGTAGGACAACTTATCCAGCTGAGATACGGT
GAAGACGGACTCTGTGGAGAGATGGTAGAGTTTCAATATTTAGCAACAGTCAAATTAAGTAACAAG
GCGTTTGAGAGAAAATTCAGATTTGATCCAAGTAATGAAAGGTATTTGAGAAGAGTTTTCAATGAA
GAAGTTATCAAGCAACTGATGGGTTCAGGGGAAGTCATTTCCGAACTTGAGAGAGAATGGGAACAA
CTCCAGAAAGACAGAGAAGCCTTAAGACAAATCTTCCCTAGCGGAGAATCTAAAGTAGTACTCCCC
TGTAACTTACAACGTATGATCTGGAATGTACAAAAAATTTTCCACATAAACAAACGAGCCCCGACA
GACCTGTCCCCGTTAAGAGTTATCCAAGGCGTTCGAGAATTACTCAGGAAATGCGTCATCGTAGCT
GGCGAGGATCGTCTGTCCAAACAAGCCAACGAAAACGCAACGTTACTCTTCCAGTGTCTAGTCAGA
TCGACCCTCTGCACCAAATGCGTTTCTGAAGAATTCAGGCTCAGCACCGAAGCCTTCGAGTGGTTG
ATAGGAGAAATCGAGACGAGGTTCCAACAAGCCCAAGCCAATCCTGGAGAAATGGTGGCGCTCTG
GCCGCGCAGTCACTGGGAGAACCCGCTACTCAGATGACACTGAACACTTTCCATTTTGCTGGTGTA
TCCTCCAAGAACGTAACCCTGGGTGTACCTAGATTAAAGGAAATTATTAATATTTCCAAGAAACCC
AAGGCTCCATCTCTAACCGTGTTTTTAACTGGTGCGGCTGCTAGAGATGCGGAAAAAGCGAAGAAT
GTGTTATGCAGACTTGAACACACCACTCTTCGTAAAGTAACCGCCAACACCGCCATCTATTACGAT
CCTGACCCACAAAATACCGTCATTCCTGAGGATCAGGAGTTCGTTAACGTCTACTATGAAATGCCC
GATTTCGATCCTACCCGTATATCGCCGTGGTTGCTTCGTATCGAACTGGACAGAAAGAGAATGACA
GATAAGAAACTAACTATGGAACAAATTGCTGAAAAGATCAACGCTGGGTTCGGGGACGATTTGAAT
TGTATTTTCAACGACGACAATGCTGAAAAGTTGGTGCTGCGTATCAGAATCATGAACAGCGACGAT
GGAAAATTCGGAGAAGGTGCTGATGAGGACGTAGACAAAATGGATGACGACATGTTTTTGAGATGC
ATCGAAGCGAACATGCTGAGCGATATGACCTTGCAAGGTATAGAAGCGATTTCCAAGGTATACATG
CACTTGCCACAGACTGACTCGAAAAAAAGGATCGTCATCACTGAAACAGGCGAATTTAAGGCCATC
GCAGAATGGCTATTGGAAACTGACGGTACCAGCATGATGAAAGTACTGTCAGAAAGAGACGTCGAT
CCGGTCAGGACGTTTTCTAACGACATTTGTGAAATATTTTCGGTACTTGGTATCGAGGCTGTGCGT
AAGTCTGTAGAGAAAGAAATGAACGCTGTCCTTTCATTCTACGGTCTGTACGTAAACTATCGCCAT
CTTGCCTTGCTTTGTGACGTAATGACAGCCAAAGGTCACTTAATGGCCATCACCCGTCACGGTATC
AACAGACAAGACACTGGAGCTCTGATGAGGTGTTCCTTCGAGGAAACTGTAGATGTATTGATGGAC
GCTGCCAGTCATGCGGAGGTCGACCCAATGAGAGGAGTATCTGAAAACATTATCCTCGGTCAACTA
CCAAGAATGGGCACAGGCTGCTTCGATCTTTTGCTGGACGCCGAAAAATGTAAAATGGGAATTGCC
ATACCTCAAGCGCACAGCAGCGATCTAATGGCTTCAGGAATGTTCTTTGGATTAGCCGCTACACCC
AGCAGTATGAGTCCAGGTGGTGCTATGACCCCATGGAATCAAGCAGCTACACCATACGTTGGCAGT
ATCTGGTCTCCACAGAATTTAATGGGCAGTGGAATGACACCAGGTGGTGCCGCTTTCTCCCCATCA
GCTGCGTCAGATGCATCAGGAATGTCACCAGCTTATGGCGGTTGGTCACCAACACCACAATCTCCT
GCAATGTCGCCATATATGGCTTCTCCACATGGACAATCGCCTTCCTACAGTCCATCAAGTCCAGCG
TTCCAACCTACTTCACCATCCATGACGCCGACCTCTCCTGGATATTCTCCCAGTTCTCCTGGTTAT
TCACCTACCAGTCTCAATTACAGTCCAACGAGTCCCAGTTATTCACCCACTTCTCAGAGTTACTCC
CCAACCTCACCTAGTTACTCACCGACTTCTCCAAATTATTCACCTACTTCCCCAAGCTACAGTCCA
ACATCCCCTAACTATTCACCAACATCTCCCAACTATTCACCCACTTCACCTAGTTATCCTTCAACT
TCGCCAGGTTACAGCCCCACTTCACGCAGCTACTCACCCACATCTCCTAGTTACTCAGGAACTTCG
CCCTCTTATTCACCAACTTCGCCAAGTTACTCCCCTACTTCTCCTAGTTATTCGCCGTCGTCTCCT
AATTACTCTCCCACTTCTCCAAATTACAGTCCCACTTCTCCTAATTACTCACCGTCCTCTCCTAGG
TACACGCCCGGTTCTCCTAGTTTTTCCCCAAGTTCGAACAGTTACTCTCCCACATCTCCTCAATAT
TCTCCAACATCTCCAAGTTATTCGCCTTCTTCGCCCAAATATTCACCAACTTCCCCCAATTATTCG
CCAACATCTCCATCATTTTCTGGAGGAAGTCCACAATATTCACCCACATCACCGAAATACTCTCCA
ACCTCGCCCAATTACACTCTGTCGAGTCCGCAGCACACTCCAACAGGTAGCAGTCGATATTCACCG
TCAACTTCGAGTTATTCTCCTAATTCGCCCAATTATTCACCGACGTCTCCACAATACTCCATCCAC

AGTACAAAATATTCCCCTGCAAGTCCTACATTCACACCCACCAGTCCTAGTTTCTCTCCCGCTTCA
CCCGCATATTCGCCTCAACCTATGTATTCACCTTCTTCTCCTAATTATTCTCCCACTAGTCCCAGT
CAAGACACTGACTAAATATAATCATAAGATTGTAGTGGTTAGTTGTATTTTATACATAGATTTTAA
TTCAGAATTTAATATTATTTTTTACTATTTACCAGGGACATTTTTAAAGTTGTAAAAACACTTACA
TTTGTTCCAACGGATTTTTGCACAAACGTAACGAAGTTAAATCAAAACATTACAACTGAAACATAC
GTCGGTATGTACTGTCAATGTGATCATTAGGAAATGGCTATTATCCCGGAGGACGTATTTTATAAA
GTTATTTTATTGAAGTGTTTGATCTTTTTTCACTATTGAGGAGATTTATGGACTCAACATTAAACA
GCTTGAACATCATACCGACTACTACTAATATAAAGATAAATATAGAACGGTAAGAAATAGATTAAA
AAAAAATACAATAAGTTAAACAGTAATCATAAAAATAAATACGTTTCCGTTCGACAGAACTATAGC
CAGATTCTTGTAGTATAATGAAAATTTGTAGGTTAAAAATATTACTTGTCACATTAGCTTAAAAAT
AAAAAATTACCGGAAGTAATCAAATAAGAGAGCAACAGTTAGTCGTTCTAACAATTATGTTTGAAA
ATAAAAATTACAATGAGTTATACAAACGAAGACTACAAGTTTAAATAGTATGAAAAACTATTTGTA
AACACAACAAATGCGCATTGAAATTTATTTATCGTACTTAACTTATTTGCCTTACAAAAATAATAC
TCCGCGAGTATTTTTTATGAACTGTAAAACTAAAAAGTTGTACAGTTCACACAAAAACATCGAAAA
ATTTTGTTTTTGTATGTTTCTATTATTAAAAAAATACTTTTTATCTTTCACCTTATAGGTACTATT
TGACTCTATGACATTTTCTCTACATTTCTTTAAATCTGTTCTATTTATTATGTACATGAATCTATA
AGCACAAATAATATACATAATCATTTTGATAAAAAATCATAGTTTTAAATAAAACAGATTTCAACA
CAATATTCATAAGTCTACTTTTTTAAAAATTTATAGAGACAAAGGCCATTTTTCAGAAACAGATTA
AACAAAAATCACTATAAATTATTTTGAGTATGTTGAATAAGTTTATATTGCTTCTACAATTTTTAA
ATATAAAATTATAACATTAGCAGAGGAACAACGAGAATTAAGGTCGGGAAGATCATGCACCGA

SEQ ID NO:4 shows the amino acid sequence of a *Diabrotica* RPI1 polypeptide, also referred to herein in some places as RPI1-2 encoded by an exemplary *Diabrotica rpl1-2* DNA:

MATNDSKAPLRTVKRVQFGILSPDEIRRMSVTEGGIRFPETMEAGRPKLCGLMDPRQGVIDRSSRC
QTCAGNMTECPGHFGHIELAKPVFHVGFVTKTIKILRCVCFFCSKLLVSPNNPKIKEVVMKSKGQP
RKRLAFVYDLCKGKNICEGGDEMDVGKESEDPNKKAGHGGCGRYQPNIRRAGLDLTAEWKHVNEDT
QEKKIALSAERVWEILKHITDEECFILGMDPKFARPDWMIVTVLPVPPLAVRPAVVMHGSARNQDD
ITHKLADIIKANNELQKNESAGAAAHIITENIKMLQFHVATLVDNDMPGMPRAMQKSGKPLKAIKA
RLKGKEGRIRGNLMGKRVDFSARTVITPDPNLRIDQVGVPRSIAQNMTFPEIVTPFNFDKMLELVQ
RGNSQYPGAKYIIRDNGERIDLRFHPKPSDLHLQCGYKVERHIRDGDLVIFNRQPTLHKMSMMGHR
VKVLPWSTFRMNLSCTSPYNADFDGDEMNLHVPQSMETRAEVENLHITPRQIITPQANQPVMGIVQ
DTLTAVRKMTKRDVFIEKEQMMNILMFLPIWDGKMPRPAILKPKPLWTGKQIFSLIIPGNVNMIRT
HSTHPDDEDDGPYKWISPGDTKVMVEHGELVMGILCKKSLGTSAGSLLHICMLELGHEVCGRFYGN
IQTVINNWLLLEGHSIGIGDTIADPQTYTEIQRAIRKAKEDVIEVIQKAHNMELEPTPGNTLRQTF
ENQVNRILNDARDKTGGSAKKSLTEYNNLKAMVVSGSKGSNINISQVIACVGQQNVEGKRIPFGFR
KRTLPHFIKDDYGPESRGFVENSYLAGLTPSEFYFHAMGGREGLIDTAVKTAETGYIQRRLIKAME
SVMVHYDGTVRNSVGQLIQLRYGEDGLCGEMVEFQYLATVKLSNKAFERKFRFDPSNERYLRRVFN
EEVIKQLMGSGEVISELEREWEQLQKDREALRQIFPSGESKVVLPCNLQRMIWNVQKIFHINKRAP
TDLSPLRVIQGVRELLRKCVIVAGEDRLSKQANENATLLFQCLVRSTLCTKCVSEEFRLSTEAFEW
LIGEIETRFQQAQANPGEMVGALAAQSLGEPATQMTLNTFHFAGVSSKNVTLGVPRLKEIINISKK
PKAPSLTVFLTGAAARDAEKAKNVLCRLEHTTLRKVTANTAIYYDPDPQNTVIPEDQEFVNVYYEM
PDFDPTRISPWLLRIELDRKRMTDKKLTMEQIAEKINAGFGDDLNCIFNDDNAEKLVLRIRIMNSD
DGKFGEGADEDVDKMDDDMFLRCIEANMLSDMTLQGIEAISKVYMHLPQTDSKKRIVITETGEFKA
IAEWLLETDGTSMMKVLSERDVDPVRTFSNDICEIFSVLGIEAVRKSVEKEMNAVLSFYGLYVNYR
HLALLCDVMTAKGHLMAITRHGINRQDTGALMRCSFEETVDVLMDAASHAEVDPMRGVSENIILGQ
LPRMGTGCFDLLLDAEKCKMGIAIPQAHSSDLMASGMFFGLAATPSSMSPGGAMTPWNQAATPYVG
SIWSPQNLMGSGMTPGGAAFSPSAASDASGMSPAYGGWSPTPQSPAMSPYMASPHGQSPSYSPSSP
AFQPTSPSMTPTSPGYSPSSPGYSPTSLNYSPTSPSYSPTSQSYSPTSPSYSPTSPNYSPTSPSYS
PTSPNYSPTSPNYSPTSPSYPSTSPGYSPTSRSYSPTSPSYSGTSPSYSPTSPSYSPTSPSYSPSS

PNYSPTSPNYSPTSPNYSPSSPRYTPGSPSFSPSSNSYSPTSPQYSPTSPSYSPSSPKYSPTSPNY
SPTSPSFSGGSPQYSPTSPKYSPTSPNYTLSSPQHTPTGSSRYSPSTSSYSPNSPNYSPTSPQYSI
HSTKYSPASPTFTPTSPSFSPASPAYSPQPMYSPSSPNYSPTSPSQDTD

SEQ ID NO:5 shows an exemplary *Diabrotica rpl1* DNA, referred to herein in some places as *rpl1-1* reg1 (region 1), which is used in some examples for the production of a dsRNA:

CTGAGGTCATAATTAGAGGTGGTGAACTTCTTGTTGGGGTTCTAGATAAAACTCACTACGGTTCTA
CTCCTTATGGTTTAGTACACTGTATTTATGAGTTATATGGGGGTACCTATGCAATCAGATTACTTT
CCTCGTTGACAAAACTTTTCATGAGATTTTTGCAACAAGAAGGGTTTACACTTGGAGTACATGATA
TACTTACAGTAGAAAGAGCTGATGTTAGGAGAAGGGAAATTATAAAAGACTGTAGACAAGTAGGAA
AAGAAGCCGTAACTAAAGCTTTAGATGTACCTTTAGACACTCCTGATGCTGAAGTTGTTGAAACAA
TAGAAAAACTAAGTGCTGCTGATCCCAAAATTAGAGCTACAATCGACAGGTCCTACAAGTCTTCGA
TGGATATTTTTACCAATGAAATTAATAGAACTTGTTTGCCTGCTGGTCTGGTTTGTAAATTTCCTG
AAAATAATCTTCAATTGATGGTACAATC

SEQ ID NO:6 shows a further exemplary *Diabrotica rpl1* DNA, referred to herein in some places as *rpl1-2* reg2 (region 2), which is used in some examples for the production of a dsRNA:

GTTATAAGGTAGAAAGACACATCAGAGACGGCGATCTAGTAATCTTCAACCGTCAACCAACCCTCC
ACAAGATGAGTATGATGGGCCACAGAGTCAAAGTCTTACCCTGGTCGACGTTCCGTATGAATCTCT
CGTGCACCTCTCCCTACAACGCCGATTTTGACGGCGACGAAATGAACCTCCATGTGCCCCAAAGTA
TGGAAACTCGAGCTGAAGTCGAAAACCTCCACATCACTCCCAGGCAAATCATTACTCCGCAAGCTA
ACCAACCCGTCATGGGTATTGTACAAGATACGTTGACAGCTGTTAGGAAGATGACAAAAAGGGATG
TATTCATCGAGAAGGAACAAATGATGAATATATTGATGTTCTTGCCAATTTGGGATGGTAAAATGC
CCCGTCCAGCCATCCTCAAACCCAAACCGTTGTGGACAGGAAAACAGATATTTTCCCTGATCATTC
CTGGCAATGTAAATATGATACGTACCCATTCTACGC

SEQ ID NO:7 shows a further exemplary *Diabrotica rpl1* DNA, referred to herein in some places as *rpl1-2* v1 (version 1), which is used in some examples for the production of a dsRNA:

ACCCTCCACAAGATGAGTATGATGGGCCACAGAGTCAAAGTCTTACCCTGGTCGACGTTCCGTATG
AATCTCTCGTGCACCTCTCCCTACAACGCCGATTTTGACGGCGACGAA

SEQ ID NO:8 shows a further exemplary *Diabrotica rpl1* DNA, referred to herein in some places as *rpl1-2* v2 (version 2), which is used in some examples for the production of a dsRNA:

ATGCCCCGTCCAGCCATCCTCAAACCCAAACCGTTGTGGACAGGAAAACAGATATTTTCCCTGATCATTCCT
GGCAATGTAAATATGATACGTACCCATTCTACGC

SEQ ID NO:9 shows a the nucleotide sequence of T7 phage promoter.
SEQ ID NO: 10 shows an exemplary *YFP* gene.
SEQ ID NOs:11-18 show primers used to amplify portions of exemplary *rpl1* sequences comprising *rpl1-1* reg1, *rpl1-2* reg2, *rpl1-2* v1, and/or *rpl1-2* v2, used in some examples for dsRNA production.
SEQ ID NO: 19 shows a DNA sequence of *annexin* region 1.
SEQ ID NO:20 shows a DNA sequence of *annexin* region 2.
SEQ ID NO:21 shows a DNA sequence of *beta spectrin* 2 region 1.
SEQ ID NO:22 shows a DNA sequence of *beta spectrin* 2 region 2.
SEQ ID NO:23 shows a DNA sequence of *mtRP-L4* region 1.
SEQ ID NO:24 shows a DNA sequence of *mtRP-L4* region 2.

SEQ ID NOs:25-52 show primers used to amplify gene regions of *annexin, beta spectrin* 2, *mtRP-L4,* and *YFP* for dsRNA synthesis.

SEQ ID NO:53 shows a maize DNA sequence encoding a TIP41-like protein.

SEQ ID NO:54 shows the nucleotide sequence of a T20VN primer oligonucleotide.

SEQ ID NOs:55-59 show primers and probes used for dsRNA transcript expression analyses.

SEQ ID NO:60 shows a nucleotide sequence of a portion of a *SpecR* coding region used for binary vector backbone detection.

SEQ ID NO:61 shows a nucleotide sequence of an *AAD1* coding region used for genomic copy number analysis.

SEQ ID NO:62 shows a DNA sequence of a maize *invertase* gene.

SEQ ID NOs:63-71 show the nucleotide sequences of DNA oligonucleotides used for gene copy number determinations and binary vector backbone detection.

SEQ ID NOs:72-74 show primers and probes used for dsRNA transcript maize expression analyses.

SEQ ID NOs:75-80 show exemplary RNAs transcribed from nucleic acids comprising exemplary *rpl1* polynucleotides and fragments thereof.

SEQ ID NO:81 shows an exemplary DNA encoding a *Diabrotica rpl-2* v1 dsRNA; containing a sense polynucleotide, a loop sequence (italics), and an antisense polynucleotide (underlined font):

ACCCTCCACAAGATGAGTATGATGGGCCACAGAGTCAAAGTCTTACCCTGGTCGACGTTCCGTATG
AATCTCTCGTGCACCTCTCCCTACAACGCCGATTTTGACGGCGACGAA*GAAGCTAGTACCAGTCAT*
*CACGCTGGAGCGCACATATAGGCCCTCCATCAGAAAGTCATTGTGTATATCTCTCATAGGGAACGA*
*GCTGCTTGCGTATTTCCCTTCCGTAGTCAGAGTCATCAATCAGCTGCACCGTGTCGTAAAGCGGGA*
*CGTTCGCAAGCTCGTCCGCGGTA*TTCGTCGCCGTCAAAATCGGCGTTGTAGGGAGAGGTGCACGAG
AGATTCATACGGAACGTCGACCAGGGTAAGACTTTGACTCTGTGGCCCATCATACTCATCTTGTGG
AGGGT

SEQ ID NO:82 shows a probe used for dsRNA expression analysis.

SEQ ID NO:83 shows an exemplary DNA nucleotide sequence encoding an intervening loop in a dsRNA.

SEQ ID NO:84 shows an exemplary dsRNA transcribed from a nucleic acid comprising exemplary *rpl-2* polynucleotide fragments.

## DETAILED DESCRIPTION

### *I. Overview of several embodiments*

We developed RNA interference (RNAi) as a tool for insect pest management, using one of the most likely target pest species for transgenic plants that express dsRNA; the western corn rootworm. Thus far, most genes proposed as targets for RNAi in rootworm larvae do not actually achieve their purpose. Herein, we describe RNAi-mediated knockdown of *RNA polymerase I* largest subunit (*rpl1*) in the exemplary insect pest, western corn rootworm, which is shown to have a lethal phenotype when, for example, iRNA molecules are delivered *via* ingested *rpl1* dsRNA. In embodiments herein, the ability to deliver *rpl1* dsRNA by feeding to insects confers an RNAi effect that is very useful for insect (*e.g.*, coleopteran) pest management. By combining *rpl1*-mediated RNAi with other useful RNAi targets (*e.g.,* ROP (U.S. Patent Application Publication No. 14/577811); RNAPII (U.S. Patent Application Publication No. 14/577854); *RNA polymerase II215* RNAi targets, as described in U.S. Patent Application No. 62/133202; *RNA polymerase II33* RNAi targets, as described in U.S. Patent Application No.62/133210; *ncm* RNAi targets, as described in U.S. Patent Application No. 62/095487; *Dre4* RNAi targets, as described in U.S. Patent Application No. 14/705,807; *COPI alpha* RNAi targets, as described in U.S. Patent Application No. 62/063,199; *COPI beta* RNAi targets, as described in U.S. Patent Application No. 62/063,203; *COPI gamma* RNAi targets, as described in U.S. Patent Application No. 62/063,192; and *COPI delta* RNAi targets, as described in U.S. Patent Application No. 62/063,216), the potential to affect multiple target sequences, for example, in rootworms (*e.g.*, larval rootworms), may increase opportunities to develop sustainable approaches to insect pest management involving RNAi technologies.

Disclosed herein are methods and compositions for genetic control of insect *(e.g.,* coleopteran) pest infestations. Methods for identifying one or more gene(s) essential to the lifecycle of an insect pest for use as a target gene for RNAi-mediated control of an insect pest population are also provided. DNA plasmid vectors encoding a RNA molecule may be designed to suppress one or more target gene(s) essential for growth, survival, and/or development. In some embodiments, the RNA molecule may be capable of forming dsRNA molecules. In some embodiments, methods are provided for post-transcriptional repression of expression or inhibition of a target gene *via* nucleic acid molecules that are complementary to a coding or non-coding sequence of the target gene in an insect pest. In these and further embodiments, a pest may ingest one or more dsRNA, siRNA, shRNA, miRNA, and/or hpRNA molecules transcribed from all or a portion of a nucleic acid molecule that is complementary to a coding or non-coding sequence of a target gene, thereby providing a plant-protective effect.

Thus, some embodiments involve sequence-specific inhibition of expression of target gene products, using dsRNA, siRNA, shRNA, miRNA, and/or hpRNA that is complementary to coding and/or non-coding sequences of the target gene(s) to achieve at least partial control of an insect (*e.g*., coleopteran) pest. Disclosed is a set of isolated and purified nucleic acid molecules comprising a polynucleotide, for example, as set forth in one of SEQ ID NOs:1, 3, and fragments thereof. In some embodiments, a stabilized dsRNA molecule may be expressed from these polynucleotides, fragments thereof, or a gene comprising one of these polynucleotides, for the post-transcriptional silencing or inhibition of a target gene. In certain embodiments, isolated and purified nucleic acid molecules comprise all or part of any of SEQ ID NOs:1; 3; and 5-8.

Some embodiments involve a recombinant host cell (*e.g.,* a plant cell) having in its genome at least one recombinant DNA encoding at least one iRNA (*e.g*., dsRNA) molecule(s). In particular embodiments, an encoded dsRNA molecule(s) may be provided when ingested by an insect (*e.g*., coleopteran) pest to post-transcriptionally silence or inhibit the expression of a target gene in the pest. The recombinant DNA may comprise, for example, any of SEQ ID NOs:1; 3; and 5-8; fragments of any of SEQ ID NOs:1; 3; and 5-8; a polynucleotide consisting of a partial sequence of a gene comprising one of SEQ ID NOs:1; 3; and 5-8; and/or complements thereof.

Some embodiments involve a recombinant host cell having in its genome a recombinant DNA encoding at least one iRNA (*e.g*., dsRNA) molecule(s) comprising all or part of SEQ ID NO:75 or SEQ ID NO:76 (*e.g.,* at least one polynucleotide selected from a group comprising SEQ ID NOs:77-80). When ingested by an insect (*e.g*., coleopteran) pest, the iRNA molecule(s) may silence or inhibit the expression of a target *rpl1* DNA *(e.g.,* a DNA comprising all or part of a polynucleotide selected from the group consisting of SEQ ID NOs:1, 3, and 5-8) in the pest, and thereby result in cessation of growth, development, and/or feeding in the pest.

In some embodiments, a recombinant host cell having in its genome at least one recombinant DNA encoding at least one RNA molecule capable of forming a dsRNA molecule may be a transformed plant cell. Some embodiments involve transgenic plants comprising such a transformed plant cell. In addition to such transgenic plants, progeny plants of any transgenic plant generation, transgenic seeds, and transgenic plant products, are all provided, each of which comprises recombinant DNA(s). In particular embodiments, a RNA molecule capable of forming a dsRNA molecule may be expressed in a transgenic plant cell. Therefore, in these and other embodiments, a dsRNA molecule may be isolated from a transgenic plant cell. In particular embodiments, the transgenic plant is a plant selected from the group comprising corn (*Zea mays*) and plants of the family *Poaceae.*

Some embodiments involve a method for modulating the expression of a target gene in an insect (*e.g*., coleopteran) pest cell. In these and other embodiments, a nucleic acid molecule may be provided, wherein the nucleic acid molecule comprises a polynucleotide encoding a RNA molecule capable of forming a dsRNA molecule. In particular embodiments, a polynucleotide encoding a RNA molecule capable of forming a dsRNA molecule may be operatively linked to a promoter, and may also be operatively linked to a transcription termination sequence. In particular embodiments, a method for modulating the expression of a target gene in an insect pest cell may comprise: (a) transforming a plant cell with a vector comprising a polynucleotide encoding a RNA molecule capable of forming a dsRNA molecule; (b) culturing the transformed plant cell under conditions sufficient to allow for development of a plant cell culture comprising a plurality of transformed plant cells; (c) selecting for a transformed plant cell that has integrated the vector into its genome; and (d) determining that the selected transformed plant cell comprises the RNA molecule capable of forming a dsRNA molecule encoded by the polynucleotide of the vector. A plant may be regenerated from a plant cell that has the vector integrated in its genome and comprises the dsRNA molecule encoded by the polynucleotide of the vector.

Thus, also disclosed is a transgenic plant comprising a vector having a polynucleotide encoding a RNA molecule capable of forming a dsRNA molecule integrated in its genome, wherein the transgenic plant comprises the dsRNA molecule encoded by the polynucleotide of the vector. In particular embodiments, expression of a RNA molecule capable of forming a dsRNA molecule in the plant is sufficient to modulate the expression of a target gene in a cell of an insect (*e.g*., coleopteran) pest that contacts the transformed plant or plant cell (for example, by feeding on the transformed plant, a part of the plant (*e.g.,* root) or plant cell), such that growth and/or survival of the pest is inhibited. Transgenic plants disclosed herein may display protection and/or enhanced protection to insect pest infestations. Particular transgenic plants may display protection and/or enhanced protection to one or more coleopteran pest(s) selected from the group consisting of: WCR; NCR; SCR; MCR; *D. balteata* LeConte; *D. u. tenella*; *D. speciosa* Germar; and *D. u. undecimpunctata* Mannerheim.

Also disclosed herein are methods for delivery of control agents, such as an iRNA molecule, to an insect (*e.g*., coleopteran) pest. Such control agents may cause, directly or indirectly, an impairment in the ability of an insect pest population to feed, grow, or otherwise cause damage in a host. In some embodiments, a method is provided comprising delivery of a stabilized dsRNA molecule to an insect pest to suppress at least one target gene in the pest, thereby causing RNAi and reducing or eliminating plant damage in a host of the pest. In some embodiments, a method of inhibiting expression of a target gene in the insect pest may result in cessation of growth, survival, and/or development in the pest.

In some embodiments, compositions (*e.g.*, a topical composition) are provided that comprise an iRNA (*e.g.*, dsRNA) molecule for use in plants, animals, and/or the environment of a plant or animal to achieve the elimination or reduction of an insect (*e.g.*, coleopteran) pest infestation. In particular embodiments, the composition may be a nutritional composition or food source to be fed to the insect pest, or an RNAi bait. Some embodiments comprise making the nutritional composition or food source available to the pest. Ingestion of a composition comprising iRNA molecules may result in the uptake of the molecules by one or more cells of the pest, which may in turn result in the inhibition of expression of at least one target gene in cell(s) of the pest. Ingestion of or damage to a plant or plant cell by an insect pest infestation may be limited or eliminated in or on any host tissue or environment in which the pest is present by providing one or more compositions comprising an iRNA molecule in the host of the pest.

The compositions and methods disclosed herein may be used together in combinations with other methods and compositions for controlling damage by insect *(e.g.,* coleopteran) pests. For example, an iRNA molecule as described herein for protecting plants from insect pests may be used in a method comprising the additional use of one or more chemical agents effective against an insect pest, biopesticides effective against such a pest, crop rotation, recombinant genetic techniques that exhibit features different from the features of RNAi-mediated methods and RNAi compositions (*e.g.*, recombinant production of proteins in plants that are harmful to an insect pest (*e.g., Bt* toxins)), and/or recombinant expression of other iRNA molecules.

## *II. Abbreviations*

| | |
|---|---|
| dsRNA | double-stranded ribonucleic acid |
| GI | growth inhibition |
| NCBI | National Center for Biotechnology Information |
| gDNA | genomic Deoxyribonucleic Acid |
| iRNA | inhibitory ribonucleic acid |
| ORF | open reading frame |
| RNAi | ribonucleic acid interference |
| miRNA | micro ribonucleic acid |
| shRNA | small hairpin ribonucleic acid |
| siRNA | small inhibitory ribonucleic acid |
| hpRNA | hairpin ribonucleic acid |
| UTR | untranslated region |
| WCR | Western corn rootworm (*Diabrotica virgifera virgifera* LeConte) |
| NCR | Northern corn rootworm (*Diabrotica barberi* Smith and Lawrence) |
| MCR | Mexican corn rootworm (*Diabrotica virgifera zeae* Krysan and Smith) |
| PCR | Polymerase chain reaction |
| qPCR | quantitative polymerase chain reaction |
| RISC | RNA-induced Silencing Complex |
| SCR | Southern corn rootworm (*Diabrotica undecimpunctata* howardi Barber) |
| SEM | standard error of the mean |
| YFP | yellow fluorescent protein |

## *III. Terms*

In the description and tables which follow, a number of terms are used. In order to provide a clear and consistent understanding of the specification and claims, including the scope to be given such terms, the following definitions are provided:

Coleopteran pest: As used herein, the term "coleopteran pest" refers to pest insects of the order Coleoptera, including pest insects in the genus *Diabrotica*, which feed upon agricultural crops and crop products, including corn and other true grasses. In particular examples, a coleopteran pest is selected from a list comprising *D. v. virgifera* LeConte (WCR); *D. barberi* Smith and Lawrence (NCR); *D. u. howardi* (SCR); *D. v. zeae* (MCR); *D. balteata* LeConte; *D. u. tenella; D. speciosa* Germar; and *D. u. undecimpunctata* Mannerheim.

Contact (with an organism): As used herein, the term "contact with" or "uptake by" an organism (*e.g.*, a coleopteran pest), with regard to a nucleic acid molecule, includes internalization of the nucleic acid molecule into the organism, for example and without limitation: ingestion of the molecule by the organism (*e.g.*, by feeding); contacting the organism with a composition comprising the nucleic acid molecule; and soaking of organisms with a solution comprising the nucleic acid molecule.

Contig: As used herein the term "contig" refers to a DNA sequence that is reconstructed from a set of overlapping DNA segments derived from a single genetic source.

Corn plant: As used herein, the term "corn plant" refers to a plant of the species, *Zea mays* (maize).

Expression: As used herein, "expression" of a coding polynucleotide (for example, a gene or a transgene) refers to the process by which the coded information of a nucleic acid transcriptional unit (including, *e.g.,* gDNA or cDNA) is converted into an operational, non-operational, or structural part of a cell, often including the synthesis of a protein. Gene expression can be influenced by external signals; for example, exposure of a cell, tissue, or organism to an agent that increases or decreases gene expression. Expression of a gene can also be regulated anywhere in the pathway from DNA to RNA to protein. Regulation of gene expression occurs, for example, through controls acting on transcription, translation, RNA transport and processing, degradation of intermediary molecules such as mRNA, or through activation, inactivation, compartmentalization, or degradation of specific protein molecules after they have been made, or by combinations thereof. Gene expression can be measured at the RNA level or the protein level by any method known in the art, including, without limitation, northern blot, RT-PCR, western blot, or *in vitro, in situ*, or *in vivo* protein activity assay(s).

Genetic material: As used herein, the term "genetic material" includes all genes, and nucleic acid molecules, such as DNA and RNA.

Inhibition: As used herein, the term "inhibition," when used to describe an effect on a coding polynucleotide (for example, a gene), refers to a measurable decrease in the cellular level of mRNA transcribed from the coding polynucleotide and/or peptide, polypeptide, or protein product of the coding polynucleotide. In some examples, expression of a coding polynucleotide may be inhibited such that expression is approximately eliminated. "Specific inhibition" refers to the inhibition of a target coding polynucleotide without consequently affecting expression of other coding polynucleotides (*e.g*., genes) in the cell wherein the specific inhibition is being accomplished.

Insect: As used herein with regard to pests, the term "insect pest" specifically includes coleopteran insect pests. In some examples, the term "insect pest" specifically refers to a coleopteran pest in the genus *Diabrotica* selected from a list comprising *D. v. virgifera* LeConte (WCR); *D. barberi* Smith and Lawrence (NCR); *D. u. howardi* (SCR); *D. v. zeae* (MCR); *D. balteata* LeConte; *D. u. tenella; D. speciosa* Germar, and *D. u. undecimpunctata* Mannerheim.

Isolated: An "isolated" biological component (such as a nucleic acid or protein) has been substantially separated, produced apart from, or purified away from other biological components in the cell of the organism in which the component naturally occurs (*i.e.*, other chromosomal and extra-chromosomal DNA and RNA, and proteins), while effecting a chemical or functional change in the component *(e.g.,* a nucleic acid may be isolated from a chromosome by breaking chemical bonds connecting the nucleic acid to the remaining DNA in the chromosome). Nucleic acid molecules and proteins that have been "isolated" include nucleic acid molecules and proteins purified by standard purification methods. The term also embraces nucleic acids and proteins prepared by recombinant expression in a host cell, as well as chemically-synthesized nucleic acid molecules, proteins, and peptides.

Nucleic acid molecule: As used herein, the term "nucleic acid molecule" may refer to a polymeric form of nucleotides, which may include both sense and anti-sense strands of RNA, cDNA, gDNA, and synthetic forms and mixed polymers of the above. A nucleotide or nucleobase may refer to a ribonucleotide, deoxyribonucleotide, or a modified form of either type of nucleotide. A "nucleic acid molecule" as used herein is synonymous with "nucleic acid" and "polynucleotide." A nucleic acid molecule is usually at least 10 bases in length, unless otherwise specified. By convention, the nucleotide sequence of a nucleic acid molecule is read from the 5' to the 3' end of the molecule. The "complement" of a nucleic acid molecule refers to a polynucleotide having nucleobases that may form base pairs with the nucleobases of the nucleic acid molecule (*i.e.,* A-T/U, and G-C).

Some embodiments include nucleic acids comprising a template DNA that is transcribed into a RNA molecule that is the complement of a mRNA molecule. In these embodiments, the complement of the nucleic acid transcribed into the mRNA molecule is present in the 5' to 3' orientation, such that RNA polymerase (which transcribes DNA in the 5' to 3' direction) will transcribe a nucleic acid from the complement that can hybridize to the mRNA molecule. Unless explicitly stated otherwise, or it is clear to be otherwise from the context, the term "complement" therefore refers to a polynucleotide having nucleobases, from 5' to 3', that may form base pairs with the nucleobases of a reference nucleic acid. Similarly, unless it is explicitly stated to be otherwise (or it is clear to be otherwise from the context), the "reverse complement" of a nucleic acid refers to the complement in reverse orientation. The foregoing is demonstrated in the following illustration:

| | |
|---|---|
| ATGATGATG | polynucleotide |
| TACTACTAC | "complement" of the polynucleotide |
| CATCATCAT | "reverse complement" of the polynucleotide |

Some embodiments of the invention may include hairpin RNA-forming RNAi molecules. In these RNAi molecules, both the complement of a nucleic acid to be targeted by RNA interference and the reverse complement may be found in the same molecule, such that the single-stranded RNA molecule may "fold over" and hybridize to itself over a region comprising the complementary and reverse complementary polynucleotides.

"Nucleic acid molecules" include all polynucleotides, for example: single- and double-stranded forms of DNA; single-stranded forms of RNA; and double-stranded forms of RNA (dsRNA). The term "nucleotide sequence" or "nucleic acid sequence" refers to both the sense and antisense strands of a nucleic acid as either individual single strands or in the duplex. The term "ribonucleic acid" (RNA) is inclusive of iRNA (inhibitory RNA), dsRNA (double stranded RNA), siRNA (small interfering RNA), shRNA (small hairpin RNA), mRNA (messenger RNA), miRNA (micro-RNA), hpRNA (hairpin RNA), tRNA (transfer RNAs, whether charged or discharged with a corresponding acylated amino acid), and cRNA (complementary RNA). The term "deoxyribonucleic acid" (DNA) is inclusive of cDNA, gDNA, and DNA-RNA hybrids. The terms " polynucleotide" and "nucleic acid," and "fragments" thereof will be understood by those in the art as a term that includes both gDNAs, ribosomal RNAs, transfer RNAs, messenger RNAs, operons, and smaller engineered polynucleotides that encode or may be adapted to encode, peptides, polypeptides, or proteins.

Oligonucleotide: An oligonucleotide is a short nucleic acid polymer. Oligonucleotides may be formed by cleavage of longer nucleic acid segments, or by polymerizing individual nucleotide precursors. Automated synthesizers allow the synthesis of oligonucleotides up to several hundred bases in length. Because oligonucleotides may bind to a complementary nucleic acid, they may be used as probes for detecting DNA or RNA. Oligonucleotides composed of DNA (oligodeoxyribonucleotides) may be used in PCR, a technique for the amplification of DNAs. In PCR, the oligonucleotide is typically referred to as a "primer," which allows a DNA polymerase to extend the oligonucleotide and replicate the complementary strand.

A nucleic acid molecule may include either or both naturally occurring and modified nucleotides linked together by naturally occurring and/or non-naturally occurring nucleotide linkages. Nucleic acid molecules may be modified chemically or biochemically, or may contain non-natural or derivatized nucleotide bases, as will be readily appreciated by those of skill in the art. Such modifications include, for example, labels, methylation, substitution of one or more of the naturally occurring nucleotides with an analog, internucleotide modifications (*e.g.*, uncharged linkages: for example, methyl phosphonates, phosphotriesters, phosphoramidates, carbamates, *etc.*; charged linkages: for example, phosphorothioates, phosphorodithioates, *etc.*; pendent moieties: for example, peptides; intercalators: for example, acridine, psoralen, *etc.*; chelators; alkylators; and modified linkages: for example, alpha anomeric nucleic acids, *etc.*). The term "nucleic acid molecule" also includes any topological conformation, including single-stranded, double-stranded, partially duplexed, triplexed, hairpinned, circular, and padlocked conformations.

As used herein with respect to DNA, the term "coding polynucleotide," "structural polynucleotide," or "structural nucleic acid molecule" refers to a polynucleotide that is ultimately translated into a polypeptide, *via* transcription and mRNA, when placed under the control of appropriate regulatory elements. With respect to RNA, the term "coding polynucleotide" refers to a polynucleotide that is translated into a peptide, polypeptide, or protein. The boundaries of a coding polynucleotide are determined by a translation start codon at the 5'-terminus and a translation stop codon at the 3'-terminus. Coding polynucleotides include, but are not limited to: gDNA; cDNA; EST; and recombinant polynucleotides.

As used herein, "transcribed non-coding polynucleotide" refers to segments of mRNA molecules such as 5'UTR, 3'UTR, and intron segments that are not translated into a peptide, polypeptide, or protein. Further, "transcribed non-coding polynucleotide" refers to a nucleic acid that is transcribed into a RNA that functions in the cell, for example, structural RNAs *(e.g.,* ribosomal RNA (rRNA) as exemplified by 5S rRNA, 5.8S rRNA, 16S rRNA, 18S rRNA, 23S rRNA, and 28S rRNA, and the like); transfer RNA (tRNA); and snRNAs such as U4, U5, U6, and the like. Transcribed non-coding polynucleotides also include, for example and without limitation, small RNAs (sRNA), which term is often used to describe small bacterial non-coding RNAs; small nucleolar RNAs (snoRNA); microRNAs; small interfering RNAs (siRNA); Piwi-interacting RNAs (piRNA); and long non-coding RNAs. Further still, "transcribed non-coding polynucleotide" refers to a polynucleotide that may natively exist as an intragenic "spacer" in a nucleic acid and which is transcribed into a RNA molecule.

Lethal RNA interference: As used herein, the term "lethal RNA interference" refers to RNA interference that results in death or a reduction in viability of the subject individual to which, for example, a dsRNA, miRNA, siRNA, shRNA, and/or hpRNA is delivered.

Genome: As used herein, the term "genome" refers to chromosomal DNA found within the nucleus of a cell, and also refers to organelle DNA found within subcellular components of the cell. In some embodiments of the invention, a DNA molecule may be introduced into a plant cell, such that the DNA molecule is integrated into the genome of the plant cell. In these and further embodiments, the DNA molecule may be either integrated into the nuclear DNA of the plant cell, or integrated into the DNA of the chloroplast or mitochondrion of the plant cell. The term "genome," as it applies to bacteria, refers to both the chromosome and plasmids within the bacterial cell. In some embodiments of the invention, a DNA molecule may be introduced into a bacterium such that the DNA molecule is integrated into the genome of the bacterium. In these and further embodiments, the DNA molecule may be either chromosomally-integrated or located as or in a stable plasmid.

Sequence identity: The term "sequence identity" or "identity," as used herein in the context of two polynucleotides or polypeptides, refers to the residues in the sequences of the two molecules that are the same when aligned for maximum correspondence over a specified comparison window.

As used herein, the term "percentage of sequence identity" may refer to the value determined by comparing two optimally aligned sequences (*e.g.*, nucleic acid sequences or polypeptide sequences) of a molecule over a comparison window, wherein the portion of the sequence in the comparison window may comprise additions or deletions (*i.e.,* gaps) as compared to the reference sequence (which does not comprise additions or deletions) for optimal alignment of the two sequences. The percentage is calculated by determining the number of positions at which the identical nucleotide or amino acid residue occurs in both sequences to yield the number of matched positions, dividing the number of matched positions by the total number of positions in the comparison window, and multiplying the result by 100 to yield the percentage of sequence identity. A sequence that is identical at every position in comparison to a reference sequence is said to be 100% identical to the reference sequence, and vice-versa.

Methods for aligning sequences for comparison are well-known in the art. Various programs and alignment algorithms are described in, for example: Smith and Waterman (1981) Adv. Appl. Math. 2:482; Needleman and Wunsch (1970) J. Mol. Biol. 48:443; Pearson and Lipman (1988) Proc. Natl. Acad. Sci. U.S.A. 85:2444; Higgins and Sharp (1988) Gene 73:237-44; Higgins and Sharp (1989) CABIOS 5:151-3; Corpet et al. (1988) Nucleic Acids Res. 16:10881-90; Huang et al. (1992) Comp. Appl. Biosci. 8:155-65; Pearson et al. (1994) Methods Mol. Biol. 24:307-31; and Tatiana et al. (1999) FEMS Microbiol. Lett. 174:247-50. A detailed consideration of sequence alignment methods and homology calculations can be found in, *e.g.,* Altschul et al. (1990) J. Mol. Biol.215:403-10.

The National Center for Biotechnology Information (NCBI) Basic Local Alignment Search Tool (BLAST™; Altschul *et al.* (1990)) is available from several sources, including the National Center for Biotechnology Information (Bethesda, MD), and on the internet, for use in connection with several sequence analysis programs. A description of how to determine sequence identity using this program is available on the internet under the "help" section for BLAST™. For comparisons of nucleic acid sequences, the "Blast 2 sequences" function of the BLAST™ (Blastn) program may be employed using the default BLOSUM62 matrix set to default parameters. Nucleic acids with even greater sequence similarity to the sequences of the reference polynucleotides will show increasing percentage identity when assessed by this method.

Specifically hybridizable/Specifically complementary: As used herein, the terms "Specifically hybridizable" and "Specifically complementary" are terms that indicate a sufficient degree of complementarity such that stable and specific binding occurs between the nucleic acid molecule and a target nucleic acid molecule. Hybridization between two nucleic acid molecules involves the formation of an anti-parallel alignment between the nucleobases of the two nucleic acid molecules. The two molecules are then able to form hydrogen bonds with corresponding bases on the opposite strand to form a duplex molecule that, if it is sufficiently stable, is detectable using methods well known in the art. A polynucleotide need not be 100% complementary to its target nucleic acid to be specifically hybridizable. However, the amount of complementarity that must exist for hybridization to be specific is a function of the hybridization conditions used.

Hybridization conditions resulting in particular degrees of stringency will vary depending upon the nature of the hybridization method of choice and the composition and length of the hybridizing nucleic acids. Generally, the temperature of hybridization and the ionic strength (especially the $Na^+$ and/or $Mg^{++}$ concentration) of the hybridization buffer will determine the stringency of hybridization, though wash times also influence stringency. Calculations regarding hybridization conditions required for attaining particular degrees of stringency are known to those of ordinary skill in the art, and are discussed, for example, in Sambrook et al. (ed.) Molecular Cloning: A Laboratory Manual, 2nd ed., vol. 1-3, Cold Spring Harbor Laboratory Press, Cold Spring Harbor, NY, 1989, chapters 9 and 11; and Hames and Higgins (eds.) Nucleic Acid Hybridization, IRL Press, Oxford, 1985. Further detailed instruction and guidance with regard to the hybridization of nucleic acids may be found, for example, in Tijssen, "Overview of principles of hybridization and the strategy of nucleic acid probe assays," in Laboratory Techniques in Biochemistry and Molecular Biology- Hybridization with Nucleic Acid Probes, Part I, Chapter 2, Elsevier, NY, 1993; and Ausubel et al., Eds., Current Protocols in Molecular Biology, Chapter 2, Greene Publishing and Wiley-Interscience, NY, 1995.

As used herein, "stringent conditions" encompass conditions under which hybridization will only occur if there is less than 20% mismatch between the sequence of the hybridization molecule and a homologous polynucleotide within the target nucleic acid molecule. "Stringent conditions" include further particular levels of stringency. Thus, as used herein, "moderate stringency" conditions are those under which molecules with more than 20% sequence mismatch will not hybridize; conditions of "high stringency" are those under which sequences with more than 10% mismatch will not hybridize; and conditions of "very high stringency" are those under which sequences with more than 5% mismatch will not hybridize.

The following are representative, non-limiting hybridization conditions. High Stringency condition (detects polynucleotides that share at least 90% sequence identity): Hybridization in 5x SSC buffer at 65 °C for 16 hours; wash twice in 2x SSC buffer at room temperature for 15 minutes each; and wash twice in 0.5x SSC buffer at 65 °C for 20 minutes each.

Moderate Stringency condition (detects polynucleotides that share at least 80% sequence identity): Hybridization in 5x-6x SSC buffer at 65-70 °C for 16-20 hours; wash twice in 2x SSC buffer at room temperature for 5-20 minutes each; and wash twice in 1x SSC buffer at 55-70 °C for 30 minutes each.

Non-stringent control condition (polynucleotides that share at least 50% sequence identity will hybridize): Hybridization in 6x SSC buffer at room temperature to 55 °C for 16-20 hours; wash at least twice in 2x-3x SSC buffer at room temperature to 55 °C for 20-30 minutes each.

As used herein, the term "substantially homologous" or "substantial homology," with regard to a nucleic acid, refers to a polynucleotide having contiguous nucleobases that hybridize under stringent conditions to the reference nucleic acid. For example, nucleic acids that are substantially homologous to a reference nucleic acid of any of SEQ ID NOs:1, 3, and 5-8 are those nucleic acids that hybridize under stringent conditions (*e.g.*, the Moderate Stringency conditions set forth, *supra*) to the reference nucleic acid. Substantially homologous polynucleotides may have at least 80% sequence identity. For example, substantially homologous polynucleotides may have from about 80% to 100% sequence identity, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. The property of substantial homology is closely related to specific hybridization. For example, substantially homologous polynucleotides may have from about 80% to 100% sequence identity to SEQ ID NO: 1, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100% to SEQ ID NO:1. For example, substantially homologous polynucleotides may have from about 80% to 100% sequence identity to SEQ ID NO:3, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100% to SEQ ID NO:3. For example, substantially homologous polynucleotides may have from about 80% to 100% sequence identity to SEQ ID NO:5, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100% to SEQ ID NO:5. For example, substantially homologous polynucleotides may have from about 80% to 100% sequence identity to SEQ ID NO:6, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100% to SEQ ID NO:6. For example, substantially homologous polynucleotides may have from about 80% to 100% sequence identity to SEQ ID NO:7, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100% to SEQ ID NO:7. For example, substantially homologous polynucleotides may have from about 80% to 100% sequence identity to SEQ ID NO:8, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100% to SEQ ID NO:8. For example, a nucleic acid molecule is specifically hybridizable when there is a sufficient degree of complementarity to avoid non-specific binding of the nucleic acid to non-target polynucleotides under conditions where specific binding is desired, for example, under stringent hybridization conditions.

As used herein, the term "ortholog" refers to a gene in two or more species that has evolved from a common ancestral nucleic acid, and may retain the same function in the two or more species.

As used herein, two nucleic acid molecules are said to exhibit "complete complementarity" when every nucleotide of a polynucleotide read in the 5' to 3' direction is complementary to every nucleotide of the other polynucleotide when read in the 3' to 5' direction. A polynucleotide that is complementary to a reference polynucleotide will exhibit a sequence identical to the reverse complement of the reference polynucleotide. These terms and descriptions are well defined in the art and are easily understood by those of ordinary skill in the art.

Operably linked: A first polynucleotide is operably linked with a second polynucleotide when the first polynucleotide is in a functional relationship with the second polynucleotide. When recombinantly produced, operably linked polynucleotides are generally contiguous, and, where necessary to join two protein-coding regions, in the same reading frame *(e.g.,* in a translationally fused ORF). However, nucleic acids need not be contiguous to be operably linked.

The term, "operably linked," when used in reference to a regulatory genetic element and a coding polynucleotide, means that the regulatory element affects the expression of the linked coding polynucleotide. "Regulatory elements," or "control elements," refer to polynucleotides that influence the timing and level/amount of transcription, RNA processing or stability, or translation of the associated coding polynucleotide. Regulatory elements may include promoters; translation leaders; introns; enhancers; stem-loop structures; repressor binding polynucleotides; polynucleotides with a termination sequence; polynucleotides with a polyadenylation recognition sequence; *etc.* Particular regulatory elements may be located upstream and/or downstream of a coding polynucleotide operably linked thereto. Also, particular regulatory elements operably linked to a coding polynucleotide may be located on the associated complementary strand of a double-stranded nucleic acid molecule.

Promoter: As used herein, the term "promoter" refers to a region of DNA that may be upstream from the start of transcription, and that may be involved in recognition and binding of RNA polymerase and other proteins to initiate transcription. A promoter may be operably linked to a coding polynucleotide for expression in a cell, or a promoter may be operably linked to a polynucleotide encoding a signal peptide which may be operably linked to a coding polynucleotide for expression in a cell. A "plant promoter" may be a promoter capable of initiating transcription in plant cells. Examples of promoters under developmental control include promoters that preferentially initiate transcription in certain tissues, such as leaves, roots, seeds, fibers, xylem vessels, tracheids, or sclerenchyma. Such promoters are referred to as "tissue-preferred". Promoters which initiate transcription only in certain tissues are referred to as "tissue-specific". A "cell type-specific" promoter primarily drives expression in certain cell types in one or more organs, for example, vascular cells in roots or leaves. An "inducible" promoter may be a promoter which may be under environmental control. Examples of environmental conditions that may initiate transcription by inducible promoters include anaerobic conditions and the presence of light. Tissue-specific, tissue-preferred, cell type specific, and inducible promoters constitute the class of "non-constitutive" promoters. A "constitutive" promoter is a promoter which may be active under most environmental conditions or in most tissue or cell types.

Any inducible promoter can be used in some embodiments of the invention. *See* Ward et al. (1993) Plant Mol. Biol. 22:361-366. With an inducible promoter, the rate of transcription increases in response to an inducing agent. Exemplary inducible promoters include, but are not limited to: Promoters from the ACEI system that respond to copper; *In2* gene from maize that responds to benzenesulfonamide herbicide safeners; Tet repressor from Tn10; and the inducible promoter from a steroid hormone gene, the transcriptional activity of which may be induced by a glucocorticosteroid hormone (Schena et al. (1991) Proc. Natl. Acad. Sci. USA 88:0421).

Exemplary constitutive promoters include, but are not limited to: Promoters from plant viruses, such as the 35S promoter from Cauliflower Mosaic Virus (CaMV); promoters from rice actin genes; ubiquitin promoters; pEMU; MAS; maize H3 histone promoter; and the ALS promoter, *Xba1*/*NcoI* fragment 5' to the *Brassica napus ALS3* structural gene (or a polynucleotide similar to said *Xba1*/*NcoI* fragment) (International PCT Publication No. WO96/30530).

Additionally, any tissue-specific or tissue-preferred promoter may be utilized in some embodiments of the invention. Plants transformed with a nucleic acid molecule comprising a coding polynucleotide operably linked to a tissue-specific promoter may produce the product of the coding polynucleotide exclusively, or preferentially, in a specific tissue. Exemplary tissue-specific or tissue-preferred promoters include, but are not limited to: A seed-preferred promoter, such as that from the phaseolin gene; a leaf-specific and light-induced promoter such as that from *cab* or *rubisco*; an anther-specific promoter such as that from *LAT52*; a pollen-specific promoter such as that from *Zm13*; and a microspore-preferred promoter such as that from *apg.*

Transformation: As used herein, the term "transformation" or "transduction" refers to the transfer of one or more nucleic acid molecule(s) into a cell. A cell is "transformed" by a nucleic acid molecule transduced into the cell when the nucleic acid molecule becomes stably replicated by the cell, either by incorporation of the nucleic acid molecule into the cellular genome, or by episomal replication. As used herein, the term "transformation" encompasses all techniques by which a nucleic acid molecule can be introduced into such a cell. Examples include, but are not limited to: transfection with viral vectors; transformation with plasmid vectors; electroporation (Fromm et al. (1986) Nature 319:791-3); lipofection (Felgner et al. (1987) Proc. Natl. Acad. Sci. USA 84:7413-7); microinjection (Mueller et al. (1978) Cell 15:579-85); *Agrobacterium-mediated* transfer (Fraley et al. (1983) Proc. Natl. Acad. Sci. USA 80:4803-7); direct DNA uptake; and microprojectile bombardment (Klein et al. (1987) Nature 327:70).

Transgene: An exogenous nucleic acid. In some examples, a transgene may be a DNA that encodes one or both strand(s) of a RNA capable of forming a dsRNA molecule that comprises a polynucleotide that is complementary to a nucleic acid molecule found in a coleopteran pest. In further examples, a transgene may be an antisense polynucleotide, wherein expression of the antisense polynucleotide inhibits expression of a target nucleic acid, thereby producing an RNAi phenotype. In still further examples, a transgene may be a gene *(e.g.,* a herbicide-tolerance gene, a gene encoding an industrially or pharmaceutically useful compound, or a gene encoding a desirable agricultural trait). In these and other examples, a transgene may contain regulatory elements operably linked to a coding polynucleotide of the transgene *(e.g.,* a promoter).

Vector: A nucleic acid molecule as introduced into a cell, for example, to produce a transformed cell. A vector may include genetic elements that permit it to replicate in the host cell, such as an origin of replication. Examples of vectors include, but are not limited to: a plasmid; cosmid; bacteriophage; or virus that carries exogenous DNA into a cell. A vector may also include one or more genes, including ones that produce antisense molecules, and/or selectable marker genes and other genetic elements known in the art. A vector may transduce, transform, or infect a cell, thereby causing the cell to express the nucleic acid molecules and/or proteins encoded by the vector. A vector optionally includes materials to aid in achieving entry of the nucleic acid molecule into the cell *(e.g.,* a liposome, protein coating, *etc.*).

Yield: A stabilized yield of about 100% or greater relative to the yield of check varieties in the same growing location growing at the same time and under the same conditions. In particular embodiments, "improved yield" or "improving yield" means a cultivar having a stabilized yield of 105% or greater relative to the yield of check varieties in the same growing location containing significant densities of the coleopteran pests that are injurious to that crop growing at the same time and under the same conditions, which are targeted by the compositions and methods herein.

Unless specifically indicated or implied, the terms "a," "an," and "the" signify "at least one," as used herein.

Unless otherwise specifically explained, all technical and scientific terms used herein have the same meaning as commonly understood by those of ordinary skill in the art to which this disclosure belongs. Definitions of common terms in molecular biology can be found in, for example, Lewin's Genes X, Jones & Bartlett Publishers, 2009 (ISBN 10 0763766321); Krebs et al. (eds.), The Encyclopedia of Molecular Biology, Blackwell Science Ltd., 1994 (ISBN 0-632-02182-9); and Meyers R.A. (ed.), Molecular Biology and Biotechnology: A Comprehensive Desk Reference, VCH Publishers, Inc., 1995 (ISBN 1-56081-569-8). All percentages are by weight and all solvent mixture proportions are by volume unless otherwise noted. All temperatures are in degrees Celsius.

### *IV. Nucleic Acid Molecules Comprising an Insect Pest Sequence*

#### A. Overview

Described herein are nucleic acid molecules useful for the control of insect pests. In some examples, the insect pest is a coleopteran insect pest (*e.g.*, a coleopteran pest in the genus *Diabrotica*). Described nucleic acid molecules include target polynucleotides *(e.g.,* native genes, and non-coding polynucleotides), dsRNAs, siRNAs, shRNAs, hpRNAs, and miRNAs. For example, dsRNA, siRNA, miRNA, shRNA, and/or hpRNA molecules are described in some embodiments that may be specifically complementary to all or part of one or more native nucleic acids in a coleopteran pest. In these and further embodiments, the native nucleic acid(s) may be one or more target gene(s), the product of which may be, for example and without limitation: involved in a metabolic process or involved in larval development. Nucleic acid molecules described herein, when introduced into a cell comprising at least one native nucleic acid(s) to which the nucleic acid molecules are specifically complementary, may initiate RNAi in the cell, and consequently reduce or eliminate expression of the native nucleic acid(s). In some examples, reduction or elimination of the expression of a target gene by a nucleic acid molecule specifically complementary thereto may result in reduction or cessation of growth, development, and/or feeding in the pest.

In some embodiments, at least one target gene in an insect pest may be selected, wherein the target gene comprises a *rpl1* polynucleotide. In particular examples, a target gene in a coleopteran pest is selected, wherein the target gene comprises a polynucleotide selected from among SEQ ID NOs:1, 3, and 5-8.

In some embodiments, a target gene may be a nucleic acid molecule comprising a polynucleotide that can be reverse translated *in silico* to a polypeptide comprising a contiguous amino acid sequence that is at least about 85% identical *(e.g.,* at least 84%, 85%, about 90%, about 95%, about 96%, about 97%, about 98%, about 99%, about 100%, or 100% identical) to the amino acid sequence of a protein product of an *rpl1* polynucleotide. A target gene may be any *rpl1* polynucleotide in an insect pest, the post-transcriptional inhibition of which has a deleterious effect on the growth, survival, and/or viability of the pest, for example, to provide a protective benefit against the pest to a plant. In particular examples, a target gene is a nucleic acid molecule comprising a polynucleotide that can be reverse translated *in silico* to a polypeptide comprising a contiguous amino acid sequence that is at least about 85% identical, about 90% identical, about 95% identical, about 96% identical, about 97% identical, about 98% identical, about 99% identical, about 100% identical, or 100% identical to the amino acid sequence of SEQ ID NO:2 or at least about 85% identical, about 90% identical, about 95% identical, about 96% identical, about 97% identical, about 98% identical, about 99% identical, about 100% identical, or 100% identical to the amino acid sequence of SEQ ID NO:4.

Provided according to the invention are DNAs, the expression of which results in a RNA molecule comprising a polynucleotide that is specifically complementary to all or part of a native RNA molecule that is encoded by a coding polynucleotide in an insect (*e.g.,* coleopteran) pest. In some embodiments, after ingestion of the expressed RNA molecule by an insect pest, down-regulation of the coding polynucleotide in cells of the pest may be obtained. In particular embodiments, down-regulation of the coding polynucleotide in cells of the pest may be obtained. In particular embodiments, down-regulation of the coding polynucleotide in cells of the insect pest results in a deleterious effect on the growth and/or development of the pest.

In some embodiments, target polynucleotides include transcribed non-coding RNAs, such as 5'UTRs; 3'UTRs; spliced leaders; introns; outrons (*e.g.,* 5'UTR RNA subsequently modified in trans splicing); donatrons (*e.g.,* non-coding RNA required to provide donor sequences for *trans* splicing); and other non-coding transcribed RNA of target insect pest genes. Such polynucleotides may be derived from both mono-cistronic and poly-cistronic genes.

Thus, also described herein in connection with some embodiments are iRNA molecules (*e.g.,* dsRNAs, siRNAs, miRNAs, shRNAs, and hpRNAs) that comprise at least one polynucleotide that is specifically complementary to all or part of a target nucleic acid in an insect (*e.g.,* coleopteran) pest. In some embodiments an iRNA molecule may comprise polynucleotide(s) that are complementary to all or part of a plurality of target nucleic acids; for example, 2, 3, 4, 5, 6, 7, 8, 9, 10, or more target nucleic acids. In particular embodiments, an iRNA molecule may be produced *in vitro* or *in vivo* by a genetically-modified organism, such as a plant or bacterium. Also disclosed are cDNAs that may be used for the production of dsRNA molecules, siRNA molecules, miRNA molecules, shRNA molecules, and/or hpRNA molecules that are specifically complementary to all or part of a target nucleic acid in an insect pest. Further described are recombinant DNA constructs for use in achieving stable transformation of particular host targets. Transformed host targets may express effective levels of dsRNA, siRNA, miRNA, shRNA, and/or hpRNA molecules from the recombinant DNA constructs. Therefore, also described is a plant transformation vector comprising at least one polynucleotide operably linked to a heterologous promoter functional in a plant cell, wherein expression of the polynucleotide(s) results in a RNA molecule comprising a string of contiguous nucleobases that is specifically complementary to all or part of a target nucleic acid in an insect pest.

In particular examples, nucleic acid molecules useful for the control of insect *(e.g.,* coleopteran) pests may include: all or part of a native nucleic acid isolated from *Diabrotica* comprising a *rpl1* polynucleotide *(e.g.,* any of SEQ ID NOs:1, 3, and 5-8); DNAs that when expressed result in a RNA molecule comprising a polynucleotide that is specifically complementary to all or part of a native RNA molecule that is encoded by *rpl1*; iRNA molecules *(e.g.,* dsRNAs, siRNAs, miRNAs, shRNAs, and hpRNAs) that comprise at least one polynucleotide that is specifically complementary to all or part of *rpl1*; cDNAs that may be used for the production of dsRNA molecules, siRNA molecules, miRNA molecules, shRNA molecules, and/or hpRNA molecules that are specifically complementary to all or part of *rpl1*; and recombinant DNA constructs for use in achieving stable transformation of particular host targets, wherein a transformed host target comprises one or more of the foregoing nucleic acid molecules.

### B. Nucleic Acid Molecules

The present invention provides, *inter alia*, iRNA *(e.g.,* dsRNA, siRNA, miRNA, shRNA, and hpRNA) molecules that inhibit target gene expression in a cell, tissue, or organ of an insect (*e.g.,* coleopteran) pest; and DNA molecules capable of being expressed as an iRNA molecule in a cell or microorganism to inhibit target gene expression in a cell, tissue, or organ of an insect pest.

Some embodiments of the invention provide an isolated nucleic acid molecule comprising at least one *(e.g.,* one, two, three, or more) polynucleotide(s) selected from the group consisting of: SEQ ID NOs:1 and 3; the complement of SEQ ID NO:1 or 3; a fragment of at least 15 contiguous nucleotides of SEQ ID NO:1 or 3 *(e.g.,* any of SEQ ID NOs:5-8); the complement of a fragment of at least 15 contiguous nucleotides of SEQ ID NO: 1 or 3; a native coding polynucleotide of a *Diabrotica* organism *(e.g.,* WCR) comprising any of SEQ ID NOs:5-8; the complement of a native coding polynucleotide of a *Diabrotica* organism comprising any of SEQ ID NOs:5-8; a fragment of at least 15 contiguous nucleotides of a native coding polynucleotide of a *Diabrotica* organism comprising any of SEQ ID NOs:5-8; and the complement of a fragment of at least 15 contiguous nucleotides of a native coding polynucleotide of a *Diabrotica* organism comprising any of SEQ ID NOs:5-8.

In particular embodiments, contact with or uptake by an insect (*e.g.,* coleopteran) pest of an iRNA transcribed from the isolated polynucleotide inhibits the growth, development, and/or feeding of the pest.

In some embodiments, an isolated nucleic acid molecule of the invention may comprise at least one *(e.g.,* one, two, three, or more) polynucleotide(s) selected from the group consisting of: SEQ ID NO:75; the complement of SEQ ID NO:75; SEQ ID NO:76; the complement of SEQ ID NO:76; SEQ ID NO:77; the complement of SEQ ID NO:77; SEQ ID NO:78; the complement of SEQ ID NO:78; SEQ ID NO:79; the complement of SEQ ID NO:79; SEQ ID NO:80; the complement of SEQ ID NO:80; a fragment of at least 15 contiguous nucleotides of any of SEQ ID NOs:75-80, a fragment of at least 15 contiguous nucleotides of any of SEQ ID NOs:75, a fragment of at least 15 contiguous nucleotides of any of SEQ ID NOs:76, a fragment of at least 15 contiguous nucleotides of any of SEQ ID NOs:77, a fragment of at least 15 contiguous nucleotides of any of SEQ ID NOs:78, a fragment of at least 15 contiguous nucleotides of any of SEQ ID NOs:79, or a fragment of at least 15 contiguous nucleotides of any of SEQ ID NOs:80; the complement of a fragment of at least 15 contiguous nucleotides of any of SEQ ID NOs:75-80, the complement of a fragment of at least 15 contiguous nucleotides of any of SEQ ID NOs:75, the complement of a fragment of at least 15 contiguous nucleotides of any of SEQ ID NOs:76, the complement of a fragment of at least 15 contiguous nucleotides of any of SEQ ID NOs:77, the complement of a fragment of at least 15 contiguous nucleotides of any of SEQ ID NOs:78, the complement of a fragment of at least 15 contiguous nucleotides of any of SEQ ID NOs:79, or the complement of a fragment of at least 15 contiguous nucleotides of any of SEQ ID NOs:80; a native coding polynucleotide of a *Diabrotica* organism comprising any of SEQ ID NOs:77-80; the complement of a native coding polynucleotide of a *Diabrotica* organism comprising any of SEQ ID NOs:77-80; a fragment of at least 15 contiguous nucleotides of a native coding polynucleotide of a *Diabrotica* organism comprising any of SEQ ID NOs:77-80, a fragment of at least 15 contiguous nucleotides of a native coding polynucleotide of a *Diabrotica* organism comprising any of SEQ ID NOs:77, a fragment of at least 15 contiguous nucleotides of a native coding polynucleotide of a *Diabrotica* organism comprising any of SEQ ID NOs:78, a fragment of at least 15 contiguous nucleotides of a native coding polynucleotide of a *Diabrotica* organism comprising any of SEQ ID NOs:79, or a fragment of at least 15 contiguous nucleotides of a native coding polynucleotide of a *Diabrotica* organism comprising any of SEQ ID NOs:80; and the complement of a fragment of at least 15 contiguous nucleotides of a native coding polynucleotide of a *Diabrotica* organism comprising any of SEQ ID NOs:77-80, and the complement of a fragment of at least 15 contiguous nucleotides of a native coding polynucleotide of a *Diabrotica* organism comprising any of SEQ ID NOs:77, and the complement of a fragment of at least 15 contiguous nucleotides of a native coding polynucleotide of a *Diabrotica* organism comprising any of SEQ ID NOs:78, and the complement of a fragment of at least 15 contiguous nucleotides of a native coding polynucleotide of a *Diabrotica* organism comprising any of SEQ ID NOs:79, or and the complement of a fragment of at least 15 contiguous nucleotides of a native coding polynucleotide of a *Diabrotica* organism comprising any of SEQ ID NOs:80.

In particular embodiments, contact with or uptake by a coleopteran pest of the isolated polynucleotide inhibits the growth, development, and/or feeding of the pest.

In certain embodiments, dsRNA molecules provided by the invention comprise polynucleotides complementary to a transcript from a target gene comprising either of SEQ ID NOs:1 and 3, and fragments thereof, the inhibition of which target gene in an insect pest results in the reduction or removal of a polypeptide or polynucleotide agent that is essential for the pest's growth, development, or other biological function. A selected polynucleotide may exhibit from about 80% to about 100% sequence identity to either of SEQ ID NOs:1 and 3; a contiguous fragment of SEQ ID NOs:1 and 3; and the complement of any of the foregoing. For example, a selected polynucleotide may exhibit about 79%; about 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to SEQ ID NOs:1 and/or about 79%; about 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to either of SEQ ID NOs:3; a contiguous fragment of either of SEQ ID NOs:1 and 3; and the complement of any of the foregoing.

In some embodiments, a DNA molecule capable of being expressed as an iRNA molecule in a cell or microorganism to inhibit target gene expression may comprise a single polynucleotide that is specifically complementary to all or part of a native polynucleotide found in one or more target insect pest species (*e.g.*, a coleopteran pest species), or the DNA molecule can be constructed as a chimera from a plurality of such specifically complementary polynucleotides.

In some embodiments, a nucleic acid molecule may comprise a first and a second polynucleotide separated by a "spacer." A spacer may be a region comprising any sequence of nucleotides that facilitates secondary structure formation between the first and second polynucleotides, where this is desired. In one embodiment, the spacer is part of a sense or antisense coding polynucleotide for mRNA. The spacer may alternatively comprise any combination of nucleotides or homologues thereof that are capable of being linked covalently to a nucleic acid molecule.

For example, in some embodiments, the DNA molecule may comprise a polynucleotide coding for one or more different iRNA molecules, wherein each of the different iRNA molecules comprises a first polynucleotide and a second polynucleotide, wherein the first and second polynucleotides are complementary to each other. The first and second polynucleotides may be connected within a RNA molecule by a spacer. The spacer may constitute part of the first polynucleotide or the second polynucleotide. Expression of a RNA molecule comprising the first and second nucleotide polynucleotides may lead to the formation of a dsRNA molecule, by specific intramolecular base-pairing of the first and second nucleotide polynucleotides. The first polynucleotide or the second polynucleotide may be substantially identical to a polynucleotide (*e.g.,* a target gene, or transcribed non-coding polynucleotide) native to an insect pest *(e.g.,* a coleopteran pest), a derivative thereof, or a complementary polynucleotide thereto.

dsRNA nucleic acid molecules comprise double strands of polymerized ribonucleotides, and may include modifications to either the phosphate-sugar backbone or the nucleoside. Modifications in RNA structure may be tailored to allow specific inhibition. In one embodiment, dsRNA molecules may be modified through an ubiquitous enzymatic process so that siRNA molecules may be generated. This enzymatic process may utilize an RNase III enzyme, such as DICER in eukaryotes, either *in vitro* or *in vivo. See* Elbashir et al. (2001) Nature 411:494-8; and Hamilton and Baulcombe (1999) Science 286(5441):950-2. DICER or functionally-equivalent RNase III enzymes cleave larger dsRNA strands and/or hpRNA molecules into smaller oligonucleotides (*e.g.,* siRNAs), each of which is about 19-25 nucleotides in length. The siRNA molecules produced by these enzymes have 2 to 3 nucleotide 3' overhangs, and 5' phosphate and 3' hydroxyl termini. The siRNA molecules generated by RNase III enzymes are unwound and separated into single-stranded RNA in the cell. The siRNA molecules then specifically hybridize with RNAs transcribed from a target gene, and both RNA molecules are subsequently degraded by an inherent cellular RNA-degrading mechanism. This process may result in the effective degradation or removal of the RNA encoded by the target gene in the target organism. The outcome is the post-transcriptional silencing of the targeted gene. In some embodiments, siRNA molecules produced by endogenous RNase III enzymes from heterologous nucleic acid molecules may efficiently mediate the down-regulation of target genes in insect pests.

In some embodiments, a nucleic acid molecule may include at least one non-naturally occurring polynucleotide that can be transcribed into a single-stranded RNA molecule capable of forming a dsRNA molecule *in vivo* through intermolecular hybridization. Such dsRNAs typically self-assemble, and can be provided in the nutrition source of an insect (*e.g.,* coleopteran) pest to achieve the post-transcriptional inhibition of a target gene. In these and further embodiments, a nucleic acid molecule may comprise two different non-naturally occurring polynucleotides, each of which is specifically complementary to a different target gene in an insect pest. When such a nucleic acid molecule is provided as a dsRNA molecule to, for example, a coleopteran pest, the dsRNA molecule inhibits the expression of at least two different target genes in the pest.

**C. Obtaining Nucleic Acid Molecules**

A variety of polynucleotides in insect (*e.g.,* coleopteran) pests may be used as targets for the design of nucleic acid molecules, such as iRNAs and DNA molecules encoding iRNAs. Selection of native polynucleotides is not, however, a straight-forward process. For example, only a small number of native polynucleotides in a coleopteran pest will be effective targets. It cannot be predicted with certainty whether a particular native polynucleotide can be effectively down-regulated by nucleic acid molecules of the invention, or whether down-regulation of a particular native polynucleotide will have a detrimental effect on the growth, viability, and/or development of an insect pest. The vast majority of native coleopteran pest polynucleotides, such as ESTs isolated therefrom (for example, the coleopteran pest polynucleotides listed in U.S. Patent 7,612,194), do not have a detrimental effect on the growth and/or viability of the pest. Neither is it predictable which of the native polynucleotides that may have a detrimental effect on an insect pest are able to be used in recombinant techniques for expressing nucleic acid molecules complementary to such native polynucleotides in a host plant and providing the detrimental effect on the pest upon feeding without causing harm to the host plant.

In some embodiments, nucleic acid molecules (*e.g.,* dsRNA molecules to be provided in the host plant of an insect (*e.g.,* coleopteran) pest) are selected to target cDNAs that encode proteins or parts of proteins essential for pest growth and/or development, such as polypeptides involved in metabolic or catabolic biochemical pathways, cell division, reproduction, energy metabolism, digestion, host plant recognition, and the like. As described herein, ingestion of compositions by a target pest organism containing one or more dsRNAs, at least one segment of which is specifically complementary to at least a substantially identical segment of RNA produced in the cells of the target pest organism, can result in the death or other inhibition of the target. A polynucleotide, either DNA or RNA, derived from an insect pest can be used to construct plant cells protected against infestation by the pests. The host plant of the coleopteran pest *(e.g., Z. mays),* for example, can be transformed to contain one or more polynucleotides derived from the coleopteran pest as provided herein. The polynucleotide transformed into the host may encode one or more RNAs that form into a dsRNA structure in the cells or biological fluids within the transformed host, thus making the dsRNA available if/when the pest forms a nutritional relationship with the transgenic host. This may result in the suppression of expression of one or more genes in the cells of the pest, and ultimately death or inhibition of its growth or development.

Thus, in some embodiments, a gene is targeted that is essentially involved in the growth and development of an insect (*e.g.,* coleopteran) pest. Other target genes for use in the present invention may include, for example, those that play important roles in pest viability, movement, migration, growth, development, infectivity, and establishment of feeding sites. A target gene may therefore be a housekeeping gene or a transcription factor. Additionally, a native insect pest polynucleotide for use in the present invention may also be derived from a homolog *(e.g.,* an ortholog), of a plant, viral, bacterial or insect gene, the function of which is known to those of skill in the art, and the polynucleotide of which is specifically hybridizable with a target gene in the genome of the target pest. Methods of identifying a homolog of a gene with a known nucleotide sequence by hybridization are known to those of skill in the art.

In some embodiments, the invention provides methods for obtaining a nucleic acid molecule comprising a polynucleotide for producing an iRNA (*e.g.,* dsRNA, siRNA, miRNA, shRNA, and hpRNA) molecule. One such embodiment comprises: (a) analyzing one or more target gene(s) for their expression, function, and phenotype upon dsRNA-mediated gene suppression in an insect (*e.g.,* coleopteran) pest; (b) probing a cDNA or gDNA library with a probe comprising all or a portion of a polynucleotide or a homolog thereof from a targeted pest that displays an altered *(e.g.,* reduced) growth or development phenotype in a dsRNA-mediated suppression analysis; (c) identifying a DNA clone that specifically hybridizes with the probe; (d) isolating the DNA clone identified in step (b); (e) sequencing the cDNA or gDNA fragment that comprises the clone isolated in step (d), wherein the sequenced nucleic acid molecule comprises all or a substantial portion of the RNA or a homolog thereof; and (f) chemically synthesizing all or a substantial portion of a gene, or an siRNA, miRNA, hpRNA, mRNA, shRNA, or dsRNA.

In further embodiments, a method for obtaining a nucleic acid fragment comprising a polynucleotide for producing a substantial portion of an iRNA (*e.g.,* dsRNA, siRNA, miRNA, shRNA, and hpRNA) molecule includes: (a) synthesizing first and second oligonucleotide primers specifically complementary to a portion of a native polynucleotide from a targeted insect (*e.g.,* coleopteran) pest; and (b) amplifying a cDNA or gDNA insert present in a cloning vector using the first and second oligonucleotide primers of step (a), wherein the amplified nucleic acid molecule comprises a substantial portion of a siRNA, miRNA, hpRNA, mRNA, shRNA, or dsRNA molecule.

Nucleic acids can be isolated, amplified, or produced by a number of approaches. For example, an iRNA (*e.g.,* dsRNA, siRNA, miRNA, shRNA, and hpRNA) molecule may be obtained by PCR amplification of a target polynucleotide (*e.g.,* a target gene or a target transcribed non-coding polynucleotide) derived from a gDNA or cDNA library, or portions thereof. DNA or RNA may be extracted from a target organism, and nucleic acid libraries may be prepared therefrom using methods known to those ordinarily skilled in the art. gDNA or cDNA libraries generated from a target organism may be used for PCR amplification and sequencing of target genes. A confirmed PCR product may be used as a template for *in vitro* transcription to generate sense and antisense RNA with minimal promoters. Alternatively, nucleic acid molecules may be synthesized by any of a number of techniques *(See, e.g.,* Ozaki et al. (1992) Nucleic Acids Research, 20: 5205-5214; and Agrawal et al. (1990) Nucleic Acids Research, 18: 5419-5423), including use of an automated DNA synthesizer (for example, a P.E. Biosystems, Inc. (Foster City, Calif.) model 392 or 394 DNA/RNA Synthesizer), using standard chemistries, such as phosphoramidite chemistry. *See, e.g.,* Beaucage et al. (1992) Tetrahedron, 48: 2223-2311; U.S. Patents 4,980,460, 4,725,677, 4,415,732, 4,458,066, and 4,973,679. Alternative chemistries resulting in non-natural backbone groups, such as phosphorothioate, phosphoramidate, and the like, can also be employed.

A RNA, dsRNA, siRNA, miRNA, shRNA, or hpRNA molecule of the present invention may be produced chemically or enzymatically by one skilled in the art through manual or automated reactions, or *in vivo* in a cell comprising a nucleic acid molecule comprising a polynucleotide encoding the RNA, dsRNA, siRNA, miRNA, shRNA, or hpRNA molecule. RNA may also be produced by partial or total organic synthesis- any modified ribonucleotide can be introduced by *in vitro* enzymatic or organic synthesis. A RNA molecule may be synthesized by a cellular RNA polymerase or a bacteriophage RNA polymerase *(e.g.,* T3 RNA polymerase, T7 RNA polymerase, and SP6 RNA polymerase). Expression constructs useful for the cloning and expression of polynucleotides are known in the art. *See, e.g.,* International PCT Publication No. WO97/32016; and U.S. Patents 5,593,874, 5,698,425, 5,712,135, 5,789,214, and 5,804,693. RNA molecules that are synthesized chemically or by *in vitro* enzymatic synthesis may be purified prior to introduction into a cell. For example, RNA molecules can be purified from a mixture by extraction with a solvent or resin, precipitation, electrophoresis, chromatography, or a combination thereof. Alternatively, RNA molecules that are synthesized chemically or by *in vitro* enzymatic synthesis may be used with no or a minimum of purification, for example, to avoid losses due to sample processing. The RNA molecules may be dried for storage or dissolved in an aqueous solution. The solution may contain buffers or salts to promote annealing, and/or stabilization of dsRNA molecule duplex strands.

In embodiments, a dsRNA molecule may be formed by a single self-complementary RNA strand or from two complementary RNA strands. dsRNA molecules may be synthesized either *in vivo* or *in vitro.* An endogenous RNA polymerase of the cell may mediate transcription of the one or two RNA strands *in vivo,* or cloned RNA polymerase may be used to mediate transcription *in vivo* or *in vitro.* Post-transcriptional inhibition of a target gene in an insect pest may be host-targeted by specific transcription in an organ, tissue, or cell type of the host *(e.g.,* by using a tissue-specific promoter); stimulation of an environmental condition in the host *(e.g.,* by using an inducible promoter that is responsive to infection, stress, temperature, and/or chemical inducers); and/or engineering transcription at a developmental stage or age of the host *(e.g.,* by using a developmental stage-specific promoter). RNA strands that form a dsRNA molecule, whether transcribed *in vitro* or *in vivo,* may or may not be polyadenylated, and may or may not be capable of being translated into a polypeptide by a cell's translational apparatus.

### D. Recombinant Vectors and Host Cell Transformation

In some embodiments, the invention also provides a DNA molecule for introduction into a cell *(e.g.,* a bacterial cell, a yeast cell, or a plant cell), wherein the DNA molecule comprises a polynucleotide that, upon expression to RNA and ingestion by an insect *(e.g.,* coleopteran) pest, achieves suppression of a target gene in a cell, tissue, or organ of the pest. Thus, some embodiments provide a recombinant nucleic acid molecule comprising a polynucleotide capable of being expressed as an iRNA (*e.g.,* dsRNA, siRNA, miRNA, shRNA, and hpRNA) molecule in a plant cell to inhibit target gene expression in an insect pest. In order to initiate or enhance expression, such recombinant nucleic acid molecules may comprise one or more regulatory elements, which regulatory elements may be operably linked to the polynucleotide capable of being expressed as an iRNA. Methods to express a gene suppression molecule in plants are known, and may be used to express a polynucleotide of the present invention. *See, e.g.,* International PCT Publication No. WO06/073727 and U.S. Patent Publication No. 2006/0200878 A1)

In specific embodiments, a recombinant DNA molecule of the invention may comprise a polynucleotide encoding a RNA that may form a dsRNA molecule. Such recombinant DNA molecules may encode RNAs that may form dsRNA molecules capable of inhibiting the expression of endogenous target gene(s) in an insect (*e.g.,* coleopteran) pest cell upon ingestion. In many embodiments, a transcribed RNA may form a dsRNA molecule that may be provided in a stabilized form; *e.g.,* as a hairpin and stem and loop structure.

In some embodiments, one strand of a dsRNA molecule may be formed by transcription from a polynucleotide which is substantially homologous to a polynucleotide selected from the group consisting of SEQ ID NOs:1 and 3; the complements of SEQ ID NOs:1 and 3; a fragment of at least 15 contiguous nucleotides of either of SEQ ID NOs:1 and 3 *(e.g.,* SEQ ID NOs:5-8); the complement of a fragment of at least 15 contiguous nucleotides of either of SEQ ID NOs:1 and 3; a native coding polynucleotide of a *Diabrotica* organism *(e.g.,* WCR) comprising any of SEQ ID NOs:5-8; the complement of a native coding polynucleotide of a *Diabrotica* organism comprising any of SEQ ID NOs:5-8; a fragment of at least 15 contiguous nucleotides of a native coding polynucleotide of a *Diabrotica* organism comprising any of SEQ ID NOs:5-8; the complement of a fragment of at least 15 contiguous nucleotides of a native coding polynucleotide of a *Diabrotica* organism comprising any of SEQ ID NOs:5-8.

In some embodiments, one strand of a dsRNA molecule may be formed by transcription from a polynucleotide that is substantially homologous to a polynucleotide selected from the group consisting of SEQ ID NOs:5-8; the complement of any of SEQ ID NOs:5-8; fragments of at least 15 contiguous nucleotides of either of SEQ ID NOs:1 and 3; and the complements of fragments of at least 15 contiguous nucleotides of either of SEQ ID NOs:1 and 3.

In particular embodiments, a recombinant DNA molecule encoding a RNA that may form a dsRNA molecule may comprise a coding region wherein at least two polynucleotides are arranged such that one polynucleotide is in a sense orientation, and the other polynucleotide is in an antisense orientation, relative to at least one promoter, wherein the sense polynucleotide and the antisense polynucleotide are linked or connected by a spacer of, for example, from about five (~5) to about one thousand (~1000) nucleotides. The spacer may form a loop between the sense and antisense polynucleotides. The sense polynucleotide or the antisense polynucleotide may be substantially homologous to a target gene *(e.g.,* a *rpl1* gene comprising any of SEQ ID NOs:1, 3, and 5-8) or fragment thereof. In some embodiments, however, a recombinant DNA molecule may encode a RNA that may form a dsRNA molecule without a spacer. In embodiments, a sense coding polynucleotide and an antisense coding polynucleotide may be different lengths.

Polynucleotides identified as having a deleterious effect on an insect pest or a plant-protective effect with regard to the pest may be readily incorporated into expressed dsRNA molecules through the creation of appropriate expression cassettes in a recombinant nucleic acid molecule of the invention. For example, such polynucleotides may be expressed as a hairpin with stem and loop structure by taking a first segment corresponding to a target gene polynucleotide *(e.g.,* a *rpl1* gene comprising any of SEQ ID NOs:1, 3, and 5-8, and fragments of any of the foregoing); linking this polynucleotide to a second segment spacer region that is not homologous or complementary to the first segment; and linking this to a third segment, wherein at least a portion of the third segment is substantially complementary to the first segment. Such a construct forms a stem and loop structure by intramolecular base-pairing of the first segment with the third segment, wherein the loop structure forms comprising the second segment. *See, e.g.,* U.S. Patent Publication Nos. 2002/0048814 and 2003/0018993; and International PCT Publication Nos. WO94/01550 and WO98/05770. A dsRNA molecule may be generated, for example, in the form of a double-stranded structure such as a stem-loop structure (*e.g.*, hairpin), whereby production of siRNA targeted for a native insect (*e.g.,* coleopteran) pest polynucleotide is enhanced by co-expression of a fragment of the targeted gene, for instance on an additional plant expressible cassette, that leads to enhanced siRNA production, or reduces methylation to prevent transcriptional gene silencing of the dsRNA hairpin promoter.

Embodiments of the invention include introduction of a recombinant nucleic acid molecule of the present invention into a plant *(i.e.,* transformation) to achieve insect *(e.g.,* coleopteran) pest-inhibitory levels of expression of one or more iRNA molecules. A recombinant DNA molecule may, for example, be a vector, such as a linear or a closed circular plasmid. The vector system may be a single vector or plasmid, or two or more vectors or plasmids that together contain the total DNA to be introduced into the genome of a host. In addition, a vector may be an expression vector. Nucleic acids of the invention can, for example, be suitably inserted into a vector under the control of a suitable promoter that functions in one or more hosts to drive expression of a linked coding polynucleotide or other DNA element. Many vectors are available for this purpose, and selection of the appropriate vector will depend mainly on the size of the nucleic acid to be inserted into the vector and the particular host cell to be transformed with the vector. Each vector contains various components depending on its function (*e.g.,* amplification of DNA or expression of DNA) and the particular host cell with which it is compatible.

To impart protection from an insect (*e.g.,* coleopteran) pest to a transgenic plant, a recombinant DNA may, for example, be transcribed into an iRNA molecule *(e.g.,* a RNA molecule that forms a dsRNA molecule) within the tissues or fluids of the recombinant plant. An iRNA molecule may comprise a polynucleotide that is substantially homologous and specifically hybridizable to a corresponding transcribed polynucleotide within an insect pest that may cause damage to the host plant species. The pest may contact the iRNA molecule that is transcribed in cells of the transgenic host plant, for example, by ingesting cells or fluids of the transgenic host plant that comprise the iRNA molecule. Thus, in particular examples, expression of a target gene is suppressed by the iRNA molecule within coleopteran pests that infest the transgenic host plant. In some embodiments, suppression of expression of the target gene in a target coleopteran pest may result in the plant being protected against attack by the pest.

In order to enable delivery of iRNA molecules to an insect pest in a nutritional relationship with a plant cell that has been transformed with a recombinant nucleic acid molecule of the invention, expression *(i.e.,* transcription) of iRNA molecules in the plant cell is required. Thus, a recombinant nucleic acid molecule may comprise a polynucleotide of the invention operably linked to one or more regulatory elements, such as a heterologous promoter element that functions in a host cell, such as a bacterial cell wherein the nucleic acid molecule is to be amplified, and a plant cell wherein the nucleic acid molecule is to be expressed.

Promoters suitable for use in nucleic acid molecules of the invention include those that are inducible, viral, synthetic, or constitutive, all of which are well known in the art. Non-limiting examples describing such promoters include U.S. Patents 6,437,217 (maize RS81 promoter); 5,641,876 (rice actin promoter); 6,426,446 (maize RS324 promoter); 6,429,362 (maize PR-1 promoter); 6,232,526 (maize A3 promoter); 6,177,611 (constitutive maize promoters); 5,322,938, 5,352,605, 5,359,142, and 5,530,196 (CaMV 35S promoter); 6,433,252 (maize L3 oleosin promoter); 6,429,357 (rice actin 2 promoter, and rice actin 2 intron); 6,294,714 (light-inducible promoters); 6,140,078 (salt-inducible promoters); 6,252,138 (pathogen-inducible promoters); 6,175,060 (phosphorous deficiency-inducible promoters); 6,388,170 (bidirectional promoters); 6,635,806 (gamma-coixin promoter); and U.S. Patent Publication No. 2009/757,089 (maize chloroplast aldolase promoter). Additional promoters include the nopaline synthase (NOS) promoter (Ebert et al. (1987) Proc. Natl. Acad. Sci. USA 84(16):5745-9) and the octopine synthase (OCS) promoters (which are carried on tumor-inducing plasmids of *Agrobacterium tumefaciens*); the caulimovirus promoters such as the cauliflower mosaic virus (CaMV) 19S promoter (Lawton et al. (1987) Plant Mol. Biol. 9:315-24); the CaMV 35S promoter (Odell et al. (1985) Nature 313:810-2; the figwort mosaic virus 35S-promoter (Walker et al. (1987) Proc. Natl. Acad. Sci. USA 84(19):6624-8); the sucrose synthase promoter (Yang and Russell (1990) Proc. Natl. Acad. Sci. USA 87:4144-8); the R gene complex promoter (Chandler et al. (1989) Plant Cell 1:1175-83); the chlorophyll a/b binding protein gene promoter; CaMV 35S (U.S. Patents 5,322,938, 5,352,605, 5,359,142, and 5,530,196); FMV 35S (U.S. Patents 6,051,753, and 5,378,619); a PC1SV promoter (U.S. Patent 5,850,019); the SCP1 promoter (U.S. Patent 6,677,503); and AGRtu.nos promoters (GenBank™ Accession No. V00087; Depicker et al. (1982) J. Mol. Appl. Genet. 1:561-73; Bevan et al. (1983) Nature 304:184-7).

In particular embodiments, nucleic acid molecules of the invention comprise a tissue-specific promoter, such as a root-specific promoter. Root-specific promoters drive expression of operably-linked coding polynucleotides exclusively or preferentially in root tissue. Examples of root-specific promoters are known in the art. *See, e.g.,* U.S. Patents 5,110,732; 5,459,252 and 5,837,848; and Opperman et al. (1994) Science 263:221-3; and Hirel et al. (1992) Plant Mol. Biol. 20:207-18. In some embodiments, a polynucleotide or fragment for coleopteran pest control according to the invention may be cloned between two root-specific promoters oriented in opposite transcriptional directions relative to the polynucleotide or fragment, and which are operable in a transgenic plant cell and expressed therein to produce RNA molecules in the transgenic plant cell that subsequently may form dsRNA molecules, as described, *supra*. The iRNA molecules expressed in plant tissues may be ingested by an insect pest so that suppression of target gene expression is achieved.

Additional regulatory elements that may optionally be operably linked to a nucleic acid include 5'UTRs located between a promoter element and a coding polynucleotide that function as a translation leader element. The translation leader element is present in fully-processed mRNA, and it may affect processing of the primary transcript, and/or RNA stability. Examples of translation leader elements include maize and petunia heat shock protein leaders (U.S. Patent 5,362,865), plant virus coat protein leaders, plant rubisco leaders, and others. *See, e.g.,* Turner and Foster (1995) Molecular Biotech. 3(3):225-36. Non-limiting examples of 5'UTRs include GmHsp (U.S. Patent 5,659,122); PhDnaK (U.S. Patent 5,362,865); AtAnt1; TEV (Carrington and Freed (1990) J. Virol. 64:1590-7); and AGRtunos (GenBank™ Accession No. V00087; and Bevan et al. (1983) Nature 304:184-7).

Additional regulatory elements that may optionally be operably linked to a nucleic acid also include 3' non-translated elements, 3' transcription termination regions, or polyadenylation regions. These are genetic elements located downstream of a polynucleotide, and include polynucleotides that provide polyadenylation signal, and/or other regulatory signals capable of affecting transcription or mRNA processing. The polyadenylation signal functions in plants to cause the addition of polyadenylate nucleotides to the 3' end of the mRNA precursor. The polyadenylation element can be derived from a variety of plant genes, or from T-DNA genes. A non-limiting example of a 3' transcription termination region is the nopaline synthase 3' region (nos 3'; Fraley et al. (1983) Proc. Natl. Acad. Sci. USA 80:4803-7). An example of the use of different 3' non-translated regions is provided in Ingelbrecht et al., (1989) Plant Cell 1:671-80. Non-limiting examples of polyadenylation signals include one from a *Pisum sativum* RbcS2 gene (Ps.RbcS2-E9; Coruzzi et al. (1984) EMBO J. 3:1671-9) and AGRtu.nos (GenBank™ Accession No. E01312).

Some embodiments may include a plant transformation vector that comprises an isolated and purified DNA molecule comprising at least one of the above-described regulatory elements operatively linked to one or more polynucleotides of the present invention. When expressed, the one or more polynucleotides result in one or more iRNA molecule(s) comprising a polynucleotide that is specifically complementary to all or part of a native RNA molecule in an insect (*e.g.,* coleopteran) pest. Thus, the polynucleotide(s) may comprise a segment encoding all or part of a polyribonucleotide present within a targeted coleopteran pest RNA transcript, and may comprise inverted repeats of all or a part of a targeted pest transcript. A plant transformation vector may contain polynucleotides specifically complementary to more than one target polynucleotide, thus allowing production of more than one dsRNA for inhibiting expression of two or more genes in cells of one or more populations or species of target insect pests. Segments of polynucleotides specifically complementary to polynucleotides present in different genes can be combined into a single composite nucleic acid molecule for expression in a transgenic plant. Such segments may be contiguous or separated by a spacer.

In some embodiments, a plasmid of the present invention already containing at least one polynucleotide(s) of the invention can be modified by the sequential insertion of additional polynucleotide(s) in the same plasmid, wherein the additional polynucleotide(s) are operably linked to the same regulatory elements as the original at least one polynucleotide(s). In some embodiments, a nucleic acid molecule may be designed for the inhibition of multiple target genes. In some embodiments, the multiple genes to be inhibited can be obtained from the same insect *(e.g.,* coleopteran) pest species, which may enhance the effectiveness of the nucleic acid molecule. In other embodiments, the genes can be derived from different insect pests, which may broaden the range of pests against which the agent(s) is/are effective. When multiple genes are targeted for suppression or a combination of expression and suppression, a polycistronic DNA element can be engineered.

A recombinant nucleic acid molecule or vector of the present invention may comprise a selectable marker that confers a selectable phenotype on a transformed cell, such as a plant cell. Selectable markers may also be used to select for plants or plant cells that comprise a recombinant nucleic acid molecule of the invention. The marker may encode biocide resistance, antibiotic resistance *(e.g.,* kanamycin, Geneticin (G418), bleomycin, hygromycin, *etc*.), or herbicide tolerance *(e.g.,* glyphosate, *etc*.). Examples of selectable markers include, but are not limited to: a *neo* gene which codes for kanamycin resistance and can be selected for using kanamycin, G418, *etc*.; a *bar* gene which codes for bialaphos resistance; a mutant EPSP synthase gene which encodes glyphosate tolerance; a *nitrilase* gene which confers resistance to bromoxynil; a mutant acetolactate synthase (*ALS*) gene which confers imidazolinone or sulfonylurea tolerance; and a methotrexate resistant *DHFR* gene. Multiple selectable markers are available that confer resistance to ampicillin, bleomycin, chloramphenicol, gentamycin, hygromycin, kanamycin, lincomycin, methotrexate, phosphinothricin, puromycin, spectinomycin, rifampicin, streptomycin and tetracycline, and the like. Examples of such selectable markers are illustrated in, *e.g.,* U.S. Patents 5,550,318; 5,633,435; 5,780,708 and 6,118,047.

A recombinant nucleic acid molecule or vector of the present invention may also include a screenable marker. Screenable markers may be used to monitor expression. Exemplary screenable markers include a β-glucuronidase or *uidA* gene (GUS) which encodes an enzyme for which various chromogenic substrates are known (Jefferson et al. (1987) Plant Mol. Biol. Rep. 5:387-405); an R-locus gene, which encodes a product that regulates the production of anthocyanin pigments (red color) in plant tissues (Dellaporta et al. (1988) "Molecular cloning of the maize R-nj allele by transposon tagging with Ac." In 18th Stadler Genetics Symposium, P. Gustafson and R. Appels, eds. (New York: Plenum), pp. 263-82); a β-lactamase gene (Sutcliffe et al. (1978) Proc. Natl. Acad. Sci. USA 75:3737-41); a gene which encodes an enzyme for which various chromogenic substrates are known (*e.g.,* PADAC, a chromogenic cephalosporin); a luciferase gene (Ow et al. (1986) Science 234:856-9); an *xylE* gene that encodes a catechol dioxygenase that can convert chromogenic catechols (Zukowski et al. (1983) Gene 46(2-3):247-55); an amylase gene (Ikatu et al. (1990) Bio/Technol. 8:241-2); a tyrosinase gene which encodes an enzyme capable of oxidizing tyrosine to DOPA and dopaquinone which in turn condenses to melanin (Katz et al. (1983) J. Gen. Microbiol. 129:2703-14); and an α-galactosidase.

In some embodiments, recombinant nucleic acid molecules, as described, *supra*, may be used in methods for the creation of transgenic plants and expression of heterologous nucleic acids in plants to prepare transgenic plants that exhibit reduced susceptibility to insect *(e.g.,* coleopteran) pests. Plant transformation vectors can be prepared, for example, by inserting nucleic acid molecules encoding iRNA molecules into plant transformation vectors and introducing these into plants.

Suitable methods for transformation of host cells include any method by which DNA can be introduced into a cell, such as by transformation of protoplasts *(See, e.g.,* U.S. Patent 5,508,184), by desiccation/inhibition-mediated DNA uptake *(See, e.g.,* Potrykus et al. (1985) Mol. Gen. Genet. 199:183-8), by electroporation (*See*, *e.g.,* U.S. Patent 5,384,253), by agitation with silicon carbide fibers *(See, e.g.,* U.S. Patents 5,302,523 and 5,464,765), by *Agrobacterium*-mediated transformation (*See*, *e.g*., U.S. Patents 5,563,055; 5,591,616; 5,693,512; 5,824,877; 5,981,840; and 6,384,301), and by acceleration of DNA-coated particles (*See*, *e.g*., U.S. Patents 5,015,580; 5,550,318; 5,538,880; 6,160,208; 6,399,861; and 6,403,865), *etc*. Techniques that are particularly useful for transforming corn are described, for example, in U.S. Patents 7,060,876 and 5,591,616; and International PCT Publication WO95/06722. Through the application of techniques such as these, the cells of virtually any species may be stably transformed. In some embodiments, transforming DNA is integrated into the genome of the host cell. In the case of multicellular species, transgenic cells may be regenerated into a transgenic organism. Any of these techniques may be used to produce a transgenic plant, for example, comprising one or more nucleic acids encoding one or more iRNA molecules in the genome of the transgenic plant.

The most widely utilized method for introducing an expression vector into plants is based on the natural transformation system of *Agrobacterium*. *A. tumefaciens* and A. *rhizogenes* are plant pathogenic soil bacteria which genetically transform plant cells. The Ti and Ri plasmids of A. *tumefaciens* and A. *rhizogenes*, respectively, carry genes responsible for genetic transformation of the plant. The Ti (tumor-inducing)-plasmids contain a large segment, known as T-DNA, which is transferred to transformed plants. Another segment of the Ti plasmid, the Vir region, is responsible for T-DNA transfer. The T-DNA region is bordered by terminal repeats. In modified binary vectors, the tumor-inducing genes have been deleted, and the functions of the Vir region are utilized to transfer foreign DNA bordered by the T-DNA border elements. The T-region may also contain a selectable marker for efficient recovery of transgenic cells and plants, and a multiple cloning site for inserting polynucleotides for transfer such as a dsRNA encoding nucleic acid.

Thus, in some embodiments, a plant transformation vector is derived from a Ti plasmid of A. *tumefaciens* (*See*, *e.g.*, U.S. Patents 4,536,475, 4,693,977, 4,886,937, and 5,501,967; and European Patent No. EP 0 122 791) or a Ri plasmid of A. *rhizogenes*. Additional plant transformation vectors include, for example and without limitation, those described by Herrera-Estrella et al. (1983) Nature 303:209-13; Bevan et al. (1983) Nature 304:184-7; Klee et al. (1985) Bio/Technol. 3:637-42; and in European Patent No. EP 0 120 516, and those derived from any of the foregoing. Other bacteria such as *Sinorhizobium*, *Rhizobium*, and *Mesorhizobium* that interact with plants naturally can be modified to mediate gene transfer to a number of diverse plants. These plant-associated symbiotic bacteria can be made competent for gene transfer by acquisition of both a disarmed Ti plasmid and a suitable binary vector.

After providing exogenous DNA to recipient cells, transformed cells are generally identified for further culturing and plant regeneration. In order to improve the ability to identify transformed cells, one may desire to employ a selectable or screenable marker gene, as previously set forth, with the transformation vector used to generate the transformant. In the case where a selectable marker is used, transformed cells are identified within the potentially transformed cell population by exposing the cells to a selective agent or agents. In the case where a screenable marker is used, cells may be screened for the desired marker gene trait.

Cells that survive the exposure to the selective agent, or cells that have been scored positive in a screening assay, may be cultured in media that supports regeneration of plants. In some embodiments, any suitable plant tissue culture media (*e.g.*, MS and N6 media) may be modified by including further substances, such as growth regulators. Tissue may be maintained on a basic medium with growth regulators until sufficient tissue is available to begin plant regeneration efforts, or following repeated rounds of manual selection, until the morphology of the tissue is suitable for regeneration (*e.g.,* at least 2 weeks), then transferred to media conducive to shoot formation. Cultures are transferred periodically until sufficient shoot formation has occurred. Once shoots are formed, they are transferred to media conducive to root formation. Once sufficient roots are formed, plants can be transferred to soil for further growth and maturation.

To confirm the presence of a nucleic acid molecule of interest (for example, a DNA encoding one or more iRNA molecules that inhibit target gene expression in a coleopteran pest) in the regenerating plants, a variety of assays may be performed. Such assays include, for example: molecular biological assays, such as Southern and northern blotting, PCR, and nucleic acid sequencing; biochemical assays, such as detecting the presence of a protein product, *e.g.,* by immunological means (ELISA and/or western blots) or by enzymatic function; plant part assays, such as leaf or root assays; and analysis of the phenotype of the whole regenerated plant.

Integration events may be analyzed, for example, by PCR amplification using, *e.g.,* oligonucleotide primers specific for a nucleic acid molecule of interest. PCR genotyping is understood to include, but not be limited to, polymerase-chain reaction (PCR) amplification of gDNA derived from isolated host plant callus tissue predicted to contain a nucleic acid molecule of interest integrated into the genome, followed by standard cloning and sequence analysis of PCR amplification products. Methods of PCR genotyping have been well described (for example, Rios, G. et al. (2002) Plant J. 32:243-53) and may be applied to gDNA derived from any plant species *(e.g., Z. mays)* or tissue type, including cell cultures.

A transgenic plant formed using *Agrobacterium*-dependent transformation methods typically contains a single recombinant DNA inserted into one chromosome. The polynucleotide of the single recombinant DNA is referred to as a "transgenic event" or "integration event". Such transgenic plants are heterozygous for the inserted exogenous polynucleotide. In some embodiments, a transgenic plant homozygous with respect to a transgene may be obtained by sexually mating (selfing) an independent segregant transgenic plant that contains a single exogenous gene to itself, for example a $T_0$ plant, to produce $T_1$ seed. One fourth of the $T_1$ seed produced will be homozygous with respect to the transgene. Germinating $T_1$ seed results in plants that can be tested for heterozygosity, typically using an SNP assay or a thermal amplification assay that allows for the distinction between heterozygotes and homozygotes *(i.e.,* a zygosity assay).

In particular embodiments, at least 2, 3, 4, 5, 6, 7, 8, 9 or 10 or more different iRNA molecules are produced in a plant cell that have an insect (*e.g.,* coleopteran) pest-inhibitory effect. The iRNA molecules (*e.g.,* dsRNA molecules) may be expressed from multiple nucleic acids introduced in different transformation events, or from a single nucleic acid introduced in a single transformation event. In some embodiments, a plurality of iRNA molecules are expressed under the control of a single promoter. In other embodiments, a plurality of iRNA molecules are expressed under the control of multiple promoters. Single iRNA molecules may be expressed that comprise multiple polynucleotides that are each homologous to different loci within one or more insect pests (for example, the loci defined by SEQ ID NOs:1 and 3), both in different populations of the same species of insect pest, or in different species of insect pests.

In addition to direct transformation of a plant with a recombinant nucleic acid molecule, transgenic plants can be prepared by crossing a first plant having at least one transgenic event with a second plant lacking such an event. For example, a recombinant nucleic acid molecule comprising a polynucleotide that encodes an iRNA molecule may be introduced into a first plant line that is amenable to transformation to produce a transgenic plant, which transgenic plant may be crossed with a second plant line to introgress the polynucleotide that encodes the iRNA molecule into the second plant line.

In some aspects, seeds and commodity products produced by transgenic plants derived from transformed plant cells are included, wherein the seeds or commodity products comprise a detectable amount of a nucleic acid of the invention. In some embodiments, such commodity products may be produced, for example, by obtaining transgenic plants and preparing food or feed from them. Commodity products comprising one or more of the polynucleotides of the invention includes, for example and without limitation: meals, oils, crushed or whole grains or seeds of a plant, and any food product comprising any meal, oil, or crushed or whole grain of a recombinant plant or seed comprising one or more of the nucleic acids of the invention. The detection of one or more of the polynucleotides of the invention in one or more commodity or commodity products is *de facto* evidence that the commodity or commodity product is produced from a transgenic plant designed to express one or more of the iRNA molecules of the invention for the purpose of controlling insect (*e.g.*, coleopteran) pests.

In some embodiments, a transgenic plant or seed comprising a nucleic acid molecule of the invention also may comprise at least one other transgenic event in its genome, including without limitation: a transgenic event from which is transcribed an iRNA molecule targeting a locus in a coleopteran pest other than the one defined by SEQ ID NO: 1 and SEQ ID NO: 3, such as, for example, one or more loci selected from the group consisting of *Caf1-180* (U.S. Patent Application Publication No. 2012/0174258); *VatpaseC* (U.S. Patent Application Publication No. 2012/0174259); *Rho1* (U.S. Patent Application Publication No. 2012/0174260); *VatpaseH* (U.S. Patent Application Publication No. 2012/0198586); *PPI-87B* (U.S. Patent Application Publication No. 2013/0091600); *RPA70* (U.S. Patent Application Publication No. 2013/0091601); *RPS6* (U.S. Patent Application Publication No. 2013/0097730); *ROP* (U.S. Patent Application Publication No. 14/577811); *RNAPII* (U.S. Patent Application Publication No. 14/577854); *RNA polymerase II215* (U.S. Patent Application No. 62/133202); *RNA polymerase 33* (U.S. Patent Application No. 62/133210); *ncm* (U.S. Patent Application No. 62/095487); *Dre4* (U.S. Patent Application No. 14/705,807); *COPI alpha* (U.S. Patent Application No. 62/063,199); *COPI beta* (U.S. Patent Application No. 62/063,203); *COPI gamma* (U.S. Patent Application No. 62/063,192); and *COPI delta* (U.S. Patent Application No. 62/063,216); a transgenic event from which is transcribed an iRNA molecule targeting a gene in an organism other than a coleopteran pest *(e.g.,* a plant-parasitic nematode); a gene encoding an insecticidal protein *(e.g.,* a *Bacillus thuringiensis* insecticidal protein); an herbicide tolerance gene *(e.g.,* a gene providing tolerance to glyphosate); and a gene contributing to a desirable phenotype in the transgenic plant, such as increased yield, altered fatty acid metabolism, or restoration of cytoplasmic male sterility. In particular embodiments, polynucleotides encoding iRNA molecules of the invention may be combined with other insect control and disease traits in a plant to achieve desired traits for enhanced control of plant disease and insect damage. Combining insect control traits that employ distinct modes-of-action may provide protected transgenic plants with superior durability over plants harboring a single control trait, for example, because of the reduced probability that resistance to the trait(s) will develop in the field.

### *V. Target Gene Suppression in an Insect Pest*

#### A. Overview

In some embodiments of the invention, at least one nucleic acid molecule useful for the control of insect (*e.g.,* coleopteran) pests may be provided to an insect pest, wherein the nucleic acid molecule leads to RNAi-mediated gene silencing in the pest. In particular embodiments, an iRNA molecule (*e.g.,* dsRNA, siRNA, miRNA, shRNA, and hpRNA) may be provided to a coleopteran pest. In some embodiments, a nucleic acid molecule useful for the control of insect pests may be provided to a pest by contacting the nucleic acid molecule with the pest. In these and further embodiments, a nucleic acid molecule useful for the control of insect pests may be provided in a feeding substrate of the pest, for example, a nutritional composition. In these and further embodiments, a nucleic acid molecule useful for the control of an insect pest may be provided through ingestion of plant material comprising the nucleic acid molecule that is ingested by the pest. In certain embodiments, the nucleic acid molecule is present in plant material through expression of a recombinant nucleic acid introduced into the plant material, for example, by transformation of a plant cell with a vector comprising the recombinant nucleic acid and regeneration of a plant material or whole plant from the transformed plant cell.

In some embodiments, a pest is contacted with the nucleic acid molecule that leads to RNAi-mediated gene silencing in the pest through contact with a topical composition *(e.g.,* a composition applied by spraying) or an RNAi bait. RNAi baits are formed when the dsRNA is mixed with food or an attractant or both. When the pests eat the bait, they also consume the dsRNA. Baits may take the form of granules, gels, flowable powders, liquids, or solids. In particular embodiments, *rpl1* may be incorporated into a bait formulation such as that described in U.S. Patent No. 8,530,440 which is hereby incorporated by reference. Generally, with baits, the baits are placed in or around the environment of the insect pest, for example, WCR can come into contact with, and/or be attracted to, the bait.

**B. RNAi-mediated Target Gene Suppression**

In embodiments, the invention provides iRNA molecules (*e.g.,* dsRNA, siRNA, miRNA, shRNA, and hpRNA) that may be designed to target essential native polynucleotides (*e.g.,* essential genes) in the transcriptome of an insect pest (for example, a coleopteran *(e.g.,* WCR, NCR, and SCR) pest), for example by designing an iRNA molecule that comprises at least one strand comprising a polynucleotide that is specifically complementary to the target polynucleotide. The sequence of an iRNA molecule so designed may be identical to that of the target polynucleotide, or may incorporate mismatches that do not prevent specific hybridization between the iRNA molecule and its target polynucleotide.

iRNA molecules of the invention may be used in methods for gene suppression in an insect (*e.g.,* coleopteran) pest, thereby reducing the level or incidence of damage caused by the pest on a plant (for example, a protected transformed plant comprising an iRNA molecule). As used herein the term "gene suppression" refers to any of the well-known methods for reducing the levels of protein produced as a result of gene transcription to mRNA and subsequent translation of the mRNA, including the reduction of protein expression from a gene or a coding polynucleotide including post-transcriptional inhibition of expression and transcriptional suppression. Post-transcriptional inhibition is mediated by specific homology between all or a part of an mRNA transcribed from a gene targeted for suppression and the corresponding iRNA molecule used for suppression. Additionally, post-transcriptional inhibition refers to the substantial and measurable reduction of the amount of mRNA available in the cell for binding by ribosomes.

In embodiments wherein an iRNA molecule is a dsRNA molecule, the dsRNA molecule may be cleaved by the enzyme, DICER, into short siRNA molecules (approximately 20 nucleotides in length). The double-stranded siRNA molecule generated by DICER activity upon the dsRNA molecule may be separated into two single-stranded siRNAs; the "passenger strand" and the "guide strand." The passenger strand may be degraded, and the guide strand may be incorporated into RISC. Post-transcriptional inhibition occurs by specific hybridization of the guide strand with a specifically complementary polynucleotide of an mRNA molecule, and subsequent cleavage by the enzyme, Argonaute (catalytic component of the RISC complex).

In embodiments of the invention, any form of iRNA molecule may be used. Those of skill in the art will understand that dsRNA molecules typically are more stable during preparation and during the step of providing the iRNA molecule to a cell than are single-stranded RNA molecules, and are typically also more stable in a cell. Thus, while siRNA and miRNA molecules, for example, may be equally effective in some embodiments, a dsRNA molecule may be chosen due to its stability.

In particular embodiments, a nucleic acid molecule is provided that comprises a polynucleotide, which polynucleotide may be expressed *in vitro* to produce an iRNA molecule that is substantially homologous to a nucleic acid molecule encoded by a polynucleotide within the genome of an insect *(e.g.,* coleopteran) pest. In certain embodiments, the *in vitro* transcribed iRNA molecule may be a stabilized dsRNA molecule that comprises a stem-loop structure. After an insect pest contacts the *in vitro* transcribed iRNA molecule, post-transcriptional inhibition of a target gene in the pest (for example, an essential gene) may occur.

In some embodiments of the invention, expression of a nucleic acid molecule comprising at least 15 contiguous nucleotides *(e.g.,* at least 19 contiguous nucleotides) of a polynucleotide are used in a method for post-transcriptional inhibition of a target gene in an insect (*e.g.,* coleopteran) pest, wherein the polynucleotide is selected from the group consisting of: SEQ ID NO:1; the complement of SEQ ID NO:1; SEQ ID NO:3; the complement of SEQ ID NO:3; SEQ ID NO:5; the complement of SEQ ID NO:5; SEQ ID NO:6; the complement of SEQ ID NO:6; SEQ ID NO:7; the complement of SEQ ID NO:7; SEQ ID NO:8; the complement of SEQ ID NO:8; a fragment of at least 15 contiguous nucleotides of SEQ ID NO:1 and/or SEQ ID NO:3; the complement of a fragment of at least 15 contiguous nucleotides of SEQ ID NO:1 and/or SEQ ID NO:3; a native coding polynucleotide of a *Diabrotica* organism comprising any of SEQ ID NOs:5-8; the complement of a native coding polynucleotide of a *Diabrotica* organism comprising any of SEQ ID NOs:5-8; a fragment of at least 15 contiguous nucleotides of a native coding polynucleotide of a *Diabrotica* organism comprising any of SEQ ID NOs:5-8; the complement of a fragment of at least 15 contiguous nucleotides of a native coding polynucleotide of a *Diabrotica* organism comprising any of SEQ ID NOs:5-8. In certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with any of the foregoing may be used. In certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with SEQ ID NO:1 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of SEQ ID NO:1 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with SEQ ID NO:3 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of SEQ ID NO:3 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with SEQ ID NO:5 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of SEQ ID NO:5 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with SEQ ID NO:6 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of SEQ ID NO:6 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with SEQ ID NO:7 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of SEQ ID NO:7 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with SEQ ID NO:8 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of SEQ ID NO:8 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a fragment of at least 15 contiguous nucleotides of SEQ ID NO:1 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a fragment of at least 15 contiguous nucleotides of SEQ ID NO:3 may be used, or in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a fragment of at least 15 contiguous nucleotides of SEQ ID NO:1 and SEQ ID NO:3 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a fragment of at least 15 contiguous nucleotides of SEQ ID NO:1 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a fragment of at least 15 contiguous nucleotides of SEQ ID NO:3 may be used; or in certain embodiments, expression of a nucleic acid molecule that is at least about 80% identical *(e.g.,* 79%, about 80%, about 81%, about 82%,

about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a fragment of at least 15 contiguous nucleotides of SEQ ID NO:1 and SEQ ID NO:3 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a native coding polynucleotide of a *Diabrotica* organism comprising SEQ ID NO:5 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a native coding polynucleotide of a *Diabrotica* organism comprising SEQ ID NO:6 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a native coding polynucleotide of a *Diabrotica* organism comprising SEQ ID NO:7 may be used, or in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a native coding polynucleotide of a *Diabrotica* organism comprising SEQ ID NO:8 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a native coding polynucleotide of a *Diabrotica* organism comprising any of SEQ ID NO:5 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a native coding polynucleotide of a *Diabrotica* organism comprising any of SEQ ID NO:6 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a native coding polynucleotide of a *Diabrotica* organism comprising any of SEQ ID NO:7 may be used, or in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a native coding polynucleotide of a *Diabrotica* organism comprising any of SEQ ID NO:8 may be used; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a fragment of at least 15 contiguous nucleotides of a native coding polynucleotide of a *Diabrotica* organism comprising SEQ ID NO:5 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a fragment of at least 15 contiguous nucleotides of a native coding polynucleotide of a *Diabrotica* organism comprising SEQ ID NO:6 may be used in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a fragment of at least 15 contiguous nucleotides of a native coding polynucleotide of a *Diabrotica* organism comprising SEQ ID NO:7 may be used, or in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with a fragment of at least 15 contiguous nucleotides of a native coding polynucleotide of a *Diabrotica* organism comprising SEQ ID NO:8; in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a fragment of at least 15 contiguous nucleotides of a native coding polynucleotide of a *Diabrotica* organism comprising any of SEQ ID NO:5 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a fragment of at least 15 contiguous nucleotides of a native coding polynucleotide of a *Diabrotica* organism comprising any of SEQ ID NO:6 may be used, in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a fragment of at least 15 contiguous nucleotides of a native coding polynucleotide of a *Diabrotica* organism comprising any of SEQ ID NO:7 may be used, or in certain embodiments, expression of a nucleic acid molecule that is at least about 80% identical *(e.g.,* 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 100%, and 100%) with the complement of a fragment of at least 15 contiguous nucleotides of a native coding polynucleotide of a *Diabrotica* organism comprising any of SEQ ID NO:8 may be used. In these and further embodiments, a nucleic acid molecule may be expressed that specifically hybridizes to a RNA molecule present in at least one cell of an insect *(e.g.,* coleopteran) pest.

It is an important feature of some embodiments herein that the RNAi post-transcriptional inhibition system is able to tolerate sequence variations among target genes that might be expected due to genetic mutation, strain polymorphism, or evolutionary divergence. The introduced nucleic acid molecule may not need to be absolutely homologous to either a primary transcription product or a fully-processed mRNA of a target gene, so long as the introduced nucleic acid molecule is specifically hybridizable to either a primary transcription product or a fully-processed mRNA of the target gene. Moreover, the introduced nucleic acid molecule may not need to be full-length, relative to either a primary transcription product or a fully processed mRNA of the target gene.

Inhibition of a target gene using the iRNA technology of the present invention is sequence-specific; *i.e.,* polynucleotides substantially homologous to the iRNA molecule(s) are targeted for genetic inhibition. In some embodiments, a RNA molecule comprising a polynucleotide with a nucleotide sequence that is identical to that of a portion of a target gene may be used for inhibition. In these and further embodiments, a RNA molecule comprising a polynucleotide with one or more insertion, deletion, and/or point mutations relative to a target polynucleotide may be used. In particular embodiments, an iRNA molecule and a portion of a target gene may share, for example, at least from about 80%, at least from about 81%, at least from about 82%, at least from about 83%, at least from about 84%, at least from about 85%, at least from about 86%, at least from about 87%, at least from about 88%, at least from about 89%, at least from about 90%, at least from about 91%, at least from about 92%, at least from about 93%, at least from about 94%, at least from about 95%, at least from about 96%, at least from about 97%, at least from about 98%, at least from about 99%, at least from about 100%, and 100% sequence identity. Alternatively, the duplex region of a dsRNA molecule may be specifically hybridizable with a portion of a target gene transcript. In specifically hybridizable molecules, a less than full length polynucleotide exhibiting a greater homology compensates for a longer, less homologous polynucleotide. The length of the polynucleotide of a duplex region of a dsRNA molecule that is identical to a portion of a target gene transcript may be at least about 25, 50, 100, 200, 300, 400, 500, or at least about 1000 bases. In some embodiments, a polynucleotide of greater than 20-100 nucleotides may be used. In particular embodiments, a polynucleotide of greater than about 100-500 nucleotides may be used. In particular embodiments, a polynucleotide of greater than about 500-1000 nucleotides may be used, depending on the size of the target gene.

In certain embodiments, expression of a target gene in a pest (*e.g.,* coleopteran) may be inhibited by at least 10%; at least 33%; at least 50%; or at least 80% within a cell of the pest, such that a significant inhibition takes place. Significant inhibition refers to inhibition over a threshold that results in a detectable phenotype (*e.g.,* cessation of growth, cessation of feeding, cessation of development, induced mortality, *etc.),* or a detectable decrease in RNA and/or gene product corresponding to the target gene being inhibited. Although, in certain embodiments of the invention, inhibition occurs in substantially all cells of the pest, in other embodiments inhibition occurs only in a subset of cells expressing the target gene.

In some embodiments, transcriptional suppression is mediated by the presence in a cell of a dsRNA molecule exhibiting substantial sequence identity to a promoter DNA or the complement thereof to effect what is referred to as "promoter trans suppression." Gene suppression may be effective against target genes in an insect pest that may ingest or contact such dsRNA molecules, for example, by ingesting or contacting plant material containing the dsRNA molecules. dsRNA molecules for use in promoter trans suppression may be specifically designed to inhibit or suppress the expression of one or more homologous or complementary polynucleotides in the cells of the insect pest. Post-transcriptional gene suppression by antisense or sense oriented RNA to regulate gene expression in plant cells is disclosed in U.S. Patents 5,107,065; 5,759,829; 5,283,184; and 5,231,020.

### C. Expression of iRNA Molecules Provided to an Insect Pest

Expression of iRNA molecules for RNAi-mediated gene inhibition in an insect *(e.g.,* coleopteran) pest may be carried out in any one of many *in vitro* or *in vivo* formats. The iRNA molecules may then be provided to an insect pest, for example, by contacting the iRNA molecules with the pest, or by causing the pest to ingest or otherwise internalize the iRNA molecules. Some embodiments include transformed host plants of a coleopteran pest, transformed plant cells, and progeny of transformed plants. The transformed plant cells and transformed plants may be engineered to express one or more of the iRNA molecules, for example, under the control of a heterologous promoter, to provide a pest-protective effect. Thus, when a transgenic plant or plant cell is consumed by an insect pest during feeding, the pest may ingest iRNA molecules expressed in the transgenic plants or cells. The polynucleotides of the present invention may also be introduced into a wide variety of prokaryotic and eukaryotic microorganism hosts to produce iRNA molecules. The term "microorganism" includes prokaryotic and eukaryotic species, such as bacteria and fungi.

Modulation of gene expression may include partial or complete suppression of such expression. In another embodiment, a method for suppression of gene expression in an insect (*e.g.,* coleopteran) pest comprises providing in the tissue of the host of the pest a gene-suppressive amount of at least one dsRNA molecule formed following transcription of a polynucleotide as described herein, at least one segment of which is complementary to an mRNA within the cells of the insect pest. A dsRNA molecule, including its modified form such as an siRNA, miRNA, shRNA, or hpRNA molecule, ingested by an insect pest may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to a RNA molecule transcribed from a *rpl1* DNA molecule, for example, comprising a polynucleotide selected from the group consisting of SEQ ID NOs:1, 3, and 5-8. Isolated and substantially purified nucleic acid molecules including, but not limited to, non-naturally occurring polynucleotides and recombinant DNA constructs for providing dsRNA molecules are therefore provided, which suppress or inhibit the expression of an endogenous coding polynucleotide or a target coding polynucleotide in an insect pest when introduced thereto.

Particular embodiments provide a delivery system for the delivery of iRNA molecules for the post-transcriptional inhibition of one or more target gene(s) in an insect *(e.g.,* coleopteran) plant pest and control of a population of the plant pest. In some embodiments, the delivery system comprises ingestion of a host transgenic plant cell or contents of the host cell comprising RNA molecules transcribed in the host cell. In these and further embodiments, a transgenic plant cell or a transgenic plant is created that contains a recombinant DNA construct providing a stabilized dsRNA molecule of the invention. Transgenic plant cells and transgenic plants comprising nucleic acids encoding a particular iRNA molecule may be produced by employing recombinant DNA technologies (which basic technologies are well-known in the art) to construct a plant transformation vector comprising a polynucleotide encoding an iRNA molecule of the invention (*e.g.,* a stabilized dsRNA molecule); to transform a plant cell or plant; and to generate the transgenic plant cell or the transgenic plant that contains the transcribed iRNA molecule.

To impart insect (*e.g.,* coleopteran) pest protection to a transgenic plant, a recombinant DNA molecule may, for example, be transcribed into an iRNA molecule, such as a dsRNA molecule, a siRNA molecule, a miRNA molecule, a shRNA molecule, or a hpRNA molecule. In some embodiments, a RNA molecule transcribed from a recombinant DNA molecule may form a dsRNA molecule within the tissues or fluids of the recombinant plant. Such a dsRNA molecule may be comprised in part of a polynucleotide that is identical to a corresponding polynucleotide transcribed from a DNA within an insect pest of a type that may infest the host plant. Expression of a target gene within the pest is suppressed by the dsRNA molecule, and the suppression of expression of the target gene in the pest results in the transgenic plant being protected against the pest. The modulatory effects of dsRNA molecules have been shown to be applicable to a variety of genes expressed in pests, including, for example, endogenous genes responsible for cellular metabolism or cellular transformation, including house-keeping genes; transcription factors; molting-related genes; and other genes which encode polypeptides involved in cellular metabolism or normal growth and development.

For transcription from a transgene *in vivo* or an expression construct, a regulatory region (*e.g.,* promoter, enhancer, silencer, and polyadenylation signal) may be used in some embodiments to transcribe the RNA strand (or strands). Therefore, in some embodiments, as set forth, *supra*, a polynucleotide for use in producing iRNA molecules may be operably linked to one or more promoter elements functional in a plant host cell. The promoter may be an endogenous promoter, normally resident in the host genome. The polynucleotide of the present invention, under the control of an operably linked promoter element, may further be flanked by additional elements that advantageously affect its transcription and/or the stability of a resulting transcript. Such elements may be located upstream of the operably linked promoter, downstream of the 3' end of the expression construct, and may occur both upstream of the promoter and downstream of the 3' end of the expression construct.

Some embodiments provide methods for reducing the damage to a host plant *(e.g.,* a corn plant) caused by an insect (*e.g.,* coleopteran) pest that feeds on the plant, wherein the method comprises providing in the host plant a transformed plant cell expressing at least one nucleic acid molecule of the invention, wherein the nucleic acid molecule(s) functions upon being taken up by the pest(s) to inhibit the expression of a target polynucleotide within the pest(s), which inhibition of expression results in mortality and/or reduced growth of the pest(s), thereby reducing the damage to the host plant caused by the pest(s). In some embodiments, the nucleic acid molecule(s) comprise dsRNA molecules. In these and further embodiments, the nucleic acid molecule(s) comprise dsRNA molecules that each comprise more than one polynucleotide that is specifically hybridizable to a nucleic acid molecule expressed in a coleopteran pest cell. In some embodiments, the nucleic acid molecule(s) consist of one polynucleotide that is specifically hybridizable to a nucleic acid molecule expressed in an insect pest cell.

In some embodiments, a method for increasing the yield of a corn crop is provided, wherein the method comprises introducing into a corn plant at least one nucleic acid molecule of the invention; cultivating the corn plant to allow the expression of an iRNA molecule comprising the nucleic acid, wherein expression of an iRNA molecule comprising the nucleic acid inhibits insect (*e.g.,* coleopteran) pest damage and/or growth, thereby reducing or eliminating a loss of yield due to pest infestation. In some embodiments, the iRNA molecule is a dsRNA molecule. In these and further embodiments, the nucleic acid molecule(s) comprise dsRNA molecules that each comprise more than one polynucleotide that is specifically hybridizable to a nucleic acid molecule expressed in an insect pest cell. In some examples, the nucleic acid molecule(s) comprises a polynucleotide that is specifically hybridizable to a nucleic acid molecule expressed in a coleopteran pest cell.

In some embodiments, a method for modulating the expression of a target gene in an insect (*e.g.,* coleopteran) pest is provided, the method comprising: transforming a plant cell with a vector comprising a polynucleotide encoding at least one iRNA molecule of the invention, wherein the polynucleotide is operatively-linked to a promoter and a transcription termination element; culturing the transformed plant cell under conditions sufficient to allow for development of a plant cell culture including a plurality of transformed plant cells; selecting for transformed plant cells that have integrated the polynucleotide into their genomes; screening the transformed plant cells for expression of an iRNA molecule encoded by the integrated polynucleotide; selecting a transgenic plant cell that expresses the iRNA molecule; and feeding the selected transgenic plant cell to the insect pest. Plants may also be regenerated from transformed plant cells that express an iRNA molecule encoded by the integrated nucleic acid molecule. In some embodiments, the iRNA molecule is a dsRNA molecule. In these and further embodiments, the nucleic acid molecule(s) comprise dsRNA molecules that each comprise more than one polynucleotide that is specifically hybridizable to a nucleic acid molecule expressed in an insect pest cell. In some examples, the nucleic acid molecule(s) comprises a polynucleotide that is specifically hybridizable to a nucleic acid molecule expressed in a coleopteran pest cell.

iRNA molecules of the invention can be incorporated within the seeds of a plant species *(e.g.,* corn), either as a product of expression from a recombinant gene incorporated into a genome of the plant cells, or as incorporated into a coating or seed treatment that is applied to the seed before planting. A plant cell comprising a recombinant gene is considered to be a transgenic event. Also included in embodiments of the invention are delivery systems for the delivery of iRNA molecules to insect (*e.g.,* coleopteran) pests. For example, the iRNA molecules of the invention may be directly introduced into the cells of a pest(s). Methods for introduction may include direct mixing of iRNA with plant tissue from a host for the insect pest(s), as well as application of compositions comprising iRNA molecules of the invention to host plant tissue. For example, iRNA molecules may be sprayed onto a plant surface. Alternatively, an iRNA molecule may be expressed by a microorganism, and the microorganism may be applied onto the plant surface, or introduced into a root or stem by a physical means such as an injection. As discussed, *supra*, a transgenic plant may also be genetically engineered to express at least one iRNA molecule in an amount sufficient to kill the insect pests known to infest the plant. iRNA molecules produced by chemical or enzymatic synthesis may also be formulated in a manner consistent with common agricultural practices, and used as spray-on products for controlling plant damage by an insect pest. The formulations may include the appropriate stickers and wetters required for efficient foliar coverage, as well as UV protectants to protect iRNA molecules (*e.g.,* dsRNA molecules) from UV damage. Such additives are commonly used in the bioinsecticide industry, and are well known to those skilled in the art. Such applications may be combined with other spray-on insecticide applications (biologically based or otherwise) to enhance plant protection from the pests.

All references, including publications, patents, and patent applications, cited herein are hereby incorporated by reference to the extent they are not inconsistent with the explicit details of this disclosure, and are so incorporated to the same extent as if each reference were individually and specifically indicated to be incorporated by reference and were set forth in its entirety herein. The references discussed herein are provided solely for their disclosure prior to the filing date of the present application. Nothing herein is to be construed as an admission that the inventors are not entitled to antedate such disclosure by virtue of prior invention.

The following EXAMPLES are provided to illustrate certain particular features and/or aspects. These EXAMPLES should not be construed to limit the disclosure to the particular features or aspects described.

## EXAMPLES

### EXAMPLE 1: Materials and Methods

Sample preparation and bioassays

A number of dsRNA molecules (including those corresponding to *rpl1-1* reg1 (SEQ ID NO:5), *rpl1-2* reg2 (SEQ ID NO:6), *rpl1-2* v1 (SEQ ID NO:7), and *rpl1-2* v2 (SEQ ID NO:8) were synthesized and purified using a MEGASCRIPT® RNAi kit or HiScribe® T7 In Vitro Transcription Kit. The purified dsRNA molecules were prepared in TE buffer, and all bioassays contained a control treatment consisting of this buffer, which served as a background check for mortality or growth inhibition of WCR (*Diabrotica virgifera virgifera* LeConte). The concentrations of dsRNA molecules in the bioassay buffer were measured using a NANODROP™ 8000 spectrophotometer (THERMO SCIENTIFIC, Wilmington, DE).

Samples were tested for insect activity in bioassays conducted with neonate insect larvae on artificial insect diet. WCR eggs were obtained from CROP CHARACTERISTICS, INC. (Farmington, MN).

The bioassays were conducted in 128-well plastic trays specifically designed for insect bioassays (C-D INTERNATIONAL, Pitman, NJ). Each well contained approximately 1.0 mL of an artificial diet designed for growth of coleopteran insects. A 60 μL aliquot of dsRNA sample was delivered by pipette onto the surface of the diet of each well (40 $\mu L/cm^2$). dsRNA sample concentrations were calculated as the amount of dsRNA per square centimeter ($ng/cm^2$) of surface area (1.5 $cm^2$) in the well. The treated trays were held in a fume hood until the liquid on the diet surface evaporated or was absorbed into the diet.

Within a few hours of eclosion, individual larvae were picked up with a moistened camel hair brush and deposited on the treated diet (one or two larvae per well). The infested wells of the 128-well plastic trays were then sealed with adhesive sheets of clear plastic, and vented to allow gas exchange. Bioassay trays were held under controlled environmental conditions (28 °C, ~40% Relative Humidity, 16:8 (Light:Dark)) for 9 days, after which time the total number of insects exposed to each sample, the number of dead insects, and the weight of surviving insects were recorded. Average percent mortality and average growth inhibition were calculated for each treatment. Growth inhibition (GI) was calculated as follows:

$$GI = [1 - (TWIT/TNIT)/(TWIBC/TNIBC)],$$

where TWIT is the Total Weight of live Insects in the Treatment;
TNIT is the Total Number of Insects in the Treatment;
TWIBC is the Total Weight of live Insects in the Background Check (Buffer control); and
TNIBC is the Total Number of Insects in the Background Check (Buffer control).

The statistical analysis was done using JMP™ software (SAS, Cary, NC).

The $LC_{50}$ (Lethal Concentration) is defined as the dosage at which 50% of the test insects are killed. The $GI_{50}$ (Growth Inhibition) is defined as the dosage at which the mean growth *(e.g.* live weight) of the test insects is 50% of the mean value seen in Background Check samples.

Replicated bioassays demonstrated that ingestion of particular samples resulted in a surprising and unexpected mortality and growth inhibition of corn rootworm larvae.

### EXAMPLE 2: Identification of Candidate Target Genes

Multiple stages of WCR (*Diabrotica virgifera virgifera* LeConte) development were selected for pooled transcriptome analysis to provide candidate target gene sequences for control by RNAi transgenic plant insect protection technology.

In one exemplification, total RNA was isolated from about 0.9 gm whole first-instar WCR larvae; (4 to 5 days post-hatch; held at 16 °C), and purified using the following phenol/TRI REAGENT®-based method (MOLECULAR RESEARCH CENTER, Cincinnati, OH):

Larvae were homogenized at room temperature in a 15 mL homogenizer with 10 mL of TRI REAGENT® until a homogenous suspension was obtained. Following 5 min. incubation at room temperature, the homogenate was dispensed into 1.5 mL microfuge tubes (1mL per tube), 200 μL of chloroform was added, and the mixture was vigorously shaken for 15 seconds. After allowing the extraction to sit at room temperature for 10 min, the phases were separated by centrifugation at 12,000 x g at 4 °C. The upper phase (comprising about 0.6 mL) was carefully transferred into another sterile 1.5 mL tube, and an equal volume of room temperature isopropanol was added. After incubation at room temperature for 5 to 10 min, the mixture was centrifuged 8 min at 12,000 x g (4 °C or 25 °C).

The supernatant was carefully removed and discarded, and the RNA pellet was washed twice by vortexing with 75% ethanol, with recovery by centrifugation for 5 min at 7,500 x g (4 °C or 25 °C) after each wash. The ethanol was carefully removed, the pellet was allowed to air-dry for 3 to 5 min, and then was dissolved in nuclease-free sterile water. RNA concentration was determined by measuring the absorbance (A) at 260 nm and 280 nm. A typical extraction from about 0.9 gm of larvae yielded over 1 mg of total RNA, with an $A_{260}/A_{280}$ ratio of 1.9. The RNA thus extracted was stored at -80 °C until further processed.

RNA quality was determined by running an aliquot through a 1% agarose gel. The agarose gel solution was made using autoclaved 10x TAE buffer (Tris-acetate EDTA; 1x concentration is 0.04 M Tris-acetate, 1 mM EDTA (ethylenediamine tetra-acetic acid sodium salt), pH 8.0) diluted with DEPC (diethyl pyrocarbonate)-treated water in an autoclaved container. 1x TAE was used as the running buffer. Before use, the electrophoresis tank and the well-forming comb were cleaned with RNaseAway™ (INVITROGEN INC., Carlsbad, CA). Two μL of RNA sample were mixed with 8 μL of TE buffer (10 mM Tris HCl pH 7.0; 1 mM EDTA) and 10 μL of RNA sample buffer (NOVAGEN® Catalog No 70606; EMD4 Bioscience, Gibbstown, NJ). The sample was heated at 70 °C for 3 min, cooled to room temperature, and 5 μL (containing 1 μg to 2 μg RNA) were loaded per well. Commercially available RNA molecular weight markers were simultaneously run in separate wells for molecular size comparison. The gel was run at 60 volts for 2 hrs.

A normalized cDNA library was prepared from the larval total RNA by a commercial service provider (EUROFINS MWG Operon, Huntsville, AL), using random priming. The normalized larval cDNA library was sequenced at 1/2 plate scale by GS FLX 454 Titanium™ series chemistry at EUROFINS MWG Operon, which resulted in over 600,000 reads with an average read length of 348 bp. 350,000 reads were assembled into over 50,000 contigs. Both the unassembled reads and the contigs were converted into BLASTable databases using the publicly available program, FORMATDB (available from NCBI).

Total RNA and normalized cDNA libraries were similarly prepared from materials harvested at other WCR developmental stages. A pooled transcriptome library for target gene screening was constructed by combining cDNA library members representing the various developmental stages.

Candidate genes for RNAi targeting were selected using information regarding lethal RNAi effects of particular genes in other insects such as *Drosophila* and *Tribolium.* These genes were hypothesized to be essential for survival and growth in coleopteran insects. Selected target gene homologs were identified in the transcriptome sequence database as described below. Full-length or partial sequences of the target genes were amplified by PCR to prepare templates for double-stranded RNA (dsRNA) production.

TBLASTN searches using candidate protein coding sequences were run against BLASTable databases containing the unassembled *Diabrotica* sequence reads or the assembled contigs. Significant hits to a *Diabrotica* sequence (defined as better than $e^{-20}$ for contig homologies and better than $e^{-10}$ for unassembled sequence read homologies) were confirmed using BLASTX against the NCBI non-redundant database. The results of this BLASTX search confirmed that the *Diabrotica* homolog candidate gene sequences identified in the TBLASTN search indeed comprised *Diabrotica* genes, or were the best hit to the non-*Diabrotica* candidate gene sequence present in the *Diabrotica* sequences. In a few cases, it was clear that some of the *Diabrotica* contigs or unassembled sequence reads selected by homology to a non-*Diabrotica* candidate gene overlapped, and that the assembly of the contigs had failed to join these overlaps. In those cases, Sequencher™ v4.9 (GENE CODES CORPORATION, Ann Arbor, MI) was used to assemble the sequences into longer contigs.

A candidate target gene encoding *Diabrotica rpl1* (SEQ ID NO:1 and SEQ ID NO:3) was identified as a gene that may lead to coleopteran pest mortality, inhibition of growth, or inhibition of development in WCR. RNA polymerase I-1 (*rpl1*) encodes the largest subunit of RNA polymerase I (RNAPI). Knackmuss et al. (1997) Mol. Gen. Genet. 253(5):529-34; Seither et al. (1997) Mol. Gen. Genet. 255(2):180-6. In eukaryotes, the transcription of the genome is carried out by three distinct classes of nuclear multi-subunit RNA polymerases (RNAP). RNAPI transcribes essential genes which are involved in cellular processes, such as ribosome and protein biogenesis. RNAPI activity is tightly co-regulated and dominates cellular transcription. The combined expression exceeds 80% of total RNA synthesis and results in 1-2 million ribosomes per cell generation.

*Rpl1* dsRNA transgenes can be combined with other dsRNA molecules to provide redundant RNAi targeting and synergistic RNAi effects. Transgenic corn events expressing dsRNA that targets *Rpl1* are useful for preventing root feeding damage by corn rootworm. *Rpl1* dsRNA transgenes represent new modes of action for combining with *Bacillus thuringiensis* insecticidal protein technology in Insect Resistance Management gene pyramids to mitigate against the development of rootworm populations resistant to either of these rootworm control technologies.

Full-length or partial clones of sequences of a *Diabrotica* candidate gene, herein referred to as *Rpl1,* were used to generate PCR amplicons for dsRNA synthesis.

**EXAMPLE 3: Amplification of Target Genes to produce dsRNA**

Primers were designed to amplify portions of coding regions of each target gene by PCR. **Table 1.** Where appropriate, a T7 phage promoter sequence (TTAATACGACTCACTATAGGGAGA; SEQ ID NO:9) was incorporated into the 5' ends of the amplified sense or antisense strands. **Table 1.** Total RNA was extracted from WCR using TRIzol® (Life Technologies, Grand Island, NY), where WCR larvae and adults were homogenized at room temperature in a 1.5 mL microfuge tube with 1 mL of TRIzol® using a Pestle Motor Mixer (Cole-Parmer, Vernon Hills, IL) until a homogenous suspension was obtained. Following 5 min. incubation at room temperature, the homogenate was centrifuged to remove cell debris and 1 mL supernatant was transferred to a new tube. 200 μL of chloroform was added, and the mixture was vigorously shaken for 15 seconds. After allowing the extraction to sit at room temperature for 2-3 min, the phases were separated by centrifugation at 12,000 x g at 4 °C. The upper phase (comprising about 0.6 mL) was carefully transferred into another sterile 1.5 mL tube, and 500 uL of room temperature isopropanol was added. After incubation at room temperature for 10 min, the mixture was centrifuged 10 min at 12,000 x g at 4 °C. The supernatant was carefully removed and discarded, and the RNA pellet was washed twice by vortexing with 75% ethanol, with recovery by centrifugation for 5 min at 7,500 x g (4 °C or 25 °C) after each wash. The ethanol was carefully removed, the pellet was allowed to air-dry for 3 to 5 min, and then was dissolved in nuclease-free sterile water.

Total RNA was then used to make first-strand cDNA with SuperScriptIII® First-Strand Synthesis System and manufacturers Oligo dT primed instructions (Life Technologies, Grand Island, NY). This first-strand cDNA was used as template for PCR reactions using opposing primers positioned to amplify all or part of the native target gene sequence. dsRNA was also amplified from a DNA clone comprising the coding region for a yellow fluorescent protein (YFP) (SEQ ID NO: 10; Shagin et al. (2004) Mol. Biol. Evol. 21(5):841-50).

**Table 1.** Primers and Primer Pairs used to amplify portions of coding regions of exemplary *rpl1* target gene and YFP negative control gene.

| | Gene ID | Primer ID | Sequence |
|---|---|---|---|
| **Pair 1** | *rpl1-1* reg1 | Dvv_Rpl1-1_For | TTAATACGACTCACTATAGGGAGACTGAGGTCA TAATTAGAGGTGGTG (SEQ ID NO:11) |
| | | Dvv_rpl1-1_Rev | TTAATACGACTCACTATAGGGAGAGATTGTACC ATCAATTGAAGATTATTTC (SEQ ID NO:12) |
| **Pair 2** | *rpl1-2* reg2 | Dvv_rpl1-2_For | TTAATACGACTCACTATAGGGAGAGTTATAAGG TAGAAAGACACATCAG (SEQ ID NO:13) |
| | | Dvv_rpl1-2_Rev | TTAATACGACTCACTATAGGGAGAGCGTAGAAT GGGTACGTATC (SEQ ID NO:14) |
| **Pair 3** | *rpl1-2* v1 | Dvv_rpl1-2 v1_For | TTAATACGACTCACTATAGGGAGAACCCTCCAC AAGATGAGTATGATG (SEQ ID NO:15) |
| | | Dvv_rpl1-2 v1_Rev | TTAATACGACTCACTATAGGGAGATTCGTCGCC GTCAAAATCGG (SEQ ID NO:16) |
| **Pair 4** | *rpl1-2* v2 | Dvv_rpl1-2 v2_For | TTAATACGACTCACTATAGGGAGAATGCCCCGT CCAGCCATCCTCAAAC (SEQ ID NO:17) |
| | | Dvv_rpl1-2 v2_Rev | TTAATACGACTCACTATAGGGAGAGCGTAGAAT GGGTACGTATC (SEQ ID NO:18) |

(continued)

| | Gene ID | Primer ID | Sequence |
|---|---|---|---|
| **Pair 5** | YFP | YFP-F_T7 | TTAATACGACTCACTATAGGGAGACACCATGGG CTCCAGCGGCGCCC (SEQ ID NO:25) |
| | | YFP-R_T7 | TTAATACGACTCACTATAGGGAGAAGATCTTGA AGGCGCTCTTCAGG (SEQ ID NO:28) |

**EXAMPLE 4: RNAi Constructs**

Template preparation by PCR and dsRNA synthesis

A strategy used to provide specific templates for *rpl1* and YFP dsRNA production is shown in **FIG. 1.** Template DNAs intended for use in *rpl1* dsRNA synthesis were prepared by PCR using the primer pairs in **Table 1** and (as PCR template) first-strand cDNA prepared from total RNA isolated from WCR eggs, first-instar larvae, or adults. For each selected *rpl1* and YFP target gene region, PCR amplifications introduced a T7 promoter sequence at the 5' ends of the amplified sense and antisense strands (the YFP segment was amplified from a DNA clone of the YFP coding region). The two PCR amplified fragments for each region of the target genes were then mixed in approximately equal amounts, and the mixture was used as transcription template for dsRNA production. *See* **FIG. 1.** The sequences of the dsRNA templates amplified with the particular primer pairs were: SEQ ID NO:5 (*rpl1-1* reg1), SEQ ID NO:6 (*rpl1-2* reg2), SEQ ID NO:7 (*rpl1-2* v1), SEQ ID NO:8 (*rpl1-2* v2) and YFP (SEQ ID NO:10). Double-stranded RNA for insect bioassay was synthesized and purified using an AMBION® MEGASCRIPT® RNAi kit following the manufacturer's instructions (INVITROGEN) or HiScube® T7 In Vitro Transcription Kit following the manufacturer's instructions (New England Biolabs, Ipswich, MA). The concentrations of dsRNAs were measured using a NANODROP™ 8000 spectrophotometer (THERMO SCIENTIFIC, Wilmington, DE).

Construction of plant transformation vectors

Entry vectors harboring a target gene construct for hairpin formation comprising segments of *rpl1* (SEQ ID NO:1 and SEQ ID NO:3) are assembled using a combination of chemically synthesized fragments (DNA2.0, Menlo Park, CA) and standard molecular cloning methods. Intramolecular hairpin formation by RNA primary transcripts is facilitated by arranging (within a single transcription unit) two copies of a target gene segment in opposite orientation to one another, the two segments being separated by a linker polynucleotide (*e.g.,* SEQ ID NO:83, and an ST-LS1 intron (Vancanneyt et al. (1990) Mol. Gen. Genet. 220(2):245-50)). Thus, the primary mRNA transcript contains the two *rpl1* gene segment sequences as large inverted repeats of one another, separated by the intron sequence. A copy of a promoter (*e.g.* maize ubiquitin 1, U.S. Patent No. 5,510,474; 35S from Cauliflower Mosaic Virus (CaMV); Sugarcane bacilliform badnavirus (ScBV) promoter; promoters from rice actin genes; ubiquitin promoters; pEMU; MAS; maize H3 histone promoter; ALS promoter; phaseolin gene promoter; *cab; rubisco*; *LAT52*; *Zm13*; and/or *apg*) is used to drive production of the primary mRNA hairpin transcript, and a fragment comprising a 3' untranslated region *(e.g.,* a maize peroxidase 5 gene (ZmPer5 3'UTR v2; U.S. Patent No. 6,699,984), AtUbi10, AtEf1, or StPinII) is used to terminate transcription of the hairpin-RNA-expressing gene.

Entry vector pDAB126156 comprises a *rpl1-2 v1*-RNA construct (SEQ ID NO:81) that comprises a segment of *rpl1* (SEQ ID NO:7).

Entry vectors are used in standard GATEWAY® recombination reactions with a typical binary destination vector to produce *rpl1* hairpin RNA expression transformation vectors for *Agrobacterium*-mediated maize embryo transformations.

The binary destination vector comprises a herbicide tolerance gene (aryloxyalknoate dioxygenase; AAD-1 v3) (U.S. Patent No. 7838733(B2), and Wright et al. (2010) Proc. Natl. Acad. Sci. U.S.A. 107:20240-5) under the regulation of a plant operable promoter (*e.g.,* sugarcane bacilliform badnavirus (ScBV) promoter (Schenk et al. (1999) Plant Mol. Biol. 39:1221-30) or ZmUbi1 (U.S. Patent 5,510,474)). A 5'UTR and intron are positioned between the 3' end of the promoter segment and the start codon of the *AAD-1* coding region. A fragment comprising a 3' untranslated region from a maize lipase gene (ZmLip 3'UTR; U.S. Patent No. 7,179,902) is used to terminate transcription of the *AAD-1* mRNA.

A negative control binary vector that comprises a gene that expresses a YFP protein is constructed by means of standard GATEWAY® recombination reactions with a typical binary destination vector and entry vector. The binary destination vector comprises a herbicide tolerance gene (aryloxyalknoate dioxygenase; AAD-1 v3) (as above) under the expression regulation of a maize ubiquitin 1 promoter (as above) and a fragment comprising a 3' untranslated region from a maize lipase gene (ZmLip 3'UTR; as above). The entry vector comprises a YFP coding region under the expression control of a maize ubiquitin 1 promoter (as above) and a fragment comprising a 3' untranslated region from a maize peroxidase 5 gene (as above).

**EXAMPLE** 5: **Screening of Candidate Target Genes**

Synthetic dsRNA designed to inhibit target gene sequences identified in EXAMPLE 2 caused mortality and growth inhibition when administered to WCR in diet-based assays. *rpl1-2* reg2, *rpl1-2* v1 and *rpl1-2* v2 were observed to exhibit greatly increased efficacy in this assay over other dsRNAs screened.

Replicated bioassays demonstrated that ingestion of dsRNA preparations derived from *rpl1-1* reg1, *rpl1-2* reg2, *rpl1-2* v1 and *rpl1-2* v2 each resulted in mortality and/or growth inhibition of western corn rootworm larvae. **Table 2** and **Table 3** show the results of diet-based feeding bioassays of WCR larvae following 9-day exposure to these dsRNAs, as well as the results obtained with a negative control sample of dsRNA prepared from a yellow fluorescent protein (YFP) coding region (SEQ ID NO: 10).

**Table 2.** Results of *rpl1* dsRNA diet feeding assays obtained with western corn rootworm larvae after 9 days of feeding. ANOVA analysis found significance differences in Mean % Mortality and Mean % Growth Inhibition (GI). Means were separated using the Tukey-Kramer test.

| Gene Name | Dose (ng/cm$^2$) | N | Mean (%Mortality) ± SEM* | Mean (GI) ± SEM |
|---|---|---|---|---|
| ***rpl1-1* reg1** | 500 | 2 | 5.88±5.88 (B) | 0.55±0.00 (A) |
| ***rpl1-2* reg2** | 500 | 10 | 77.67±8.10 (A) | 0.91±0.04 (A) |
| ***rpl1-2* v1** | 500 | 10 | 79.41±6.97 (A) | 0.93±0.03 (A) |
| ***rpl1-2* v2** | 500 | 10 | 65.55±4.97 (A) | 0.88±0.02 (A) |
| **TE**** | 0 | 23 | 13.88±1.40 (B) | 0.08±0.04 (B) |
| **WATER** | 0 | 23 | 13.59±2.06 (B) | -0.10±0.06 (B) |
| **YFP***** | 500 | 23 | 14.62±2.00 (B) | 0.08±0.06 (B) |

*SEM =Standard Error of the Mean. Letters in parentheses designate statistical levels. Levels not connected by same letter are significantly different (P<0.05).
**TE = Tris HCl (1 mM) plus EDTA (0.1 mM) buffer, pH7.2.
***YFP = Yellow Fluorescent Protein

**Table 3.** Summary of oral potency of *rpl1* dsRNA on WCR larvae (ng/cm$^2$).

| Gene Name | $LC_{50}$ | Range | $GI_{50}$ | Range |
|---|---|---|---|---|
| ***rpl1-2* v1** | 25.9 | 36.78 - 53.29 | 17.41 | 6.21-48.77 |
| ***rpl1-2* v2** | 176.65 | 255.29 - 399.79 | 58.52 | 32.69 - 104.73 |

It has previously been suggested that certain genes of *Diabrotica* spp. may be exploited for RNAi-mediated insect control. *See* U.S. Patent Publication No. 2007/0124836, which discloses 906 sequences, and U.S. Patent No. 7,612,194, which discloses 9,112 sequences. However, it was determined that many genes suggested to have utility for RNAi-mediated insect control are not efficacious in controlling *Diabrotica.* It was also determined that sequences *rpl1-1* reg1, *rpl1-2* reg2, *rpl1-2* v1 and *rpl1-2* v2 each provide surprising and unexpected superior control of *Diabrotica,* compared to other genes suggested to have utility for RNAi-mediated insect control.

For example, *annexin, beta spectrin* 2, and *mtRP-L4* were each suggested in U.S. Patent No. 7,612,194 to be efficacious in RNAi-mediated insect control. SEQ ID NO:19 is the DNA sequence of *annexin* region 1 (Reg 1) and SEQ ID NO:20 is the DNA sequence of *annexin* region 2 (Reg 2). SEQ ID NO:21 is the DNA sequence of *beta spectrin* 2 region 1 (Reg 1) and SEQ ID NO:22 is the DNA sequence of *beta spectrin* 2 region 2 (Reg2). SEQ ID NO:23 is the DNA sequence of *mtRP-L4* region 1 (Reg 1) and SEQ ID NO:24 is the DNA sequence of *mtRP-L4* region 2 (Reg 2). A YFP sequence (SEQ ID NO:10) was also used to produce dsRNA as a negative control.

Each of the aforementioned sequences was used to produce dsRNA by the methods of EXAMPLE 3. The strategy used to provide specific templates for dsRNA production is shown in **FIG. 2.** Template DNAs intended for use in dsRNA synthesis were prepared by PCR using the primer pairs in **Table 4** and (as PCR template) first-strand cDNA prepared from total RNA isolated from WCR first-instar larvae. (YFP was amplified from a DNA clone.) For each selected target gene region, two separate PCR amplifications were performed. The first PCR amplification introduced a T7 promoter sequence at the 5' end of the amplified sense strands. The second reaction incorporated the T7 promoter sequence at the 5' ends of the antisense strands. The two PCR amplified fragments for each region of the target genes were then mixed in approximately equal amounts, and the mixture was used as transcription template for dsRNA production. *See* **FIG. 2.** Double-stranded RNA was synthesized and purified using an AMBION® MEGAscupt® RNAi kit following the manufacturer's instructions (INVITROGEN). The concentrations of dsRNAs were measured using a NANODROP™ 8000 spectrophotometer (THERMO SCIENTIFIC, Wilmington, DE) and the dsRNAs were each tested by the same diet-based bioassay methods described above. **Table 4** lists the sequences of the primers used to produce the *annexin* Reg1, *annexin* Reg2, *beta spectrin* 2 Reg1, *beta spectrin* 2 Reg2, *mtRP-L4* Reg1, and *mtRP-L4* Reg2 dsRNA molecules. YFP primer sequences for use in the method depicted in **FIG. 2** are also listed in **Table 4. Table 5** presents the results of diet-based feeding bioassays of WCR larvae following 9-day exposure to these dsRNA molecules. Replicated bioassays demonstrated that ingestion of these dsRNAs resulted in no mortality or growth inhibition of western corn rootworm larvae above that seen with control samples of TE buffer, water, or YFP protein.

**Table 4.** Primers and Primer Pairs used to amplify portions of coding regions of genes.

| | Gene (Region) | Primer ID | Sequence |
|---|---|---|---|
| Pair 6 | YFP | YFP-F_T7 | TTAATACGACTCACTATAGGGAGACACCATGGGCTCCAGCGGCGCCC (SEQ ID NO:25) |
| | YFP | YFP-R | AGATCTTGAAGGCGCTCTTCAGG (SEQ ID NO:26) |
| Pair 7 | YFP | YFP-F | CACCATGGGCTCCAGCGGCGCCC (SEQ ID NO:27) |
| | YFP | YFP-R_T7 | TTAATACGACTCACTATAGGGAGAAGATCTTGAAGGCGCTCTTCAGG (SEQ ID NO:28) |
| Pair 8 | Annexin (Reg 1) | Ann-F1_T7 | TTAATACGACTCACTATAGGGAGAGCTCCAACAGTGGTTCCTTATC (SEQ ID NO:29) |
| | Annexin (Reg 1) | Ann-R1 | CTAATAATTCTTTTTTAATGTTCCTGAGG (SEQ ID NO:30) |
| Pair 9 | Annexin (Reg 1) | Ann-F1 | GCTCCAACAGTGGTTCCTTATC (SEQ ID NO:31) |
| | Annexin (Reg 1) | Ann-R1_T7 | TTAATACGACTCACTATAGGGAGACTAATAATTCTTTTTTAATGTTCCTGAGG (SEQ ID NO:32) |

(continued)

| | Gene (Region) | Primer ID | Sequence |
|---|---|---|---|
| Pair 10 | Annexin (Reg 2) | Ann-F2_T7 | TTAATACGACTCACTATAGGGAGATTGTTACAAGCTGGAGAACTTCTC (SEQ ID NO:33) |
| | Annexin (Reg 2) | Ann-R2 | CTTAACCAACAACGGCTAATAAGG (SEQ ID NO:34) |
| Pair 11 | Annexin (Reg 2) | Ann-F2 | TTGTTACAAGCTGGAGAACTTCTC (SEQ ID NO:35) |
| | Annexin (Reg 2) | Ann-R2T7 | TTAATACGACTCACTATAGGGAGACTTAACCAACAACGGCTAATAAGG (SEQ ID NO:36) |
| Pair 12 | Beta-spect2 (Reg 1) | Betasp2-F1_T7 | TTAATACGACTCACTATAGGGAGAAGATGTTGGCTGCATCTAGAGAA (SEQ ID NO:37) |
| | Beta-spect2 (Reg 1) | Betasp2-R1 | GTCCATTCGTCCATCCACTGCA (SEQ ID NO:38) |
| Pair 13 | Beta-spect2 (Reg 1) | Betasp2-F1 | AGATGTTGGCTGCATCTAGAGAA (SEQ ID NO:39) |
| | Beta-spect2 (Reg 1) | Betasp2-R1_T7 | TTAATACGACTCACTATAGGGAGAGTCCATTCGTCCATCCACTGCA (SEQ ID NO:40) |
| Pair 14 | Beta-spect2 (Reg 2) | Betasp2-F2_T7 | TTAATACGACTCACTATAGGGAGAGCAGATGAACACCAGCGAGAAA (SEQ ID NO:41) |
| | Beta-spect2 (Reg 2) | Betasp2-R2 | CTGGGCAGCTTCTTGTTTCCTC (SEQ ID NO:42) |
| Pair 15 | Beta-spect2 (Reg 2) | Betasp2-F2 | GCAGATGAACACCAGCGAGAAA (SEQ ID NO:43) |
| | Beta-spect2 (Reg 2) | Betasp2-R2_T7 | TTAATACGACTCACTATAGGGAGACTGGGCAGCTTCTTGTTTCCTC (SEQ ID NO:44) |
| Pair 16 | mtRP-L4 (Reg 1) | L4-F1_T7 | TTAATACGACTCACTATAGGGAGAAGTGAAATGTTAGCAAATATAACATCC (SEQ ID NO:45) |
| | mtRP-L4 (Reg 1) | L4-R1 | ACCTCTCACTTCAAATCTTGACTTTG (SEQ ID NO:46) |
| Pair 17 | mtRP-L4 (Reg 1) | L4-F1 | AGTGAAATGTTAGCAAATATAACATCC (SEQ ID NO:47) |
| | mtRP-L4 (Reg 1) | L4-R1_T7 | TTAATACGACTCACTATAGGGAGAACCTCTCACTTCAAATCTTGACTTTG (SEQ ID NO:48) |

(continued)

| | Gene (Region) | Primer ID | Sequence |
|---|---|---|---|
| Pair 18 | mtRP-L4 (Reg 2) | L4-F2_T7 | TTAATACGACTCACTATAGGGAGACAAAGTC AAGATTTGAAGTGAGAGGT (SEQ ID NO:49) |
| | mtRP-L4 (Reg 2) | L4-R2 | CTACAAATAAAACAAGAAGGACCCC (SEQ ID NO:50) |
| Pair 19 | mtRP-L4 (Reg 2) | L4-F2 | CAAAGTCAAGATTTGAAGTGAGAGGT (SEQ ID NO:51) |
| | mtRP-L4 (Reg 2) | L4-R2_T7 | TTAATACGACTCACTATAGGGAGACTACAAA TAAAACAAGAAGGACCCC (SEQ ID NO:52) |

**Table 5.** Results of diet feeding assays obtained with western corn rootworm larvae after 9 days.

| Gene Name | Dose (ng/cm$^2$) | Mean Live Larval Weight (mg) | Mean % Mortality | Mean Growth Inhibition |
|---|---|---|---|---|
| ***annexin*-Reg 1** | 1000 | 0.545 | 0 | -0.262 |
| ***annexin*-Reg 2** | 1000 | 0.565 | 0 | -0.301 |
| ***beta spectrin2* Reg 1** | 1000 | 0.340 | 12 | -0.014 |
| ***beta spectrin2* Reg 2** | 1000 | 0.465 | 18 | -0.367 |
| ***mtRP-L4* Reg 1** | 1000 | 0.305 | 4 | -0.168 |
| ***mtRP-L4* Reg 2** | 1000 | 0.305 | 7 | -0.180 |
| **TE buffer*** | 0 | 0.430 | 13 | 0.000 |
| **Water** | 0 | 0.535 | 12 | 0.000 |
| **YFP**** | 1000 | 0.480 | 9 | -0.386 |
| *TE = Tris HCl (10 mM) plus EDTA (1 mM) buffer, pH8.<br>**YFP = Yellow Fluorescent Protein | | | | |

**EXAMPLE 6: Production of Transgenic Maize Tissues Comprising Insecticidal Hairpin dsRNAs**

*Agrobacterium-mediated* Transformation. Transgenic maize cells, tissues, and plants that produce one or more insecticidal dsRNA molecules (for example, at least one dsRNA molecule including a dsRNA molecule targeting a gene comprising *rpl1*; SEQ ID NO:1 and SEQ ID NO:3) through expression of a chimeric gene stably-integrated into the plant genome are produced following *Agrobacterium-mediated* transformation. Maize transformation methods employing superbinary or binary transformation vectors are known in the art, as described, for example, in U.S. Patent 8,304,604, which is herein incorporated by reference in its entirety. Transformed tissues are selected by their ability to grow on Haloxyfop-containing medium and are screened for dsRNA production, as appropriate. Portions of such transformed tissue cultures may be presented to neonate corn rootworm larvae for bioassay, essentially as described in EXAMPLE 1.

*Agrobacterium* Culture Initiation. Glycerol stocks of *Agrobacterium* strain DAt13192 cells (WO 2012/016222A2) harboring a binary transformation vector described above (EXAMPLE 4) are streaked on AB minimal medium plates (Watson, et al. (1975) J. Bacteriol. 123:255-264) containing appropriate antibiotics, and are grown at 20 °C for 3 days. The cultures are then streaked onto YEP plates (gm/L: yeast extract, 10; Peptone, 10; NaCl, 5) containing the same antibiotics and are incubated at 20 °C for 1 day.

*Agrobacterium* culture. On the day of an experiment, a stock solution of Inoculation Medium and acetosyringone is prepared in a volume appropriate to the number of constructs in the experiment and pipetted into a sterile, disposable, 250 mL flask. Inoculation Medium (Frame et al. (2011) Genetic Transformation Using Maize Immature Zygotic Embryos.

IN Plant Embryo Culture Methods and Protocols: Methods in Molecular Biology. T. A. Thorpe and E. C. Yeung, (Eds), Springer Science and Business Media, LLC. pp 327-341) contains: 2.2 gm/L MS salts; 1X ISU Modified MS Vitamins (Frame *et al., ibid.*) 68.4 gm/L sucrose; 36 gm/L glucose; 115 mg/L L-proline; and 100 mg/L myo-inositol; at pH 5.4.) Acetosyringone is added to the flask containing Inoculation Medium to a final concentration of 200 μM from a 1 M stock solution in 100% dimethyl sulfoxide, and the solution is thoroughly mixed.

For each construct, 1 or 2 inoculating loops-full of *Agrobacterium* from the YEP plate are suspended in 15 mL Inoculation Medium/acetosyringone stock solution in a sterile, disposable, 50 mL centrifuge tube, and the optical density of the solution at 550 nm ($OD_{550}$) is measured in a spectrophotometer. The suspension is then diluted to $OD_{550}$ of 0.3 to 0.4 using additional Inoculation Medium/acetosyringone. The tube of *Agrobacterium* suspension is then placed horizontally on a platform shaker set at about 75 rpm at room temperature and shaken for 1 to 4 hours while embryo dissection is performed.

Ear sterilization and embryo isolation. Maize immature embryos are obtained from plants of *Zea mays* inbred line B104 (Hallauer et al. (1997) Crop Science 37:1405-1406), grown in the greenhouse and self- or sib-pollinated to produce ears. The ears are harvested approximately 10 to 12 days post-pollination. On the experimental day, de-husked ears are surface-sterilized by immersion in a 20% solution of commercial bleach (ULTRA CLOROX® Germicidal Bleach, 6.15% sodium hypochlorite; with two drops of TWEEN 20) and shaken for 20 to 30 min, followed by three rinses in sterile deionized water in a laminar flow hood. Immature zygotic embryos (1.8 to 2.2 mm long) are aseptically dissected from each ear and randomly distributed into microcentrifuge tubes containing 2.0 mL of a suspension of appropriate *Agrobacterium* cells in liquid Inoculation Medium with 200 μM acetosyringone, into which 2 μL of 10% BREAK-THRU® S233 surfactant (EVONIK INDUSTRIES; Essen, Germany) is added. For a given set of experiments, embryos from pooled ears are used for each transformation.

*Agrobacterium* co-cultivation. Following isolation, the embryos are placed on a rocker platform for 5 minutes. The contents of the tube are then poured onto a plate of Cocultivation Medium, which contains 4.33 gm/L MS salts; 1X ISU Modified MS Vitamins; 30 gm/L sucrose; 700 mg/L L-proline; 3.3 mg/L Dicamba in KOH (3,6-dichloro-o-anisic acid or 3,6-dichloro-2-methoxybenzoic acid); 100 mg/L myo-inositol; 100 mg/L Casein Enzymatic Hydrolysate; 15 mg/L $AgNO_3$; 200 μM acetosyringone in DMSO; and 3 gm/L GELZAN™, at pH 5.8. The liquid *Agrobacterium* suspension is removed with a sterile, disposable, transfer pipette. The embryos are then oriented with the scutellum facing up using sterile forceps with the aid of a microscope. The plate is closed, sealed with 3M™ MICROPORE™ medical tape, and placed in an incubator at 25 °C with continuous light at approximately 60 μmol $m^{-2}s^{-1}$ of Photosynthetically Active Radiation (PAR).

Callus Selection and Regeneration of Transgenic Events. Following the CoCultivation period, embryos are transferred to Resting Medium, which is composed of 4.33 gm/L MS salts; 1X ISU Modified MS Vitamins; 30 gm/L sucrose; 700 mg/L L-proline; 3.3 mg/L Dicamba in KOH; 100 mg/L myo-inositol; 100 mg/L Casein Enzymatic Hydrolysate; 15 mg/L $AgNO_3$; 0.5 gm/L MES (2-(N-morpholino)ethanesulfonic acid monohydrate; PHYTOTECHNOLOGIES LABR.; Lenexa, KS); 250 mg/L Carbenicillin; and 2.3 gm/L GELZAN™; at pH 5.8. No more than 36 embryos are moved to each plate. The plates are placed in a clear plastic box and incubated at 27 °C with continuous light at approximately 50 μmol $m^{-2}s^{-1}$ PAR for 7 to 10 days. Callused embryos are then transferred (<18/plate) onto Selection Medium I, which is comprised of Resting Medium (above) with 100 nM R-Haloxyfop acid (0.0362 mg/L; for selection of calli harboring the *AAD-1* gene). The plates are returned to clear boxes and incubated at 27 °C with continuous light at approximately 50 μmol $m^{-2}s^{-1}$ PAR for 7 days. Callused embryos are then transferred (<12/plate) to Selection Medium II, which is comprised of Resting Medium (above) with 500 nM R-Haloxyfop acid (0.181 mg/L). The plates are returned to clear boxes and incubated at 27 °C with continuous light at approximately 50 μmol $m^{-2}s^{-1}$ PAR for 14 days. This selection step allows transgenic callus to further proliferate and differentiate.

Proliferating, embryogenic calli are transferred (<9/plate) to Pre-Regeneration medium. Pre-Regeneration Medium contains 4.33 gm/L MS salts; 1X ISU Modified MS Vitamins; 45 gm/L sucrose; 350 mg/L L-proline; 100 mg/L myo-inositol; 50 mg/L Casein Enzymatic Hydrolysate; 1.0 mg/L $AgNO_3$; 0.25 gm/L MES; 0.5 mg/L naphthaleneacetic acid in NaOH; 2.5 mg/L abscisic acid in ethanol; 1 mg/L 6-benzylaminopurine; 250 mg/L Carbenicillin; 2.5 gm/L GELZAN™; and 0.181 mg/L Haloxyfop acid; at pH 5.8. The plates are stored in clear boxes and incubated at 27 °C with continuous light at approximately 50 μmol $m^{-2}s^{-1}$ PAR for 7 days. Regenerating calli are then transferred (<6/plate) to Regeneration Medium in PHYTATRAYS™ (SIGMA-ALDRICH) and incubated at 28 °C with 16 hours light/8 hours dark per day (at approximately 160 μmol $m^{-2}s^{-1}$ PAR) for 14 days or until shoots and roots develop. Regeneration Medium contains 4.33 gm/L MS salts; 1X ISU Modified MS Vitamins; 60 gm/L sucrose; 100 mg/L myo-inositol; 125 mg/L Carbenicillin; 3 gm/L GELLAN™ gum; and 0.181 mg/L R-Haloxyfop acid; at pH 5.8. Small shoots with primary roots are then isolated and transferred to Elongation Medium without selection. Elongation Medium contains 4.33 gm/L MS salts; 1X ISU Modified MS Vitamins; 30 gm/L sucrose; and 3.5 gm/L GELRITE™: at pH 5.8.

Transformed plant shoots selected by their ability to grow on medium containing Haloxyfop are transplanted from PHYTATRAYS™ to small pots filled with growing medium (PROMIX BX; PREMIER TECH HORTICULTURE), covered with cups or HUMI-DOMES (ARCO PLASTICS), and then hardened-off in a CONVIRON growth chamber (27 °C day/24 °C night, 16-hour photoperiod, 50-70% RH, 200 μmol $m^{-2}s^{-1}$ PAR). In some instances, putative transgenic plantlets are analyzed for transgene relative copy number by quantitative real-time PCR assays using primers designed to detect the *AAD1* herbicide tolerance gene integrated into the maize genome. Further, RT-qPCR assays are used to detect the presence of the linker sequence and/or of target sequence in putative transformants. Selected transformed plantlets are then moved into a greenhouse for further growth and testing.

Transfer and establishment of $T_0$ plants in the greenhouse for bioassay and seed production. When plants reach the V3-V4 stage, they are transplanted into IE CUSTOM BLEND (PROFILE/METRO MIX 160) soil mixture and grown to flowering in the greenhouse (Light Exposure Type: Photo or Assimilation; High Light Limit: 1200 PAR; 16-hour day length; 27 °C day/24 °C night).

Plants to be used for insect bioassays are transplanted from small pots to TINUS™ 350-4 ROOTRAINERS® (SPENCER-LEMAIRE INDUSTRIES, Acheson, Alberta, Canada;) (one plant per event per ROOTRAINER®). Approximately four days after transplanting to ROOTRAINERS®, plants are infested for bioassay.

Plants of the $T_1$ generation are obtained by pollinating the silks of $T_0$ transgenic plants with pollen collected from plants of non-transgenic inbred line B104 or other appropriate pollen donors, and planting the resultant seeds. Reciprocal crosses are performed when possible.

**EXAMPLE** 7: **Molecular Analyses of Transgenic Maize Tissues**

Molecular analyses (e.g. RT-qPCR) of maize tissues are performed on samples from leaves that were collected from greenhouse grown plants on the day before or same day that root feeding damage is assessed.

Results of RT-qPCR assays for the target gene are used to validate expression of the transgene. Results of RT-qPCR assays for intervening sequence between repeat sequences (which is integral to the formation of dsRNA hairpin molecules) in expressed RNAs are alternatively used to validate the presence of hairpin transcripts. Transgene RNA expression levels are measured relative to the RNA levels of an endogenous maize gene.

DNA qPCR analyses to detect a portion of the *AAD1* coding region in gDNA are used to estimate transgene insertion copy number. Samples for these analyses are collected from plants grown in environmental chambers. Results are compared to DNA qPCR results of assays designed to detect a portion of a single-copy native gene, and simple events (having one or two copies of the transgenes) are advanced for further studies in the greenhouse.

Additionally, qPCR assays designed to detect a portion of the spectinomycin-resistance gene (*SpecR*; harbored on the binary vector plasmids outside of the T-DNA) are used to determine if the transgenic plants contain extraneous integrated plasmid backbone sequences.

RNA transcript expression level: target qPCR. Callus cell events or transgenic plants are analyzed by real time quantitative PCR (qPCR) of the target sequence to determine the relative expression level of the transgene, as compared to the transcript level of an internal maize gene (for example, GENBANK Accession No. BT069734), which encodes a TIP41-like protein *(i.e.* a maize homolog of GENBANK Accession No. AT4G34270; having a tBLASTX score of 74% identity). RNA is isolated using Norgen BioTek™ Total RNA Isolation Kit (Norgen, Thorold, ON). The total RNA is subjected to an On-Column™ DNase1 treatment according to the kit's suggested protocol. The RNA is then quantified on a NANODROP 8000 spectrophotometer (THERMO SCIENTIFIC) and concentration is normalized to 50 ng/μL. First strand cDNA is prepared using a HIGH CAPACITY cDNA SYNTHESIS KIT (INVITROGEN) in a 10 μL reaction volume with 5 μL denatured RNA, substantially according to the manufacturer's recommended protocol. The protocol is modified slightly to include the addition of 10 μL of 100 μM T20VN oligonucleotide (IDT) (TTTTTTTTTTTTTTTTTTTTVN, where V is A, C, or G, and N is A, C, G, or T; SEQ ID NO:54) into the 1 mL tube of random primer stock mix, in order to prepare a working stock of combined random primers and oligo dT.

Following cDNA synthesis, samples are diluted 1:3 with nuclease-free water, and stored at -20 °C until assayed.

Separate real-time PCR assays for the target gene and TIP41-like transcript are performed on a LIGHTCYCLER™ 480 (ROCHE DIAGNOSTICS, Indianapolis, IN) in 10 μL reaction volumes. For the target gene assay, reactions are run with Primers rpl1 FWD Set 1 (SEQ ID NO:55) and rpl1 REV Set 1 (SEQ ID NO:56), and an IDT Custom Oligo probe rpl1 PRB Set1, labeled with FAM and double quenched with Zen and Iowa Black quenchers. For the TIP41-like reference gene assay, primers TIPmxF (SEQ ID NO:57) and TIPmxR (SEQ ID NO:58), and Probe HXTIP (SEQ ID NO:59) labeled with HEX (hexachlorofluorescein) are used.

All assays include negative controls of no-template (mix only). For the standard curves, a blank (water in source well) is also included in the source plate to check for sample cross-contamination. Primer and probe sequences are set forth in **Table 6.** Reaction components recipes for detection of the various transcripts are disclosed in **Table 7,** and PCR reactions conditions are summarized in **Table 8.** The FAM (6-Carboxy Fluorescein Amidite) fluorescent moiety is excited at 465 nm and fluorescence is measured at 510 nm; the corresponding values for the HEX (hexachlorofluorescein) fluorescent moiety are 533 nm and 580 nm.

**Table 6.** Oligonucleotide sequences used for molecular analyses of transcript levels in transgenic maize.

| Target | Oligonucleotide | Sequence |
|---|---|---|
| rpl1 | RPI-2v1 FWD Set 1 | CCTCCACAAGATGAGTATGATGG (SEQ ID NO:55) |
| rpl1 | RPI-2v1 REV Set 1 | GAGGTGCACGAGAGATTCATAC (SEQ ID NO:56) |
| rll1 | RPI-2v1 PRB Set 1 | /56-FAM/AAAGTCTTA/ZEN/CCCTGGTCGACGTTCC/3IABkFQ/ (SEQ ID NO:82) |
| TIP41 | TIPmxF | TGAGGGTAATGCCAACTGGTT (SEQ NO:57) |
| TIP41 | TIPmxR | GCAATGTAACCGAGTGTCTCTCAA (SEQ ID NO:58) |
| TIP41 | HXTIP (HEX-Probe) | TTTTTGGCTTAGAGTTGATGGTGTACTGATGA (SEQ ID NO:59) |
| *TIP41-like protein. | | |

**Table 7.** PCR reaction recipes for transcript detection.

| | *rpl1* | TIP-like Gene |
|---|---|---|
| **Component** | **Final Concentration** | |
| Roche Buffer | 1X | 1X |
| *rpl1* F | 0.4 μM | 0 |
| *rpl1* R | 0.4 μM | 0 |
| *rpl1* FAM | 0.2 μM | 0 |
| HEXtipZM F | 0 | 0.4 μM |
| HEXtipZM R | 0 | 0.4 μM |
| HEXtipZMP (HEX) | 0 | 0.2 μM |
| cDNA (2.0 μL) | NA | NA |
| Water | To 10 μL | To 10 μL |

**Table 8.** Thermocycler conditions for RNA qPCR.

| Target Gene and TIP41-like Gene Detection | | | |
|---|---|---|---|
| **Process** | **Temp.** | **Time** | **No. Cycles** |
| Target Activation | 95 °C | 10 min | 1 |
| Denature | 95 °C | 10 sec | 40 |
| Extend | 60 °C | 40 sec | |
| Acquire FAM or HEX | 72 °C | 1 sec | |
| Cool | 40 °C | 10 sec | 1 |

Data are analyzed using LIGHTCYCLER™ Software v1.5 by relative quantification using a second derivative max algorithm for calculation of Cq values according to the supplier's recommendations. For expression analyses, expression values are calculated using the ΔΔCt method *(i.e.,* 2-(Cq TARGET - Cq REF)), which relies on the comparison of differences of Cq values between two targets, with the base value of 2 being selected under the assumption that, for optimized PCR reactions, the product doubles every cycle.

Transcript size and integrity: Northern Blot Assay. In some instances, additional molecular characterization of the transgenic plants is obtained by the use of Northern Blot (RNA blot) analysis to determine the molecular size of the *rpl1* linker RNA in transgenic plants expressing a *rpl1* linker dsRNA.

All materials and equipment are treated with RNaseZAP (AMBION/INVITROGEN) before use. Tissue samples (100 mg to 500 mg) are collected in 2 mL SAFELOCK EPPENDORF tubes, disrupted with a KLECKO™ tissue pulverizer (GARCIA MANUFACTURING, Visalia, CA) with three tungsten beads in 1 mL of TRIZOL (INVITROGEN) for 5 min, then incubated at room temperature (RT) for 10 min. Optionally, the samples are centrifuged for 10 min at 4 °C at 11,000 rpm and the supernatant is transferred into a fresh 2 mL SAFELOCK EPPENDORF tube. After 200 μL chloroform are added to the homogenate, the tube is mixed by inversion for 2 to 5 min, incubated at RT for 10 minutes, and centrifuged at 12,000 x g for 15 min at 4 °C. The top phase is transferred into a sterile 1.5 mL EPPENDORF tube, 600 μL of 100% isopropanol is added, followed by incubation at RT for 10 min to 2 hr, and then centrifuged at 12,000 x g for 10 min at 4 °C to 25 °C. The supernatant is discarded and the RNA pellet is washed twice with 1 mL 70% ethanol, with centrifugation at 7,500 x g for 10 min at 4 °C to 25 °C between washes. The ethanol is discarded and the pellet is briefly air dried for 3 to 5 min before resuspending in 50 μL of nuclease-free water.

Total RNA is quantified using the NANODROP 8000® (THERMO-FISHER) and samples are normalized to 5 μg/10 μL. 10 μL of glyoxal (AMBION/INVITROGEN) is then added to each sample. Five to 14 ng of DIG RNA standard marker mix (ROCHE APPLIED SCIENCE, Indianapolis, IN) are dispensed and added to an equal volume of glyoxal. Samples and marker RNAs are denatured at 50 °C for 45 min and stored on ice until loading on a 1.25% SEAKEM GOLD agarose (LONZA, Allendale, NJ) gel in NORTHERNMAX 10 X glyoxal running buffer (AMBION/INVITROGEN). RNAs are separated by electrophoresis at 65 volts/30 mA for 2 hours and 15 minutes.

Following electrophoresis, the gel is rinsed in 2X SSC for 5 min and imaged on a GEL DOC station (BIORAD, Hercules, CA), then the RNA is passively transferred to a nylon membrane (MILLIPORE) overnight at RT, using 10X SSC as the transfer buffer (20X SSC consists of 3 M sodium chloride and 300 mM trisodium citrate, pH 7.0). Following the transfer, the membrane is rinsed in 2X SSC for 5 minutes, the RNA is UV-crosslinked to the membrane (AGILENT/STRATAGENE), and the membrane is allowed to dry at room temperature for up to 2 days.

The membrane is pre-hybridized in ULTRAHYB™ buffer (AMBION/INVITROGEN) for 1 to 2 hr. The probe consists of a PCR amplified product containing the sequence of interest, (for example, the antisense sequence portion of SEQ ID NOs:5- 8 or 81, as appropriate) labeled with digoxigenin by means of a ROCHE APPLIED SCIENCE DIG procedure. Hybridization in recommended buffer is overnight at a temperature of 60 °C in hybridization tubes. Following hybridization, the blot is subjected to DIG washes, wrapped, exposed to film for 1 to 30 minutes, then the film is developed, all by methods recommended by the supplier of the DIG kit.

Transgene copy number determination. Maize leaf pieces approximately equivalent to 2 leaf punches are collected in 96-well collection plates (QIAGEN). Tissue disruption is performed with a KLECKO™ tissue pulverizer (GARCIA MANUFACTURING, Visalia, CA) in BIOSPRINT96 AP1 lysis buffer (supplied with a BIOSPRINT96 PLANT KIT; QIAGEN) with one stainless steel bead. Following tissue maceration, gDNA is isolated in high throughput format using a BIOSPRINT96 PLANT KIT and a BIOSPRINT96 extraction robot. gDNA is diluted 1:3 DNA:water prior to setting up the qPCR reaction.

qPCR analysis. Transgene detection by hydrolysis probe assay is performed by real-time PCR using a LIGHTCYCLER®480 system. Oligonucleotides to be used in hydrolysis probe assays to detect the target gene *(e.g., rpl1)*, the linker sequence, and/or to detect a portion of the SpecR gene *(i.e.,* the spectinomycin resistance gene borne on the binary vector plasmids; SEQ ID NO:60; SPC1 oligonucleotides in **Table 9),** are designed using LIGHTCYCLER® PROBE DESIGN SOFTWARE 2.0. Further, oligonucleotides to be used in hydrolysis probe assays to detect a segment of the AAD-1 herbicide tolerance gene (SEQ ID NO:61; GAAD1 oligonucleotides in **Table 9)** are designed using PRIMER EXPRESS software (APPLIED BIOSYSTEMS). **Table 9** shows the sequences of the primers and probes. Assays are multiplexed with reagents for an endogenous maize chromosomal gene (Invertase (SEQ ID NO:62; GENBANK Accession No: U16123; referred to herein as IVR1), which serves as an internal reference sequence to ensure gDNA is present in each assay. For amplification, LIGHTCYCLER®480 PROBES MASTER mix (ROCHE APPLIED SCIENCE) is prepared at 1x final concentration in a 10 μL volume multiplex reaction containing 0.4 μM of each primer and 0.2 μM of each probe **(Table 10).** A two step amplification reaction is performed as outlined in **Table 11.** Fluorophore activation and emission for the FAM- and HEX-labeled probes are as described above; CY5 conjugates are excited maximally at 650 nm and fluoresce maximally at 670 nm.

Cp scores (the point at which the fluorescence signal crosses the background threshold) are determined from the real time PCR data using the fit points algorithm (LIGHTCYCLER® SOFTWARE release 1.5) and the Relative Quant module (based on the ΔΔCt method). Data are handled as described previously (above; RNA qPCR).

**Table 9.** Sequences of primers and probes (with fluorescent conjugate) used for gene copy number determinations and binary vector plasmid backbone detection.

| Name | Sequence |
|---|---|
| GAAD1-F | TGTTCGGTTCCCTCTACCAA (SEQ ID NO:63) |
| GAAD1-R | CAACATCCATCACCTTGACTGA (SEQ NO:64) |
| GAAD1-P (FAM) | CACAGAACCGTCGCTTCAGCAACA (SEQ ID NO:65) |

(continued)

| Name | Sequence |
|---|---|
| IVR1-F | TGGCGGACGACGACTTGT (SEQ NO:66) |
| IVR1-R | AAAGTTTGGAGGCTGCCGT (SEQ ID NO:67) |
| IVR1-P (HEX) | CGAGCAGACCGCCGTGTACTTCTACC (SEQ NO:68) |
| SPC1A | CTTAGCTGGATAACGCCAC (SEQ NO:69) |
| SPC1S | GACCGTAAGGCTTGATGAA (SEQ NO:70) |
| TQSPEC (CY5*) | CGAGATTCTCCGCGCTGTAGA (SEQ ID NO:71) |
| LoopF | GGAACGAGCTGCTTGCGTAT (SEQ ID NO:72) |
| LoopR | CACGGTGCAGCTGATTGATG (SEQ ID NO:73) |
| Loop FAM | TCCCTTCCGTAGTCAGAG (SEQ NO:74) |
| CY5 = Cyanine-5 | |

**Table 10.** Reaction components for gene copy number analyses and plasmid backbone detection.

| Component | Amt. (μL) | Stock | Final Conc'n |
|---|---|---|---|
| 2x Buffer | 5.0 | 2x | 1x |
| Appropriate Forward Primer | 0.4 | 10 μM | 0.4 |
| Appropriate Reverse Primer | 0.4 | 10 μM | 0.4 |
| Appropriate Probe | 0.4 | 5 μM | 0.2 |
| IVR1-Forward Primer | 0.4 | 10 μM | 0.4 |
| IVR1-Reverse Primer | 0.4 | 10 μM | 0.4 |
| IVR1-Probe | 0.4 | 5 μM | 0.2 |
| $H_2O$ | 0.6 | NA* | NA |
| gDNA | 2.0 | ND** | ND |
| **Total** | 10.0 | | |
| *NA = Not Applicable<br>**ND = Not Determined | | | |

**Table 11.** Thermocycler conditions for DNA qPCR.

| Genomic copy number analyses | | | |
|---|---|---|---|
| **Process** | **Temp.** | **Time** | **No. Cycles** |
| Target Activation | 95 °C | 10 min | 1 |
| Denature | 95 °C | 10 sec | 40 |
| Extend & Acquire FAM, HEX, or CY5 | 60 °C | 40 sec | |
| Cool | 40 °C | 10 sec | 1 |

**EXAMPLE 8: Bioassay of Transgenic Maize**

Insect Bioassays. Bioactivity of dsRNA of the subject invention produced in plant cells is demonstrated by bioassay methods. *See, e.g.,* Baum et al. (2007) Nat. Biotechnol. 25(11): 1322-1326. One is able to demonstrate efficacy, for example, by feeding various plant tissues or tissue pieces derived from a plant producing an insecticidal dsRNA to target insects in a controlled feeding environment. Alternatively, extracts are prepared from various plant tissues derived from a plant producing the insecticidal dsRNA, and the extracted nucleic acids are dispensed on top of artificial diets for bioassays as previously described herein. The results of such feeding assays are compared to similarly conducted bioassays that employ appropriate control tissues from host plants that do not produce an insecticidal dsRNA, or to other control samples. Growth and survival of target insects on the test diet is reduced compared to that of the control group.

Insect Bioassays with Transgenic Maize Events. Two western corn rootworm larvae (1 to 3 days old) hatched from washed eggs are selected and placed into each well of the bioassay tray. The wells are then covered with a "PULL N' PEEL " tab cover (BIO-CV-16, BIO-SERV) and placed in a 28 °C incubator with an 18 hr:6 hr light:dark cycle. Nine days after the initial infestation, the larvae are assessed for mortality, which is calculated as the percentage of dead insects out of the total number of insects in each treatment. The insect samples are frozen at -20 °C for two days, then the insect larvae from each treatment are pooled and weighed. The percent of growth inhibition is calculated as the mean weight of the experimental treatments divided by the mean of the average weight of two control well treatments. The data are expressed as a Percent Growth Inhibition (of the negative controls). Mean weights that exceed the control mean weight are normalized to zero.

Insect bioassays in the greenhouse. Western corn rootworm (WCR, *Diabrotica virgifera virgifera* LeConte) eggs are received in soil from CROP CHARACTERISTICS (Farmington, MN). WCR eggs are incubated at 28 °C for 10 to 11 days. Eggs are washed from the soil, placed into a 0.15% agar solution, and the concentration is adjusted to approximately 75 to 100 eggs per 0.25 mL aliquot. A hatch plate is set up in a Petri dish with an aliquot of egg suspension to monitor hatch rates.

The soil around the maize plants growing in ROOTRANERS® is infested with 150 to 200 WCR eggs. The insects are allowed to feed for 2 weeks, after which time a "Root Rating" is given to each plant. A Node-Injury Scale is utilized for grading, essentially according to Oleson et al. (2005) J. Econ. Entomol. 98:1-8. Plants passing this bioassay, showing reduced injury, are transplanted to 5-gallon pots for seed production. Transplants are treated with insecticide to prevent further rootworm damage and insect release in the greenhouses. Plants are hand pollinated for seed production. Seeds produced by these plants are saved for evaluation at the $T_1$ and subsequent generations of plants.

Transgenic negative control plants are generated by transformation with vectors harboring genes designed to produce a yellow fluorescent protein (YFP). Non-transformed negative control plants are grown from seeds of parental corn varieties from which the transgenic plants were produced. Bioassays are conducted with negative controls included in each set of plant materials.

### EXAMPLE 9: Transgenic *Zea mays* Comprising Coleopteran Pest Sequences

10-20 transgenic $T_0$ *Zea mays* plants are generated as described in EXAMPLE 6. A further 10-20 $T_1$ *Zea mays* independent lines expressing hairpin dsRNA for an RNAi construct are obtained for corn rootworm challenge. Hairpin dsRNA comprise a portion of SEQ ID NO:1 and/or SEQ ID NO:3 (*e.g.,* the hairpin dsRNA transcribed from SEQ ID NO:81). Additional hairpin dsRNAs are derived, for example, from coleopteran pest sequences such as, for example, Caf1-180 (U.S. Patent Application Publication No. 2012/0174258); VatpaseC (U.S. Patent Application Publication No. 2012/0174259); Rho1 (U.S. Patent Application Publication No. 2012/0174260); VatpaseH (U.S. Patent Application Publication No. 2012/0198586); PPI-87B (U.S. Patent Application Publication No. 2013/0091600); RPA70 (U.S. Patent Application Publication No. 2013/0091601); RPS6 (U.S. Patent Application Publication No. 2013/0097730); *RNA polymerase II215* (U.S. Patent Application No. 62/133202); *RNA polymerase II33* (U.S. Patent Application No.62/133210); *ROP* (U.S. Patent Application No. 14/577,811); *RNAPII140* (U.S. Patent Application No. 14/577,854); *ncm* (U.S. Patent Application No. 62/095487); *Dre4* (U.S. Patent Application No. 14/705,807); *COPI alpha* (U.S. Patent Application No. 62/063,199); *COPI beta* (U.S. Patent Application No. 62/063,203); *COPI gamma* (U.S. Patent Application No. 62/063,192); or *COPI delta* (U.S. Patent Application No. 62/063,216). These are confirmed through RT-PCR or other molecular analysis methods.

Total RNA preparations from selected independent $T_1$ lines are optionally used for RT-PCR with primers designed to bind in the linker of the hairpin expression cassette in each of the RNAi constructs. In addition, specific primers for each target gene in an RNAi construct are optionally used to amplify and confirm the production of the pre-processed mRNA required for siRNA production *in planta.* The amplification of the desired bands for each target gene confirms the expression of the hairpin RNA in each transgenic *Zea mays* plant. Processing of the dsRNA hairpin of the target genes into siRNA is subsequently optionally confirmed in independent transgenic lines using RNA blot hybridizations.

Moreover, RNAi molecules having mismatch sequences with more than 80% sequence identity to target genes affect corn rootworms in a way similar to that seen with RNAi molecules having 100% sequence identity to the target genes. The pairing of mismatch sequence with native sequences to form a hairpin dsRNA in the same RNAi construct delivers plant-processed siRNAs capable of affecting the growth, development, and viability of feeding coleopteran pests.

*In planta* delivery of dsRNA, siRNA, or miRNA corresponding to target genes and the subsequent uptake by coleopteran pests through feeding results in down-regulation of the target genes in the coleopteran pest through RNA-mediated gene silencing. When the function of a target gene is important at one or more stages of development, the growth and/or development of the coleopteran pest is affected, and in the case of at least one of WCR, NCR, SCR, MCR, D. *balteata* LeConte, *D. speciosa* Germar, D. *u. tenella,* and D. *u. undecimpunctata* Mannerheim, leads to failure to successfully infest, feed, and/or develop, or leads to death of the coleopteran pest. The choice of target genes and the successful application of RNAi are then used to control coleopteran pests.

Phenotypic comparison of transgenic RNAi lines and nontransformed *Zea mays.* Target coleopteran pest genes or sequences selected for creating hairpin dsRNA have no similarity to any known plant gene sequence. Hence, it is not expected that the production or the activation of (systemic) RNAi by constructs targeting these coleopteran pest genes or sequences will have any deleterious effect on transgenic plants. However, development and morphological characteristics of transgenic lines are compared with non-transformed plants, as well as those of transgenic lines transformed with an "empty" vector having no hairpin-expressing gene. Plant root, shoot, foliage, and reproduction characteristics are compared. Plant shoot characteristics such as height, leaf numbers and sizes, time of flowering, floral size and appearance are recorded. In general, there are no observable morphological differences between transgenic lines and those without expression of target iRNA molecules when cultured *in vitro* and in soil in the glasshouse.

## EXAMPLE 10: Transgenic *Zea mays* Comprising a Coleopteran Pest Sequence and Additional RNAi Constructs

A transgenic *Zea mays* plant comprising a heterologous coding sequence in its genome that is transcribed into an iRNA molecule that targets an organism other than a coleopteran pest is secondarily transformed *via Agrobacterium* or WHISKERS™ methodologies *(see* Petolino and Arnold (2009) Methods Mol. Biol. 526:59-67) to produce one or more insecticidal dsRNA molecules (for example, at least one dsRNA molecule including a dsRNA molecule targeting a gene comprising SEQ ID NO:1 or SEQ ID NO:3). Plant transformation plasmid vectors prepared essentially as described in EXAMPLE 4 are delivered via *Agrobacterium* or WHISKERS™-mediated transformation methods into maize suspension cells or immature maize embryos obtained from a transgenic Hi II or B104 *Zea mays* plant comprising a heterologous coding sequence in its genome that is transcribed into an iRNA molecule that targets an organism other than a coleopteran pest.

## EXAMPLE 11: Transgenic *Zea mays* Comprising an RNAi Construct and Additional Coleopteran Pest Control Sequences

A transgenic *Zea mays* plant comprising a heterologous coding sequence in its genome that is transcribed into an iRNA molecule that targets a coleopteran pest organism (for example, at least one dsRNA molecule including a dsRNA molecule targeting a gene comprising SEQ ID NO:1 and/or SEQ ID NO:3) is secondarily transformed via *Agrobacterium* or WHISKERS™ methodologies (*see* Petolino and Arnold (2009) Methods Mol. Biol. 526:59-67) to produce one or more insecticidal protein molecules, for example, Cry3, Cry 6, Cry34 and Cry35 insecticidal proteins. Plant transformation plasmid vectors prepared essentially as described in EXAMPLE 4 are delivered via *Agrobacterium* or WHISKERS™-mediated transformation methods into maize suspension cells or immature maize embryos obtained from a transgenic B104 *Zea mays* plant comprising a heterologous coding sequence in its genome that is transcribed into an iRNA molecule that targets a coleopteran pest organism. Doubly-transformed plants are obtained that produce iRNA molecules and insecticidal proteins for control of coleopteran pests.

## Claims

1. An isolated nucleic acid comprising at least one polynucleotide operably linked to a heterologous promoter, wherein the polynucleotide is selected from the group consisting of:

    SEQ NO:1; the complement of SEQ NO:1; a fragment of at least 15 contiguous nucleotides of SEQ NO:1; the complement of a fragment of at least 15 contiguous nucleotides of SEQ NO:1; a native coding sequence of a *Diabrotica* organism comprising SEQ ID NO:1; the complement of a native coding sequence of a *Diabrotica* organism comprising SEQ ID NO:1; a fragment of at least 15 contiguous nucleotides of a native coding sequence of a *Diabrotica* organism comprising SEQ ID NO:1; the complement of a fragment of at least 15 contiguous nucleotides of a native coding sequence of a *Diabrotica* organism comprising SEQ ID NO:1;
    SEQ NO:3; the complement of SEQ NO:3; a fragment of at least 15 contiguous nucleotides of SEQ ID NO:3; the complement of a fragment of at least 15 contiguous nucleotides of SEQ NO:3; a native coding sequence of a *Diabrotica* organism comprising SEQ NO:3; the complement of a native coding sequence of a *Diabrotica* organism comprising SEQ NO:3; a fragment of at least 15 contiguous nucleotides of a native coding sequence of a *Diabrotica* organism comprising SEQ ID NO:3; and the complement of a fragment of at least 15 contiguous nucleotides of a native coding sequence of a *Diabrotica* organism comprising SEQ NO:3.

2. The nucleic acid of claim 1, wherein the polynucleotide is selected from the group consisting of SEQ ID NO:1; the complement of SEQ ID NO:1; SEQ ID NO:3; the complement of SEQ NO:3; a fragment of at least 15 contiguous nucleotides of SEQ NO:1; the complement of a fragment of at least 15 contiguous nucleotides of SEQ NO:1; a fragment of at least 15 contiguous nucleotides of SEQ NO:3; the complement of a fragment of at least 15 contiguous nucleotides of SEQ ID NO:3; a native coding sequence of a *Diabrotica* organism comprising any of SEQ NOs:5-8; the complement of a native coding sequence of a *Diabrotica* organism comprising any of SEQ NOs:5-8; a fragment of at least 15 contiguous nucleotides of a native coding sequence of a *Diabrotica* organism comprising any of SEQ NOs:5-8; and the complement of a fragment of at least 15 contiguous nucleotides of a native coding sequence of a *Diabrotica* organism comprising any of SEQ NOs:5-8.

3. The polynucleotide of claim 1 or 2, wherein the organism is selected from the group consisting of *D. v. virgifera* LeConte; *D. barberi* Smith and Lawrence; *D. u. howardi; D. v. zeae*; *D. balteata* LeConte; *D. u. tenella*; *D. speciosa* Germar; and *D. u. undecimpunctata* Mannerheim.

4. A plant transformation vector comprising the polynucleotide of any of claims 1 to 3.

5. A ribonucleic acid (RNA) molecule transcribed from the polynucleotide of any of claims 1 to 3.

6. A double-stranded ribonucleic acid (dsRNA) molecule produced from the expression of the polynucleotide of any of claims 1 to 3, optionally

    (i) wherein contacting the polynucleotide sequence with a coleopteran insect inhibits the expression of an endogenous nucleotide sequence specifically complementary to the polynucleotide, wherein preferably contacting said ribonucleotide molecule with a coleopteran insect kills or inhibits the growth, viability, and/or feeding of the insect, and/or

(ii) comprising a first, a second and a third RNA segment, wherein the first RNA segment comprises the polynucleotide, wherein the third RNA segment is linked to the first RNA segment by the second polynucleotide sequence, and wherein the third RNA segment is substantially the reverse complement of the first RNA segment, such that the first and the third RNA segments hybridize when transcribed into a ribonucleic acid to form the double-stranded RNA.

**7.** The RNA molecule of claim 6, selected from the group consisting of a double-stranded ribonucleic acid molecule and a single-stranded ribonucleic acid molecule of between about 15 and about 30 nucleotides in length.

**8.** The nucleic acid of any of claims 1 to 3, wherein the nucleic acid is a plant transformation vector, and wherein the heterologous promoter is functional in a plant cell.

**9.** A cell transformed with the nucleic acid of any of claims 1 to 3, wherein the cell is preferably (i) a prokaryotic cell or (ii) a eukaryotic cell, preferably a plant cell, preferably a *Zea mays* cell.

**10.** A plant transformed with the nucleic acid of any of claims 1 to 3, wherein preferably

(i) the at least one polynucleotide is expressed in the plant as a double-stranded ribonucleic acid molecule, and/or
(ii) the plant is *Zea mays,* and/or
(iii) at least one polynucleotide is expressed in the plant as a ribonucleic acid (RNA) molecule, and the RNA molecule inhibits the expression of an endogenous polynucleotide that is specifically complementary to the at least one polynucleotide when a coleopteran insect ingests a part of the plant.

**11.** A seed of the plant of claim 10, wherein the seed comprises the polynucleotide.

**12.** A commodity product produced from the plant of claim 10, wherein the commodity product comprises a detectable amount of the polynucleotide.

**13.** The nucleic acid of any of claims 1 to 3, further comprising at least one additional polynucleotide that encodes an RNA molecule that inhibits the expression of an endogenous pest gene, wherein preferably

(i) the nucleic acid is a plant transformation vector, and/or
(ii) the additional polynucleotide(s) are each operably linked to a heterologous promoter functional in a plant cell.

**14.** A method for controlling a coleopteran pest population, the method comprising:

A. providing an agent comprising a ribonucleic acid (RNA) molecule that functions upon contact with the pest to inhibit a biological function within the pest, wherein the RNA is specifically hybridizable with a polynucleotide selected from the group consisting of any of SEQ NOs:75-80; the complement of any of SEQ NOs:75-80; a fragment of at least 15 contiguous nucleotides of any of SEQ NOs:75-80; the complement of a fragment of at least 15 contiguous nucleotides of any of SEQ NOs:75-80; a transcript of any of SEQ NOs:1, 3, 5-8, and 81; the complement of a transcript of any of SEQ NOs:1, 3, 5-8, and 81; a fragment of at least 15 contiguous nucleotides of a transcript of either of SEQ ID NOs:1 and 3; the complement of a fragment of at least 15 contiguous nucleotides of a transcript of either of SEQ ID NOs:1 and 3, wherein optionally:

(i) the RNA of the agent is specifically hybridizable with a polynucleotide selected from the group consisting of any of SEQ ID NOs:75-81; the complement of any of SEQ ID NOs:75-81; a fragment of at least 15 contiguous nucleotides of any of SEQ ID NOs:75-81; and the complement of a fragment of at least 15 contiguous nucleotides of any of SEQ ID NOs:75-81, and/or
(ii) the agent is a double-stranded RNA molecule; and/or

B. providing an agent comprising a first and a second polynucleotide sequence that functions upon contact with the coleopteran pest to inhibit a biological function within the coleopteran pest, wherein the first polynucleotide sequence comprises a region that exhibits from about 90% to about 100% sequence identity to from about 15 to about 30 contiguous nucleotides of SEQ ID NO:75 and/or SEQ ID NO:76, and wherein the first polynucleotide sequence is specifically hybridized to the second polynucleotide sequence, wherein optionally the agent is a ribonucleic acid molecule of SEQ ID NO:84; and/or
C. providing in a host plant of a coleopteran pest a transformed plant cell comprising the nucleic acid of claim 2 or 3, wherein the polynucleotide is expressed to produce a ribonucleic acid (RNA) molecule that functions upon contact with a coleopteran pest belonging to the population to inhibit the expression of a target sequence within the coleopteran pest and results in decreased growth and/or survival of the coleopteran pest or pest population, relative to reproduction of the same pest species on a plant of the same host plant species that does not comprise the polynucleotide, wherein optionally the RNA molecule is a double-stranded ribonucleic acid molecule and, wherein preferably:

(i) the nucleic acid comprises SEQ ID NO:81 and/or
(ii) the coleopteran pest population is reduced relative to a population of the same pest species infesting a host plant of the same host plant species lacking the transformed plant cell.

**15.** A method of controlling coleopteran pest infestation in a plant, the method comprising providing in the diet of a coleopteran pest a ribonucleic acid (RNA) that is specifically hybridizable with a polynucleotide selected from the group consisting of:

SEQ ID NOs:75-80;
the complement of any of SEQ NOs:75-80;
a fragment of at least 15 contiguous nucleotides of any of SEQ NOs:75-80;
the complement of a fragment of at least 15 contiguous nucleotides of any of SEQ NOs:75-80;
a transcript of any of SEQ NOs:1, 3, and 5-8;
the complement of a transcript of any of SEQ NOs:1, 3, and 5-8;
a fragment of at least 15 contiguous nucleotides of a transcript of any of SEQ NOs:1, 3, and 5-8; and
the complement of a fragment of at least 15 contiguous nucleotides of a transcript of any of SEQ ID NOs:1,3, and 5-8,
and wherein optionally:

(i) the diet comprises a plant cell transformed to express the polynucleotide, and/or
(ii) the specifically hybridizable RNA is comprised in a double-stranded RNA molecule.

**16.** A method for improving the yield of a corn crop, the method comprising:

introducing the nucleic acid of claims 1 to 3into a corn plant to produce a transgenic corn plant; and
cultivating the corn plant to allow the expression of the at least one polynucleotide; wherein expression of the at least one polynucleotide inhibits coleopteran pest viability or growth and loss of yield due to coleopteran pest infection, wherein preferably expression of the at least one polynucleotide produces a ribonucleic acid (RNA) molecule that suppresses at least a first target gene in a coleopteran pest that has contacted a portion of the corn plant.

**17.** A method for producing a transgenic plant cell, the method comprising:

A. transforming a plant cell with the plant transformation vector of claim 8;

culturing the transformed plant cell under conditions sufficient to allow for development of a plant cell culture comprising a plurality of transformed plant cells;
selecting for transformed plant cells that have integrated the at least one polynucleotide into their genomes;
screening the transformed plant cells for expression of a ribonucleic acid (RNA) molecule encoded by the at least one polynucleotide; and
selecting a plant cell that expresses the RNA, wherein opitionally (i) the vector comprises a polynucleotide selected from the group consisting of SEQ NO:5; the complement of SEQ ID NO:5; SEQ ID NO:6; the complement of SEQ ID NO:6; SEQ ID NO:7; the complement of SEQ ID NO:7; SEQ ID NO:8; the complement of SEQ ID NO:8; a fragment of at least 15 contiguous nucleotides of any of SEQ NOs:5-8; the complement of a fragment of at least 15 contiguous nucleotides of any of SEQ NOs:5-8; a native coding sequence of a *Diabrotica* organism comprising any of SEQ NOs:5-8; the complement of a native coding sequence of a *Diabrotica* organism comprising any of SEQ NOs:5-8; a fragment of at least 15 contiguous nucleotides of a native coding sequence of a *Diabrotica* organism comprising any of SEQ NOs:5-8; and the complement of a fragment of at least 15 contiguous nucleotides of a native coding sequence of a *Diabrotica* organism comprising any of SEQ NOs:5-8, and/or (ii) the RNA molecule is a double-stranded RNA molecule.

B. transforming a plant cell with a vector comprising a means for providing coleopteran pest protection to a plant;

culturing the transformed plant cell under conditions sufficient to allow for development of a plant cell culture comprising a plurality of transformed plant cells;
selecting for transformed plant cells that have integrated the means for providing coleopteran pest protection to a plant into their genomes;
screening the transformed plant cells for expression of a means for inhibiting expression of an essential gene in a coleopteran pest; and
selecting a plant cell that expresses the means for inhibiting expression of an essential gene in a coleopteran pest.

**18.** A method for producing a transgenic plant protected against a coleopteran pest, the method comprising:

A. providing the transgenic plant cell produced by the method of claim 17, alternative A; and regenerating a transgenic plant from the transgenic plant cell, wherein expression of the RNA molecule encoded by the at least one polynucleotide is sufficient to modulate the expression of a target gene in a coleopteran pest that contacts the transformed plant and/or
B. regenerating a transgenic plant from the transgenic plant cell produced by the method of claim 17, alternative B, wherein expression of the means for inhibiting expression of an essential gene in a coleopteran pest is sufficient to modulate the expression of a target gene in a coleopteran pest that contacts the transformed plant.

**19.** The nucleic acid of any of claims 1 to 3, further comprising a polynucleotide encoding a polypeptide from *Bacillus thuringiensis, Alcaligenes* spp., or *Pseudomonas* spp, wherein optionally the polypeptide from *B. thuringiensis* is selected from a group comprising Cry1B, Cry1I, Cry2A, Cry3, Cry6, Cry7A, Cry8, Cry9D, Cry14, Cry18, Cry22, Cry23, Cry34, Cry35, Cry36, Cry37, Cry43, Cry55, Cyt1A, and/or Cyt2C.

**20.** The cell of claim 9, alternative (ii), wherein the cell comprises a polynucleotide encoding a polypeptide from *Bacillus thuringiensis, Alcaligenes* spp., or *Pseudomonas* spp, wherein optionally the polypeptide from *B. thuringiensis* is selected from a group comprising Cry1B, Cry1I, Cry2A, Cry3, Cry6, Cry7A, Cry8, Cry9D, Cry14, Cry18, Cry22, Cry23, Cry34, Cry35, Cry36, Cry37, Cry43, Cry55, Cyt1A, and/or Cyt2C.

**21.** The plant of claim 10, wherein the plant comprises a polynucleotide encoding a polypeptide from *Bacillus thuringiensis, Alcaligenes* spp., or *Pseudomonas* spp, wherein optionally the polypeptide from *B. thuringiensis* is selected from a group comprising Cry1B, Cry1I, Cry2A, Cry3, Cry6, Cry7A, Cry8, Cry9D, Cry14, Cry18, Cry22, Cry23, Cry34, Cry35, Cry36, Cry37, Cry43, Cry55, Crt1A, and/or Cyt2C.

**22.** The method according to claim 17, alternative A, wherein the transformed plant cell comprises a nucleotide sequence encoding a polypeptide from *Bacillus thuringiensis, Alcaligenes* spp., or *Pseudomonas* spp, wherein optionally the polypeptide from *B. thuringiensis* is selected from a group comprising Cry1B, Cry1I, Cry2A, Cry3, Cry6, Cry7A, Cry8, Cry9D, Cry14, Cry18, Cr22, Cry23, Cry34, Cry35, Cry36, Cry37, Cry43, Cry55, Cyt1A, and/or Cyt2C.

**FIG. 1.** Generation of dsRNA from a single transcription template with a single pair of primers

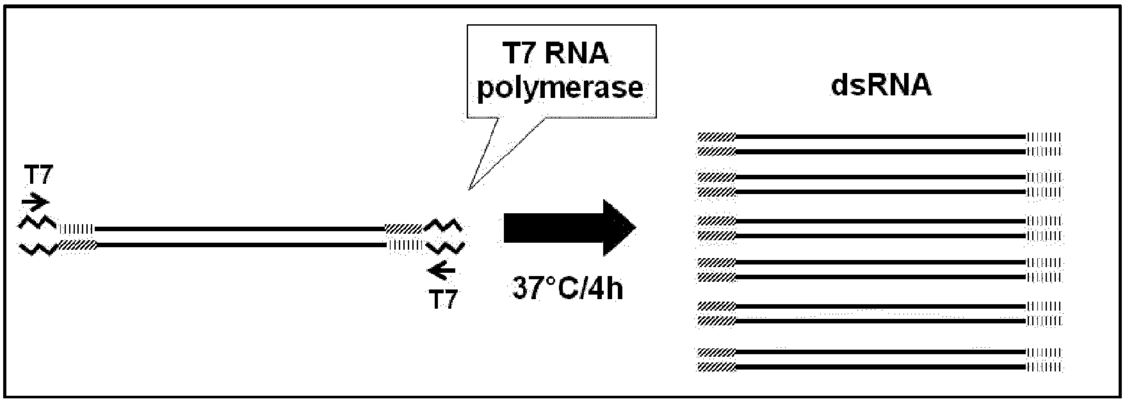


**FIG. 2.** Generation of dsRNA from two transcription templates.

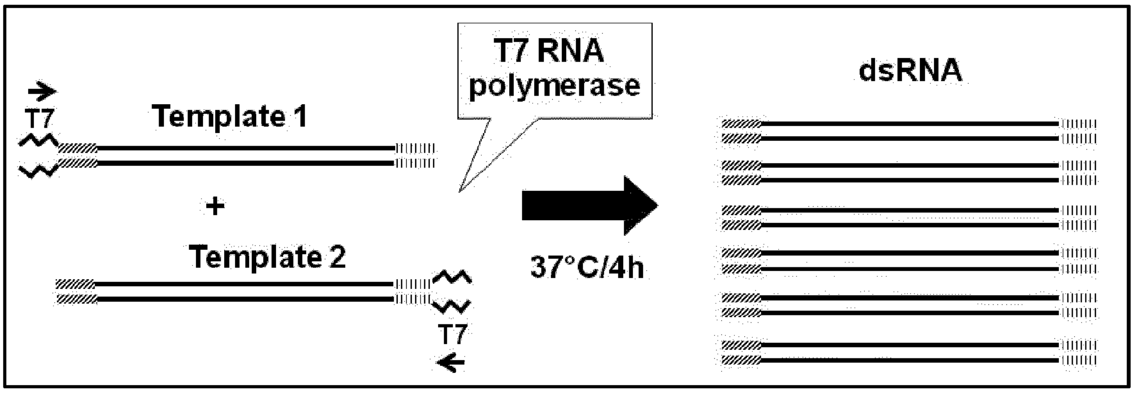

Europäisches Patentamt
European Patent Office
Office européen des brevets

# EUROPEAN SEARCH REPORT

Application Number
EP 16 16 0084

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2011/154545 A1 (ANDERSEN SCOTT E [US] ET AL) 23 June 2011 (2011-06-23)<br>* sequence 2747 *<br>* paragraphs [0041] - [0043], [0151] *<br>----- | 1,2,4,5, 8-12 | INV.<br>C12N15/82<br>C12N15/113<br>C12N9/12 |
| Y | WO 2012/092580 A2 (DOW AGROSCIENCES LLC [US]; NARVA KENNETH E [US]; LI HUARONG [US]; GENG) 5 July 2012 (2012-07-05)<br>* the whole document *<br>----- | 1-22 | |
| Y | US 2014/298536 A1 (NARVA KENNETH [US] ET AL) 2 October 2014 (2014-10-02)<br>* the whole document *<br>----- | 1-22 | |
| Y | BAUM JAMES A ET AL: "Control of coleopteran insect pests through RNA interference",<br>NATURE BIOTECHNOLOGY, NATURE PUBLISHING GROUP, US,<br>vol. 25, no. 11,<br>1 November 2007 (2007-11-01), pages 1322-1326, XP002532086,<br>ISSN: 1087-0156, DOI: 10.1038/NBT1359<br>* the whole document *<br>----- | 1-22 | TECHNICAL FIELDS SEARCHED (IPC)<br>C12N |
| Y | YAEL GARBIAN ET AL: "Bidirectional Transfer of RNAi between Honey Bee and Varroa destructor: Varroa Gene Silencing Reduces Varroa Population",<br>PLOS PATHOGENS,<br>vol. 8, no. 12,<br>20 December 2012 (2012-12-20), page e1003035, XP055069058,<br>ISSN: 1553-7366, DOI: 10.1371/journal.ppat.1003035<br>* table 1 *<br>-----<br>-/-- | 1-22 | |

The present search report has been drawn up for all claims

1

| Place of search | Date of completion of the search | Examiner |
|---|---|---|
| Munich | 21 June 2016 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 16 0084

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/192256 A1 (SYNGENTA PARTICIPATIONS AG [CH]; DONOHUE KEVIN [US]; LIU RENSHUI [US];) 27 December 2013 (2013-12-27) * abstract * ----- | 1-22 | |
| A | KAI-YUN FU ET AL: "Response of the vacuolar ATPase subunit E to RNA interference and four chemical pesticides in Leptinotarsa decemlineata (Say)", PESTICIDE BIOCHEMISTRY AND PHYSIOLOGY, vol. 114, 1 September 2014 (2014-09-01), pages 16-23, XP055190424, ISSN: 0048-3575, DOI: 10.1016/j.pestbp.2014.07.009 * abstract * ----- | 1-22 | |
| A | RENATA BOLOGNESI ET AL: "Characterizing the Mechanism of Action of Double-Stranded RNA Activity against Western Corn Rootworm (Diabrotica virgifera virgifera LeConte) (e47534)", PLOS ONE, vol. 7, no. 10, 11 October 2012 (2012-10-11), pages 1-11, XP055268106, DOI: 10.1371/journal.pone.0047534 * abstract * ----- | 1-22 | TECHNICAL FIELDS SEARCHED (IPC) |
| A | LEVINE S.L. ET AL: "Independent Action between Dv5nf7 RNA and Cry3Bb1 Protein in Southern Corn Rootworm, Diabrotica undecimpunctata howardi and Colorado Potato Beetle, Leptinotarsa decemlineata", PLOS ONE, vol. 10, no. 3, 1 March 2015 (2015-03-01), pages 1-15, XP055268108, * abstract * ----- | 1-22 | |

1

The present search report has been drawn up for all claims

| Place of search | Date of completion of the search | Examiner |
|---|---|---|
| Munich | 21 June 2016 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 16 0084

This annex lists the patent family members relating to the patent documents cited in the above-mentioned European search report. The members are as contained in the European Patent Office EDP file on
The European Patent Office is in no way liable for these particulars which are merely given for the purpose of information.

21-06-2016

| Patent document cited in search report | Publication date | Patent family member(s) | Publication date |
|---|---|---|---|
| US 2011154545 A1 | 23-06-2011 | US 2007050860 A1 | 01-03-2007 |
| | | US 2010192265 A1 | 29-07-2010 |
| | | US 2011154545 A1 | 23-06-2011 |
| | | US 2014194306 A1 | 10-07-2014 |
| WO 2012092580 A2 | 05-07-2012 | AR 084776 A1 | 26-06-2013 |
| | | EP 2658975 A2 | 06-11-2013 |
| | | US 2012198586 A1 | 02-08-2012 |
| | | UY 33853 A | 31-07-2012 |
| | | WO 2012092580 A2 | 05-07-2012 |
| US 2014298536 A1 | 02-10-2014 | BR 102013032916 A2 | 29-12-2015 |
| | | US 2014298536 A1 | 02-10-2014 |
| WO 2013192256 A1 | 27-12-2013 | AR 091512 A1 | 11-02-2015 |
| | | CA 2890235 A1 | 27-12-2013 |
| | | CL 2014003486 A1 | 22-05-2015 |
| | | CN 105143453 A | 09-12-2015 |
| | | EP 2864484 A1 | 29-04-2015 |
| | | US 2015337302 A1 | 26-11-2015 |
| | | WO 2013192256 A1 | 27-12-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62133214 B
- US 7612194 B
- US 20070050860 A
- US 20100192265 A
- US 20110154545 A
- US 7943819 B
- US 2013040173 A
- WO 2013169923 A
- US 577811 A
- US 577854 A
- US 62133202 A
- US 62095487 A
- US 705807 A
- US 62063199 A
- US 62063203 A
- US 62063192 A
- US 62063216 A
- WO 9630530 A
- US 4980460 A
- US 4725677 A
- US 4415732 A
- US 4458066 A
- US 4973679 A
- WO 9732016 A
- US 5593874 A
- US 5698425 A
- US 5712135 A
- US 5789214 A
- US 5804693 A
- WO 06073727 A
- US 20060200878 A1
- US 20020048814 A
- US 20030018993 A
- WO 9401550 A
- WO 9805770 A
- US 6437217 B
- US 5641876 A
- US 6426446 B
- US 6429362 B
- US 6232526 B
- US 6177611 B
- US 5322938 A
- US 5352605 A
- US 5359142 A
- US 5530196 A
- US 6433252 B
- US 6429357 B
- US 6294714 B
- US 6140078 A
- US 6252138 B
- US 6175060 B
- US 6388170 B
- US 6635806 B
- US 2009757089 A
- US 6051753 A
- US 5378619 A
- US 5850019 A
- US 6677503 B
- US 5110732 A
- US 5459252 A
- US 5837848 A
- US 5362865 A
- US 5659122 A
- US 5550318 A
- US 5633435 A
- US 5780708 A
- US 6118047 A
- US 5508184 A
- US 5384253 A
- US 5302523 A
- US 5464765 A
- US 5563055 A
- US 5591616 A
- US 5693512 A
- US 5824877 A
- US 5981840 A
- US 6384301 B
- US 5015580 A
- US 5538880 A
- US 6160208 A
- US 6399861 B
- US 6403865 B
- US 7060876 B
- WO 9506722 A
- US 4536475 A
- US 4693977 A
- US 4886937 A
- US 5501967 A
- EP 0122791 A
- EP 0120516 A
- US 20120174258 A
- US 20120174259 A
- US 20120174260 A
- US 20120198586 A
- US 20130091600 A
- US 20130091601 A
- US 20130097730 A

- US 62133210 A
- US 8530440 B
- US 5107065 A
- US 5759829 A
- US 5283184 A
- US 5231020 A
- US 5510474 A
- US 6699984 B
- US 7838733 B2
- US 7179902 B
- US 20070124836 A
- US 8304604 B
- WO 2012016222 A2

**Non-patent literature cited in the description**


- **ELLSBURY et al.** *Environ. Entomol.,* 2005, vol. 34, 627-34
- **FIRE et al.** *Nature,* 1998, vol. 391, 806-11
- **MARTINEZ et al.** *Cell,* 2002, vol. 110, 563-74
- **MCMANUS ; SHARP.** *Nature Rev. Genetics,* 2002, vol. 3, 737-47
- **LEE et al.** *Cell,* 2004, vol. 117 (1), 69-81
- **BOLOGNESI et al.** *PLoS ONE,* 2012, vol. 7 (10), 47534
- **BAUM et al.** *Nature Biotechnology,* 2007, vol. 25, 1322-1326
- **BAUM et al.** *Nat. Biotechnol.,* 2007, vol. 25 (11), 1322-6
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. U.S.A.,* 1988, vol. 85, 2444
- **HIGGINS ; SHARP.** *Gene,* 1988, vol. 73, 237-44
- **HIGGINS ; SHARP.** *CABIOS,* 1989, vol. 5, 151-3
- **CORPET et al.** *Nucleic Acids Res.,* 1988, vol. 16, 10881-90
- **HUANG et al.** *Comp. Appl. Biosci.,* 1992, vol. 8, 155-65
- **PEARSON et al.** *Methods Mol. Biol.,* 1994, vol. 24, 307-31
- **TATIANA et al.** *FEMS Microbiol. Lett.,* 1999, vol. 174, 247-50
- **ALTSCHUL et al.** *J. Mol. Biol.,* 1990, vol. 215, 403-10
- Molecular Cloning: A Laboratory Manual. Cold Spring Harbor Laboratory Press, 1989, vol. 1-3
- Nucleic Acid Hybridization. IRL Press, 1985
- Overview of principles of hybridization and the strategy of nucleic acid probe assays. **TIJSSEN.** Laboratory Techniques in Biochemistry and Molecular Biology- Hybridization with Nucleic Acid Probes. Elsevier, 1993
- Current Protocols in Molecular Biology. Greene Publishing and Wiley-Interscience, 1995
- **WARD et al.** *Plant Mol. Biol.,* 1993, vol. 22, 361-366
- **SCHENA et al.** *Proc. Natl. Acad. Sci. USA,* 1991, vol. 88, 0421
- **FROMM et al.** *Nature,* 1986, vol. 319, 791-3
- **FELGNER et al.** *Proc. Natl. Acad. Sci. USA,* 1987, vol. 84, 7413-7
- **MUELLER et al.** *Cell,* 1978, vol. 15, 579-85
- **FRALEY et al.** *Proc. Natl. Acad. Sci. USA,* 1983, vol. 80, 4803-7
- **KLEIN et al.** *Nature,* 1987, vol. 327, 70
- Lewin's Genes X. Jones & Bartlett Publishers, 2009
- The Encyclopedia of Molecular Biology. Blackwell Science Ltd, 1994
- Molecular Biology and Biotechnology: A Comprehensive Desk Reference. VCH Publishers, Inc, 1995
- **ELBASHIR et al.** *Nature,* 2001, vol. 411, 494-8
- **HAMILTON ; BAULCOMBE.** *Science,* 1999, vol. 286 (5441), 950-2
- **OZAKI et al.** *Nucleic Acids Research,* 1992, vol. 20, 5205-5214
- **AGRAWAL et al.** *Nucleic Acids Research,* 1990, vol. 18, 5419-5423
- **BEAUCAGE et al.** *Tetrahedron,* 1992, vol. 48, 2223-2311
- **EBERT et al.** *Proc. Natl. Acad. Sci. USA,* 1987, vol. 84 (16), 5745-9
- **LAWTON et al.** *Plant Mol. Biol.,* 1987, vol. 9, 315-24
- **ODELL et al.** *Nature,* 1985, vol. 313, 810-2
- **WALKER et al.** *Proc. Natl. Acad. Sci. USA,* 1987, vol. 84 (19), 6624-8
- **YANG ; RUSSEL.** *Proc. Natl. Acad. Sci. USA,* 1990, vol. 87, 4144-8
- **CHANDLER et al.** *Plant Cell,* 1989, vol. 1, 1175-83
- **DEPICKER et al.** *J. Mol. Appl. Genet.,* 1982, vol. 1, 561-73
- **BEVAN et al.** *Nature,* 1983, vol. 304, 184-7
- **OPPERMAN et al.** *Science,* 1994, vol. 263, 221-3
- **HIREL et al.** *Plant Mol. Biol.,* 1992, vol. 20, 207-18
- **TURNER ; FOSTER.** *Molecular Biotech.,* 1995, vol. 3 (3), 225-36
- **CARRINGTON ; FREED.** *J. Virol.,* 1990, vol. 64, 1590-7

- **INGELBRECHT et al.** *Plant Cell,* 1989, vol. 1, 671-80
- **CORUZZI et al.** *EMBO J.,* 1984, vol. 3, 1671-9
- **JEFFERSON et al.** *Plant Mol. Biol. Rep.,* 1987, vol. 5, 387-405
- Molecular cloning of the maize R-nj allele by transposon tagging with Ac. **DELLAPORTA et al.** In 18th Stadler Genetics Symposium. Plenum, 1988, 263-82
- **SUTCLIFFE et al.** *Proc. Natl. Acad. Sci. USA,* 1978, vol. 75, 3737-41
- **OW et al.** *Science,* 1986, vol. 234, 856-9
- **ZUKOWSKI et al.** *Gene,* 1983, vol. 46 (2-3), 247-55
- **IKATU et al.** *Bio/Technol.,* 1990, vol. 8, 241-2
- **KATZ et al.** *J. Gen. Microbiol.,* 1983, vol. 129, 2703-14
- **POTRYKUS et al.** *Mol. Gen. Genet.,* 1985, vol. 199, 183-8
- **HERRERA-ESTRELLA et al.** *Nature,* 1983, vol. 303, 209-13
- **KLEE et al.** *Bio/Technol.,* 1985, vol. 3, 637-42
- **RIOS, G. et al.** *Plant J.,* 2002, vol. 32, 243-53
- **KNACKMUSS et al.** *Mol. Gen. Genet.,* 1997, vol. 253 (5), 529-34
- **SEITHER et al.** *Mol. Gen. Genet.,* 1997, vol. 255 (2), 180-6
- **SHAGIN et al.** *Mol. Biol. Evol.,* 2004, vol. 21 (5), 841-50
- **VANCANNEYT et al.** *Mol. Gen. Genet.,* 1990, vol. 220 (2), 245-50
- **WRIGHT et al.** *Proc. Natl. Acad. Sci. U.S.A.,* 2010, vol. 107, 20240-5
- **SCHENK et al.** *Plant Mol. Biol.,* 1999, vol. 39, 1221-30
- **WATSON et al.** *J. Bacteriol.,* 1975, vol. 123, 255-264
- Genetic Transformation Using Maize Immature Zygotic Embryos. **FRAME et al.** IN Plant Embryo Culture Methods and Protocols: Methods in Molecular Biology. Springer Science and Business Media, 2011, 327-341
- **HALLAUER et al.** *Crop Science,* 1997, vol. 37, 1405-1406
- **BAUM et al.** *Nat. Biotechnol.,* 2007, vol. 25 (11), 1322-1326
- **OLESON et al.** *J. Econ. Entomol.,* 2005, vol. 98, 1-8
- **PETOLINO ; ARNOLD.** *Methods Mol. Biol.,* 2009, vol. 526, 59-67

SEQUENCE LISTING

<110> Dow AgroSciences LLC
Fraunhofer-Gesellschaft zur Förderung der angewandten Forschung EV

<120> RNA POLYMERASE I1 NUCLEIC ACID MOLECULES TO CONTROL INSECT PESTS

<130> 971-2 EP

<150> US 62/133,214
<151> 2015-03-13

<160> 84

<170> PatentIn version 3.5

<210> 1
<211> 5053
<212> DNA
<213> Diabrotica virgifera

<400> 1
ttcaaatgaa gagaagaaga agaagaatct tcaaagcatg tttttgttag gttaaatttc 60

aaatttttta atttgatgtt tgttattgtg tttaaaaaca agattattaa ataaacggaa 120

acatgaagat tcactttata cccgataaag tgagcttttc actttttacc acagaagaaa 180

tcaagaaaat gtgtgttact caaattatta ccccacttat gttagatccc ttaggtcatc 240

ctctacaagg aggtttatat gatcgtaaat taggaccata ctcagccaaa gatactctat 300

gtgaatcttg taaccaaaca ttcaccaatt gtcctggcca ttttggatac attgaacttc 360

ctttaccagt agtaaatcct ctgtttcata aaattattgg aaccattata aaaatatcat 420

gtttgtcatg tttccacata caactaccaa ctcatataaa aaaagtttta tgtattcaaa 480

tgaaactttt aaattgtgga ctgattacag aagccttatc agtagaaaca gcattagcgg 540

aattaatttc gaaatatgaa aagtttgaga atattccaac tgaaagtata aaaagtgtat 600

tacgatatga ggaattagct gatgaaacat taaagaatct agaaggtaaa aatgtaacca 660

gccaaaacac cgaaacactc cgcaacaact ttgtgtctaa aatgttgaaa gaggtaaaat 720

ctagacaact atgtatattt tgtaaaaaca gaattaatag gattcaggca ctaaagaaca 780

gaattatttt aacaaaaaag aaaggtgatc tagatgattc taacgtggtt atgggacaaa 840

aagtgacagg tatggagtct caatacatta cgccggaaga gtcaaggcaa tatttaagaa 900

atatatggag acaagaaaaa gaatttcttc aacaacttgt atctgtactc gcaaatgtcg 960

actgtgaaca cccaactgat gcattttatt ttgaagtgat tcctgttcca ccaccaaatg 1020

ttagacctgt taacttcgtg aatggtagaa tattagagaa taaacaatcg attggttaca 1080

aaaatatcat acaaaatgtc attcttttaa aaactataat acaagtagta caaagtaaga 1140

gcgatattaa tagcttgtca tctgtggaag ctaagagtgc ctatagcatt gccagaggaa 1200

attctcctgt agaaaaatta aattattgct gggaagaatt gcagagtgac gtaaacgggt 1260

tacttgataa tgaaaatgtt cgacaagagg gacagggatt gaaacagatc attgaaaaga 1320

aagaaggtgt tatacgtatg tgtatgatgg gtaaacgtgt taatttttcc gctagatcag 1380

ttataactcc tgaccccaac cttaatatcg acgaaatcgg aatcccggaa gaatttgcaa 1440

agaagctgac ttatccagta gctgtaactc cttggaatgt tgaagaacta aggaaaatga 1500

ttttaaatgg acctaatgtt cacccagggg caattatgat agaaaacaat gggattctaa 1560

aacgaattaa tccctataat gaagttcaac aaaaaagcat attgaaatgt ttgttaactc 1620

ctgaagccac taaaggacca aaaggacaag gaattcaaat cgtgcatagg catttatgca 1680

acggtgacgt tctgttactt aatcgccaac caactttgca caaacctagt ataatggcgc 1740

acacagcgcg aattttaaag ggagaaaaaa ctcttcgatt gcattatgct aattgtaaag 1800

cctataatgc tgattttgac ggggatgaaa tgaacgcaca ttacccccaa aatgaacttg 1860

caagaagtga aggctataat attgttaacg tttctaacca atatctcgtt ccaaaagatg 1920

gcactcctct cagtggttta attcaggacc acatgatttc tggtgtgcga ctgtctttaa 1980

gaggaagatt ttttgataaa caagattatg agcatctagt ttaccaagct ctctcgttca 2040

aaacaggtcg cataaagctc ttaccaccaa caattataaa accacaagtg ttatggtctg 2100

ggaaacaaat tttatctaca gttataatta acgtcatacc cgacgaaaga gaattaatca 2160

atcttacgtc aacagctaag atttcttcca aagcttggca gagaagaccc tcgagaagat 2220

ggagagcagg tggtactata tttcttgatg acaaggtcat gagtgaagct gaggtcataa 2280

ttagaggtgg tgaacttctt gttggggttc tagataaaac tcactacggt tctactcctt 2340

atggtttagt acactgtatt tatgagttat atgggggtac ctatgcaatc agattacttt 2400

cctcgttgac aaaacttttc atgagatttt tgcaacaaga agggtttaca cttggagtac 2460

atgatatact tacagtagaa agagctgatg ttaggagaag ggaaattata aaagactgta 2520

gacaagtagg aaaagaagcc gtaactaaag ctttagatgt acctttagac actcctgatg 2580

ctgaagttgt tgaaacaata gaaaaactaa gtgctgctga tcccaaaatt agagctacaa 2640

tcgacaggtc ctacaagtct tcgatggata tttttaccaa tgaaattaat agaacttgtt 2700

tgcctgctgg tctggtttgt aaatttcctg aaaataatct tcaattgatg gtacaatctg 2760

gagcgaaagg ttcaacagta aatactatgc aaatttcctg tcttcttggt caaatagaat 2820

tggaaggaaa acggccacct gtaatgatat ccggaaaatc tctacctagt tttccatcat 2880

tcgagtttac tccaagggcg ggaggattta tcgatggacg attcatgact ggtatccaac 2940

cgcaagaatt cttcttccat tgtatggcag gacgtgaagg tcttattgat acagctgtta 3000

aaactagtcg tagtggatat ttacaaagat gtctcatcaa acatttggaa ggtctacgtg 3060

ttggttatga tatgaccgtg agaaacagtg ataaaagtgt aatacagttt ttgtatggag 3120

aggatggaat ggatatttca aaagctcagt ttttcaatga aaaacagatg agcttcttgg 3180
ccgaaaatat caaggtgttg ggtaattctg atacgcttaa acagttgaag aatgaagaag 3240
atcaagaggc tgtaaaagaa catggaaaag cggtaaaaga atggaagaaa catcatggga 3300
atccattaaa tcacagaagg aatagtccat tttccctgtt tgctaaatat gttcaaaata 3360
ggactggaga taacaacctt ttaaccaagg agaagttaat gaaactttgg tatgaaatgg 3420
acaaggacat aaaaacaaac ttcaccgacc agtgcgagaa atgcccagat cccatagaag 3480
ccgtgtatca acctgatgca aattttggag cgataaatga aacagtgcag aaacttatca 3540
agaactataa aggattcgac aataagaaaa gtaagaaaaa atttgaagat gtcataaaat 3600
tgaaagtaat ggaatcgatg tgttctgctg gagaaccagt tggacttctt gcggcacaat 3660
caataggaga accatctacc cagatgacac tcaatacttt ccattttgct ggaagaggtg 3720
aaatgaacgt tactctcggt attccccgtt taagagaaat cttaatgatg gcttcaaaga 3780
atataaaaac gccatcgatg gagattccat tcttgcaggt tccagattta gaatataagg 3840
cgaacgagtt aaggaaactt ctgactcgag tggtggtagc cgacgtttta gaaactattg 3900
atgttactgt tgaacttcaa tttaaaccca ttaggcaata taagtatacc ttgaaattcc 3960
aatttttacc gaagaaatat tacagtatgg attattgtgt gaatcccaca aaaatactaa 4020
ggcatatgaa ggggaaatat tttggtgaaa tgtttgcgtc catcaagaaa gtcagtaaaa 4080
ttaattcaaa catagtaatg atggaggaag aaagaaacaa gaaacgtaca actaataacg 4140
aagaagatga agatcgacca gaaacaaatg aaagagaagg tgacaatcaa attgattcct 4200
cagatgacga agtggaagat aatgaagatg ctaagcagag tcataagtac caagaaacaa 4260
gggatgattt agaaccagaa gaagaagaga aagaaaaatc tgacgatgag gatgatgaaa 4320
gcgataacga aacccaagcg aaccaaaaag aaactgacaa tcaagaacaa gataatgaag 4380
tagttgatag ttacaatttt gcacaaagtt attatgaaga ccaacaaaaa caattgtggt 4440
gtgaaataac atttggtttg ccccttgtcgt tcaaaaaatt ggatcttact gcaattttaa 4500
aggagactgc cggcaaatct gttctttggg aaacgcccca aattaaaaga gccattactt 4560
atgtgaagga tgataaatta atgcttagaa cggatggtat taatattgtt gaaatgttta 4620
aatacaatac ccttttagac ttgccacaac tttattgtaa tgatatccat aaagtggcag 4680
aaacatacgg cattgaagca gcatctaaag taatagtaaa ggaggttaaa gacgtattta 4740
atgtgtacgg aattaaagta gatcctcgtc atttgtccct agtagccgac tacatgacat 4800
ttaatggtac atttgaacca ctcagcagaa gaggaatgga aaacagcgct tcccctctgc 4860
aacagatgtc atttgaatca tctttagtat ttttaaggaa tgcagcaatt agaggccgag 4920
aagatgattt acaaaaccct tcgagtagtc ttatgttagg aaaaccatgt ggaaccggca 4980
caggaagctt taccctttta cataagtcct ttgtaacatg ttaataaata aattgttata 5040

gataaaaaaa aaa 5053

<210> 2
<211> 1633
<212> PRT
<213> Diabrotica virgifera

<400> 2

Met Lys Ile His Phe Ile Pro Asp Lys Val Ser Phe Ser Leu Phe Thr
1 5 10 15

Thr Glu Glu Ile Lys Lys Met Cys Val Thr Gln Ile Ile Thr Pro Leu
20 25 30

Met Leu Asp Pro Leu Gly His Pro Leu Gln Gly Gly Leu Tyr Asp Arg
35 40 45

Lys Leu Gly Pro Tyr Ser Ala Lys Asp Thr Leu Cys Glu Ser Cys Asn
50 55 60

Gln Thr Phe Thr Asn Cys Pro Gly His Phe Gly Tyr Ile Glu Leu Pro
65 70 75 80

Leu Pro Val Val Asn Pro Leu Phe His Lys Ile Ile Gly Thr Ile Ile
85 90 95

Lys Ile Ser Cys Leu Ser Cys Phe His Ile Gln Leu Pro Thr His Ile
100 105 110

Lys Lys Val Leu Cys Ile Gln Met Lys Leu Leu Asn Cys Gly Leu Ile
115 120 125

Thr Glu Ala Leu Ser Val Glu Thr Ala Leu Ala Glu Leu Ile Ser Lys
130 135 140

Tyr Glu Lys Phe Glu Asn Ile Pro Thr Glu Ser Ile Lys Ser Val Leu
145 150 155 160

Arg Tyr Glu Glu Leu Ala Asp Glu Thr Leu Lys Asn Leu Glu Gly Lys
165 170 175

Asn Val Thr Ser Gln Asn Thr Glu Thr Leu Arg Asn Asn Phe Val Ser
180 185 190

Lys Met Leu Lys Glu Val Lys Ser Arg Gln Leu Cys Ile Phe Cys Lys
195 200 205

```
Asn Arg Ile Asn Arg Ile Gln Ala Leu Lys Asn Arg Ile Ile Leu Thr
    210                 215                 220

Lys Lys Lys Gly Asp Leu Asp Asp Ser Asn Val Val Met Gly Gln Lys
225                 230                 235                 240

Val Thr Gly Met Glu Ser Gln Tyr Ile Thr Pro Glu Glu Ser Arg Gln
                245                 250                 255

Tyr Leu Arg Asn Ile Trp Arg Gln Glu Lys Glu Phe Leu Gln Gln Leu
            260                 265                 270

Val Ser Val Leu Ala Asn Val Asp Cys Glu His Pro Thr Asp Ala Phe
        275                 280                 285

Tyr Phe Glu Val Ile Pro Val Pro Pro Pro Asn Val Arg Pro Val Asn
    290                 295                 300

Phe Val Asn Gly Arg Ile Leu Glu Asn Lys Gln Ser Ile Gly Tyr Lys
305                 310                 315                 320

Asn Ile Ile Gln Asn Val Ile Leu Leu Lys Thr Ile Ile Gln Val Val
                325                 330                 335

Gln Ser Lys Ser Asp Ile Asn Ser Leu Ser Ser Val Glu Ala Lys Ser
            340                 345                 350

Ala Tyr Ser Ile Ala Arg Gly Asn Ser Pro Val Glu Lys Leu Asn Tyr
        355                 360                 365

Cys Trp Glu Glu Leu Gln Ser Asp Val Asn Gly Leu Leu Asp Asn Glu
    370                 375                 380

Asn Val Arg Gln Glu Gly Gln Gly Leu Lys Gln Ile Ile Glu Lys Lys
385                 390                 395                 400

Glu Gly Val Ile Arg Met Cys Met Met Gly Lys Arg Val Asn Phe Ser
                405                 410                 415

Ala Arg Ser Val Ile Thr Pro Asp Pro Asn Leu Asn Ile Asp Glu Ile
            420                 425                 430

Gly Ile Pro Glu Glu Phe Ala Lys Lys Leu Thr Tyr Pro Val Ala Val
        435                 440                 445

Thr Pro Trp Asn Val Glu Glu Leu Arg Lys Met Ile Leu Asn Gly Pro
    450                 455                 460
```

Asn Val His Pro Gly Ala Ile Met Ile Glu Asn Asn Gly Ile Leu Lys
465 470 475 480

Arg Ile Asn Pro Tyr Asn Glu Val Gln Gln Lys Ser Ile Leu Lys Cys
485 490 495

Leu Leu Thr Pro Glu Ala Thr Lys Gly Pro Lys Gly Gln Gly Ile Gln
500 505 510

Ile Val His Arg His Leu Cys Asn Gly Asp Val Leu Leu Leu Asn Arg
515 520 525

Gln Pro Thr Leu His Lys Pro Ser Ile Met Ala His Thr Ala Arg Ile
530 535 540

Leu Lys Gly Glu Lys Thr Leu Arg Leu His Tyr Ala Asn Cys Lys Ala
545 550 555 560

Tyr Asn Ala Asp Phe Asp Gly Asp Glu Met Asn Ala His Tyr Pro Gln
565 570 575

Asn Glu Leu Ala Arg Ser Glu Gly Tyr Asn Ile Val Asn Val Ser Asn
580 585 590

Gln Tyr Leu Val Pro Lys Asp Gly Thr Pro Leu Ser Gly Leu Ile Gln
595 600 605

Asp His Met Ile Ser Gly Val Arg Leu Ser Leu Arg Gly Arg Phe Phe
610 615 620

Asp Lys Gln Asp Tyr Glu His Leu Val Tyr Gln Ala Leu Ser Phe Lys
625 630 635 640

Thr Gly Arg Ile Lys Leu Leu Pro Pro Thr Ile Ile Lys Pro Gln Val
645 650 655

Leu Trp Ser Gly Lys Gln Ile Leu Ser Thr Val Ile Ile Asn Val Ile
660 665 670

Pro Asp Glu Arg Glu Leu Ile Asn Leu Thr Ser Thr Ala Lys Ile Ser
675 680 685

Ser Lys Ala Trp Gln Arg Arg Pro Ser Arg Arg Trp Arg Ala Gly Gly
690 695 700

Thr Ile Phe Leu Asp Asp Lys Val Met Ser Glu Ala Glu Val Ile Ile
705 710 715 720

```
Arg Gly Gly Glu Leu Leu Val Gly Val Leu Asp Lys Thr His Tyr Gly
                725                 730                 735

Ser Thr Pro Tyr Gly Leu Val His Cys Ile Tyr Glu Leu Tyr Gly Gly
            740                 745                 750

Thr Tyr Ala Ile Arg Leu Leu Ser Ser Leu Thr Lys Leu Phe Met Arg
        755                 760                 765

Phe Leu Gln Gln Glu Gly Phe Thr Leu Gly Val His Asp Ile Leu Thr
    770                 775                 780

Val Glu Arg Ala Asp Val Arg Arg Arg Glu Ile Ile Lys Asp Cys Arg
785                 790                 795                 800

Gln Val Gly Lys Glu Ala Val Thr Lys Ala Leu Asp Val Pro Leu Asp
                805                 810                 815

Thr Pro Asp Ala Glu Val Val Glu Thr Ile Glu Lys Leu Ser Ala Ala
            820                 825                 830

Asp Pro Lys Ile Arg Ala Thr Ile Asp Arg Ser Tyr Lys Ser Ser Met
        835                 840                 845

Asp Ile Phe Thr Asn Glu Ile Asn Arg Thr Cys Leu Pro Ala Gly Leu
    850                 855                 860

Val Cys Lys Phe Pro Glu Asn Asn Leu Gln Leu Met Val Gln Ser Gly
865                 870                 875                 880

Ala Lys Gly Ser Thr Val Asn Thr Met Gln Ile Ser Cys Leu Leu Gly
                885                 890                 895

Gln Ile Glu Leu Glu Gly Lys Arg Pro Pro Val Met Ile Ser Gly Lys
            900                 905                 910

Ser Leu Pro Ser Phe Pro Ser Phe Glu Phe Thr Pro Arg Ala Gly Gly
        915                 920                 925

Phe Ile Asp Gly Arg Phe Met Thr Gly Ile Gln Pro Gln Glu Phe Phe
    930                 935                 940

Phe His Cys Met Ala Gly Arg Glu Gly Leu Ile Asp Thr Ala Val Lys
945                 950                 955                 960

Thr Ser Arg Ser Gly Tyr Leu Gln Arg Cys Leu Ile Lys His Leu Glu
```

965 970 975

Gly Leu Arg Val Gly Tyr Asp Met Thr Val Arg Asn Ser Asp Lys Ser
980 985 990

Val Ile Gln Phe Leu Tyr Gly Glu Asp Gly Met Asp Ile Ser Lys Ala
995 1000 1005

Gln Phe Phe Asn Glu Lys Gln Met Ser Phe Leu Ala Glu Asn Ile
1010 1015 1020

Lys Val Leu Gly Asn Ser Asp Thr Leu Lys Gln Leu Lys Asn Glu
1025 1030 1035

Glu Asp Gln Glu Ala Val Lys Glu His Gly Lys Ala Val Lys Glu
1040 1045 1050

Trp Lys Lys His His Gly Asn Pro Leu Asn His Arg Arg Asn Ser
1055 1060 1065

Pro Phe Ser Leu Phe Ala Lys Tyr Val Gln Asn Arg Thr Gly Asp
1070 1075 1080

Asn Asn Leu Leu Thr Lys Glu Lys Leu Met Lys Leu Trp Tyr Glu
1085 1090 1095

Met Asp Lys Asp Ile Lys Thr Asn Phe Thr Asp Gln Cys Glu Lys
1100 1105 1110

Cys Pro Asp Pro Ile Glu Ala Val Tyr Gln Pro Asp Ala Asn Phe
1115 1120 1125

Gly Ala Ile Asn Glu Thr Val Gln Lys Leu Ile Lys Asn Tyr Lys
1130 1135 1140

Gly Phe Asp Asn Lys Lys Ser Lys Lys Lys Phe Glu Asp Val Ile
1145 1150 1155

Lys Leu Lys Val Met Glu Ser Met Cys Ser Ala Gly Glu Pro Val
1160 1165 1170

Gly Leu Leu Ala Ala Gln Ser Ile Gly Glu Pro Ser Thr Gln Met
1175 1180 1185

Thr Leu Asn Thr Phe His Phe Ala Gly Arg Gly Glu Met Asn Val
1190 1195 1200

Thr Leu Gly Ile Pro Arg Leu Arg Glu Ile Leu Met Met Ala Ser
1205 1210 1215

Lys Asn Ile Lys Thr Pro Ser Met Glu Ile Pro Phe Leu Gln Val
1220 1225 1230

Pro Asp Leu Glu Tyr Lys Ala Asn Glu Leu Arg Lys Leu Leu Thr
1235 1240 1245

Arg Val Val Val Ala Asp Val Leu Glu Thr Ile Asp Val Thr Val
1250 1255 1260

Glu Leu Gln Phe Lys Pro Ile Arg Gln Tyr Lys Tyr Thr Leu Lys
1265 1270 1275

Phe Gln Phe Leu Pro Lys Lys Tyr Tyr Ser Met Asp Tyr Cys Val
1280 1285 1290

Asn Pro Thr Lys Ile Leu Arg His Met Lys Gly Lys Tyr Phe Gly
1295 1300 1305

Glu Met Phe Ala Ser Ile Lys Lys Val Ser Lys Ile Asn Ser Asn
1310 1315 1320

Ile Val Met Met Glu Glu Glu Arg Asn Lys Lys Arg Thr Thr Asn
1325 1330 1335

Asn Glu Glu Asp Glu Asp Arg Pro Glu Thr Asn Glu Arg Glu Gly
1340 1345 1350

Asp Asn Gln Ile Asp Ser Ser Asp Asp Glu Val Glu Asp Asn Glu
1355 1360 1365

Asp Ala Lys Gln Ser His Lys Tyr Gln Glu Thr Arg Asp Asp Leu
1370 1375 1380

Glu Pro Glu Glu Glu Glu Lys Glu Lys Ser Asp Asp Glu Asp Asp
1385 1390 1395

Glu Ser Asp Asn Glu Thr Gln Ala Asn Gln Lys Glu Thr Asp Asn
1400 1405 1410

Gln Glu Gln Asp Asn Glu Val Val Asp Ser Tyr Asn Phe Ala Gln
1415 1420 1425

Ser Tyr Tyr Glu Asp Gln Gln Lys Gln Leu Trp Cys Glu Ile Thr
1430 1435 1440

Phe Gly Leu Pro Leu Ser Phe Lys Lys Leu Asp Leu Thr Ala Ile
1445 1450 1455

Leu Lys Glu Thr Ala Gly Lys Ser Val Leu Trp Glu Thr Pro Gln
1460 1465 1470

Ile Lys Arg Ala Ile Thr Tyr Val Lys Asp Asp Lys Leu Met Leu
1475 1480 1485

Arg Thr Asp Gly Ile Asn Ile Val Glu Met Phe Lys Tyr Asn Thr
1490 1495 1500

Leu Leu Asp Leu Pro Gln Leu Tyr Cys Asn Asp Ile His Lys Val
1505 1510 1515

Ala Glu Thr Tyr Gly Ile Glu Ala Ala Ser Lys Val Ile Val Lys
1520 1525 1530

Glu Val Lys Asp Val Phe Asn Val Tyr Gly Ile Lys Val Asp Pro
1535 1540 1545

Arg His Leu Ser Leu Val Ala Asp Tyr Met Thr Phe Asn Gly Thr
1550 1555 1560

Phe Glu Pro Leu Ser Arg Arg Gly Met Glu Asn Ser Ala Ser Pro
1565 1570 1575

Leu Gln Gln Met Ser Phe Glu Ser Ser Leu Val Phe Leu Arg Asn
1580 1585 1590

Ala Ala Ile Arg Gly Arg Glu Asp Asp Leu Gln Asn Pro Ser Ser
1595 1600 1605

Ser Leu Met Leu Gly Lys Pro Cys Gly Thr Gly Thr Gly Ser Phe
1610 1615 1620

Thr Leu Leu His Lys Ser Phe Val Thr Cys
1625 1630

<210> 3
<211> 6927
<212> DNA
<213> Diabrotica virgifera

<400> 3
tgctcgacct gtagattctt gtaacggatt tcggagagtt cgattcgttg tcgagccttc 60

aaaatggcta ccaacgatag taaagctccg ttgaggacag ttaaaagagt gcaatttgga 120

atacttagtc cagatgaaat tagacgaatg tcagtcacag aagggggcat ccgcttccca 180
gaaaccatgg aagcaggccg ccccaaacta tgcggtctta tggaccccag acaaggtgtc 240
atagacagaa gctcaagatg ccagacatgt gccggaaata tgacagaatg tcctggacat 300
ttcggacata tcgagctggc aaaaccagtt ttccacgtag gattcgtaac aaaaacaata 360
aagatcttga gatgcgtttg cttcttttgc agtaaattat tagtcagtcc aaataatccg 420
aaaattaaag aagttgtaat gaaatcaaag ggacagccac gtaaaagatt agctttcgtt 480
tatgatctgt gtaaaggtaa aaatatttgt gaaggtggag atgaaatgga tgtgggtaaa 540
gaaagcgaag atcccaataa aaaagcaggc catggtggtt gtggtcgata tcaaccaaat 600
atcagacgtg ccggtttaga tttaacagca gaatggaaac acgtcaatga agacacacaa 660
gaaaagaaaa tcgcactatc tgccgaacgt gtctgggaaa tcctaaaaca tatcacagat 720
gaagaatgtt tcattcttgg tatggatccc aaatttgcta gaccagattg gatgatagta 780
acggtacttc ctgttcctcc cctagcagta cgacctgctg tagttatgca cggatctgca 840
aggaatcagg atgatatcac tcacaaattg gccgacatta tcaaggcgaa taacgaatta 900
cagaagaacg agtctgcagg tgcagccgct catataatca cagaaaatat taagatgttg 960
caatttcacg tcgccacttt agttgacaac gatatgccgg gaatgccgag agcaatgcaa 1020
aaatctggaa aacccctaaa agctatcaaa gctcggctga aaggtaaaga aggaaggatt 1080
cgaggtaacc ttatgggaaa gcgtgtggac ttttctgcac gtactgtcat cacaccagat 1140
cccaatttac gtatcgacca agtaggagtg cctagaagta ttgctcaaaa catgacgttt 1200
ccagaaatcg tcacaccttt caattttgac aaaatgttgg aattggtaca gagaggtaat 1260
tctcagtatc caggagctaa gtatatcatc agagacaatg gagagaggat tgatttacgt 1320
ttccacccaa aaccgtcaga tttacatttg cagtgtggtt ataaggtaga aagacacatc 1380
agagacggcg atctagtaat cttcaaccgt caaccaaccc tccacaagat gagtatgatg 1440
ggccacagag tcaaagtctt accctggtcg acgttccgta tgaatctctc gtgcacctct 1500
ccctacaacg ccgattttga cggcgacgaa atgaacctcc atgtgcccca aagtatggaa 1560
actcgagctg aagtcgaaaa cctccacatc actcccaggc aaatcattac tccgcaagct 1620
aaccaacccg tcatgggtat tgtacaagat acgttgacag ctgttaggaa gatgacaaaa 1680
agggatgtat tcatcgagaa ggaacaaatg atgaatatat tgatgttctt gccaatttgg 1740
gatggtaaaa tgccccgtcc agccatcctc aaacccaaac cgttgtggac aggaaaacag 1800
atattttccc tgatcattcc tggcaatgta aatatgatac gtacccattc tacgcatcca 1860
gacgacgagg acgacggtcc ctataaatgg atatcgccag gagatacgaa agttatggta 1920
gaacatggag aattggtcat gggtatattg tgtaagaaaa gtcttggaac atcagcaggt 1980
tccctgctgc atatttgtat gttggaatta ggacacgaag tgtgtggtag attttatggt 2040

aacattcaaa ctgtaatcaa caactggttg ttgttagaag gtcacagcat cggtattgga 2100

gacaccattg ccgatcctca gacttacaca gaaattcaga gagccatcag gaaagccaaa 2160

gaagatgtaa tagaagtcat ccagaaagct cacaacatgg aactggaacc gactcccggt 2220

aatacgttgc gtcagacttt cgaaaatcaa gtaaacagaa ttctaaacga cgctcgtgac 2280

aaaactggtg gttccgctaa gaaatctttg actgaataca ataacctaaa ggctatggtc 2340

gtatcgggat ccaagggatc caacattaat atttcccagg ttattgcttg cgtgggtcaa 2400

cagaacgtag aaggtaaacg tattccattt ggcttcagaa aacgcacgtt gccgcacttc 2460

atcaaggacg attacggtcc tgaatccaga ggtttcgtag aaaattcgta tcttgccggt 2520

ctcactcctt cggagttcta tttccacgct atgggaggtc gtgaaggtct tatcgatact 2580

gctgtaaaaa ctgccgaaac tggttacatc caacgtcgtc tgataaaggc tatggagagt 2640

gtaatggtac actacgacgg taccgtaaga aattctgtag gacaacttat ccagctgaga 2700

tacggtgaag acggactctg tggagagatg gtagagtttc aatatttagc aacagtcaaa 2760

ttaagtaaca aggcgtttga gagaaaattc agatttgatc caagtaatga aaggtatttg 2820

agaagagttt tcaatgaaga agttatcaag caactgatgg gttcagggga agtcatttcc 2880

gaacttgaga gagaatggga acaactccag aaagacagag aagccttaag acaaatcttc 2940

cctagcggag aatctaaagt agtactcccc tgtaacttac aacgtatgat ctggaatgta 3000

caaaaaattt tccacataaa caaacgagcc ccgacagacc tgtccccgtt aagagttatc 3060

caaggcgttc gagaattact caggaaatgc gtcatcgtag ctggcgagga tcgtctgtcc 3120

aaacaagcca acgaaaacgc aacgttactc ttccagtgtc tagtcagatc gaccctctgc 3180

accaaatgcg tttctgaaga attcaggctc agcaccgaag ccttcgagtg gttgatagga 3240

gaaatcgaga cgaggttcca acaagcccaa gccaatcctg gagaaatggt gggcgctctg 3300

gccgcgcagt cactgggaga acccgctact cagatgacac tgaacacttt ccattttgct 3360

ggtgtatcct ccaagaacgt aaccctgggt gtacctagat taaaggaaat tattaatatt 3420

tccaagaaac ccaaggctcc atctctaacc gtgtttttaa ctggtgcggc tgctagagat 3480

gcggaaaaag cgaagaatgt gttatgcaga cttgaacaca ccactcttcg taaagtaacc 3540

gccaacaccg ccatctatta cgatcctgac ccacaaaata ccgtcattcc tgaggatcag 3600

gagttcgtta acgtctacta tgaaatgccc gatttcgatc ctacccgtat atcgccgtgg 3660

ttgcttcgta tcgaactgga cagaaagaga atgacagata agaaactaac tatggaacaa 3720

attgctgaaa agatcaacgc tgggttcggg gacgatttga attgtatttt caacgacgac 3780

aatgctgaaa agttggtgct gcgtatcaga atcatgaaca gcgacgatgg aaaattcgga 3840

gaaggtgctg atgaggacgt agacaaaatg gatgacgaca tgtttttgag atgcatcgaa 3900

gcgaacatgc tgagcgatat gaccttgcaa ggtatagaag cgatttccaa ggtatacatg 3960
cacttgccac agactgactc gaaaaaaagg atcgtcatca ctgaaacagg cgaatttaag 4020
gccatcgcag aatggctatt ggaaactgac ggtaccagca tgatgaaagt actgtcagaa 4080
agagacgtcg atccggtcag gacgttttct aacgacattt gtgaaatatt ttcggtactt 4140
ggtatcgagg ctgtgcgtaa gtctgtagag aaagaaatga acgctgtcct ttcattctac 4200
ggtctgtacg taaactatcg ccatcttgcc ttgctttgtg acgtaatgac agccaaaggt 4260
cacttaatgg ccatcacccg tcacggtatc aacagacaag acactggagc tctgatgagg 4320
tgttccttcg aggaaactgt agatgtattg atggacgctg ccagtcatgc ggaggtcgac 4380
ccaatgagag gagtatctga aaacattatc ctcggtcaac taccaagaat gggcacaggc 4440
tgcttcgatc ttttgctgga cgccgaaaaa tgtaaaatgg gaattgccat acctcaagcg 4500
cacagcagcg atctaatggc ttcaggaatg ttctttggat tagccgctac acccagcagt 4560
atgagtccag gtggtgctat gaccccatgg aatcaagcag ctacaccata cgttggcagt 4620
atctggtctc cacagaattt aatgggcagt ggaatgacac caggtggtgc cgctttctcc 4680
ccatcagctg cgtcagatgc atcaggaatg tcaccagctt atggcggttg gtcaccaaca 4740
ccacaatctc ctgcaatgtc gccatatatg gcttctccac atggacaatc gccttcctac 4800
agtccatcaa gtccagcgtt ccaacctact tcaccatcca tgacgccgac ctctcctgga 4860
tattctccca gttctcctgg ttattcacct accagtctca attacagtcc aacgagtccc 4920
agttattcac ccacttctca gagttactcc ccaacctcac ctagttactc accgacttct 4980
ccaaattatt cacctacttc cccaagctac agtccaacat cccctaacta ttcaccaaca 5040
tctcccaact attcacccac ttcacctagt tatccttcaa cttcgccagg ttacagcccc 5100
acttcacgca gctactcacc cacatctcct agttactcag gaacttcgcc ctcttattca 5160
ccaacttcgc caagttactc ccctacttct cctagttatt cgccgtcgtc tcctaattac 5220
tctcccactt ctccaaatta cagtcccact tctcctaatt actcaccgtc ctctcctagg 5280
tacacgcccg gttctcctag tttttcccca agttcgaaca gttactctcc cacatctcct 5340
caatattctc caacatctcc aagttattcg ccttcttcgc ccaaatattc accaacttcc 5400
cccaattatt cgccaacatc tccatcattt tctggaggaa gtccacaata ttcacccaca 5460
tcaccgaaat actctccaac ctcgcccaat tacactctgt cgagtccgca gcacactcca 5520
acaggtagca gtcgatattc accgtcaact tcgagttatt ctcctaattc gcccaattat 5580
tcaccgacgt ctccacaata ctccatccac agtacaaaat attcccctgc aagtcctaca 5640
ttcacaccca ccagtcctag tttctctccc gcttcacccg catattcgcc tcaacctatg 5700
tattcacctt cttctcctaa ttattctccc actagtccca gtcaagacac tgactaaata 5760
taatcataag attgtagtgg ttagttgtat tttatacata gattttaatt cagaatttaa 5820

tattattttt tactatttac cagggacatt tttaaagttg taaaaacact tacatttgtt 5880

ccaacggatt tttgcacaaa cgtaacgaag ttaaatcaaa acattacaac tgaaacatac 5940

gtcggtatgt actgtcaatg tgatcattag gaaatggcta ttatcccgga ggacgtattt 6000

tataaagtta ttttattgaa gtgtttgatc ttttttcact attgaggaga tttatggact 6060

caacattaaa cagcttgaac atcataccga ctactactaa tataaagata aatatagaac 6120

ggtaagaaat agattaaaaa aaaatacaat aagttaaaca gtaatcataa aaataaatac 6180

gtttccgttc gacagaacta tagccagatt cttgtagtat aatgaaaatt tgtaggttaa 6240

aaatattact tgtcacatta gcttaaaaat aaaaaattac cggaagtaat caaataagag 6300

agcaacagtt agtcgttcta acaattatgt ttgaaaataa aaattacaat gagttataca 6360

aacgaagact acaagtttaa atagtatgaa aaactatttg taaacacaac aaatgcgcat 6420

tgaaatttat ttatcgtact taacttattt gccttacaaa aataatactc cgcgagtatt 6480

ttttatgaac tgtaaaacta aaaagttgta cagttcacac aaaaacatcg aaaaattttg 6540

tttttgtatg tttctattat taaaaaaata ctttttatct ttcaccttat aggtactatt 6600

tgactctatg acattttctc tacatttctt taaatctgtt ctatttatta tgtacatgaa 6660

tctataagca caaataatat acataatcat tttgataaaa aatcatagtt ttaaataaaa 6720

cagatttcaa cacaatattc ataagtctac ttttttaaaa atttatagag acaaaggcca 6780

tttttcagaa acagattaaa caaaaatcac tataaattat tttgagtatg ttgaataagt 6840

ttatattgct tctacaattt ttaaatataa aattataaca ttagcagagg aacaacgaga 6900

attaaggtcg ggaagatcat gcaccga 6927

<210> 4
<211> 1897
<212> PRT
<213> Diabrotica virgifera

<400> 4

Met Ala Thr Asn Asp Ser Lys Ala Pro Leu Arg Thr Val Lys Arg Val
1 5 10 15

Gln Phe Gly Ile Leu Ser Pro Asp Glu Ile Arg Arg Met Ser Val Thr
20 25 30

Glu Gly Gly Ile Arg Phe Pro Glu Thr Met Glu Ala Gly Arg Pro Lys
35 40 45

Leu Cys Gly Leu Met Asp Pro Arg Gln Gly Val Ile Asp Arg Ser Ser
50 55 60

```
Arg Cys Gln Thr Cys Ala Gly Asn Met Thr Glu Cys Pro Gly His Phe
65                  70                  75                  80

Gly His Ile Glu Leu Ala Lys Pro Val Phe His Val Gly Phe Val Thr
                85                  90                  95

Lys Thr Ile Lys Ile Leu Arg Cys Val Cys Phe Phe Cys Ser Lys Leu
            100                 105                 110

Leu Val Ser Pro Asn Asn Pro Lys Ile Lys Glu Val Val Met Lys Ser
        115                 120                 125

Lys Gly Gln Pro Arg Lys Arg Leu Ala Phe Val Tyr Asp Leu Cys Lys
    130                 135                 140

Gly Lys Asn Ile Cys Glu Gly Gly Asp Glu Met Asp Val Gly Lys Glu
145                 150                 155                 160

Ser Glu Asp Pro Asn Lys Lys Ala Gly His Gly Gly Cys Gly Arg Tyr
                165                 170                 175

Gln Pro Asn Ile Arg Arg Ala Gly Leu Asp Leu Thr Ala Glu Trp Lys
            180                 185                 190

His Val Asn Glu Asp Thr Gln Glu Lys Lys Ile Ala Leu Ser Ala Glu
        195                 200                 205

Arg Val Trp Glu Ile Leu Lys His Ile Thr Asp Glu Glu Cys Phe Ile
    210                 215                 220

Leu Gly Met Asp Pro Lys Phe Ala Arg Pro Asp Trp Met Ile Val Thr
225                 230                 235                 240

Val Leu Pro Val Pro Pro Leu Ala Val Arg Pro Ala Val Val Met His
                245                 250                 255

Gly Ser Ala Arg Asn Gln Asp Asp Ile Thr His Lys Leu Ala Asp Ile
            260                 265                 270

Ile Lys Ala Asn Asn Glu Leu Gln Lys Asn Glu Ser Ala Gly Ala Ala
        275                 280                 285

Ala His Ile Ile Thr Glu Asn Ile Lys Met Leu Gln Phe His Val Ala
    290                 295                 300

Thr Leu Val Asp Asn Asp Met Pro Gly Met Pro Arg Ala Met Gln Lys
305                 310                 315                 320
```

```
Ser Gly Lys Pro Leu Lys Ala Ile Lys Ala Arg Leu Lys Gly Lys Glu
                325                 330                 335

Gly Arg Ile Arg Gly Asn Leu Met Gly Lys Arg Val Asp Phe Ser Ala
            340                 345                 350

Arg Thr Val Ile Thr Pro Asp Pro Asn Leu Arg Ile Asp Gln Val Gly
        355                 360                 365

Val Pro Arg Ser Ile Ala Gln Asn Met Thr Phe Pro Glu Ile Val Thr
    370                 375                 380

Pro Phe Asn Phe Asp Lys Met Leu Glu Leu Val Gln Arg Gly Asn Ser
385                 390                 395                 400

Gln Tyr Pro Gly Ala Lys Tyr Ile Ile Arg Asp Asn Gly Glu Arg Ile
                405                 410                 415

Asp Leu Arg Phe His Pro Lys Pro Ser Asp Leu His Leu Gln Cys Gly
            420                 425                 430

Tyr Lys Val Glu Arg His Ile Arg Asp Gly Asp Leu Val Ile Phe Asn
        435                 440                 445

Arg Gln Pro Thr Leu His Lys Met Ser Met Met Gly His Arg Val Lys
    450                 455                 460

Val Leu Pro Trp Ser Thr Phe Arg Met Asn Leu Ser Cys Thr Ser Pro
465                 470                 475                 480

Tyr Asn Ala Asp Phe Asp Gly Asp Glu Met Asn Leu His Val Pro Gln
                485                 490                 495

Ser Met Glu Thr Arg Ala Glu Val Glu Asn Leu His Ile Thr Pro Arg
            500                 505                 510

Gln Ile Ile Thr Pro Gln Ala Asn Gln Pro Val Met Gly Ile Val Gln
        515                 520                 525

Asp Thr Leu Thr Ala Val Arg Lys Met Thr Lys Arg Asp Val Phe Ile
    530                 535                 540

Glu Lys Glu Gln Met Met Asn Ile Leu Met Phe Leu Pro Ile Trp Asp
545                 550                 555                 560

Gly Lys Met Pro Arg Pro Ala Ile Leu Lys Pro Lys Pro Leu Trp Thr
                565                 570                 575
```

```
Gly Lys Gln Ile Phe Ser Leu Ile Ile Pro Gly Asn Val Asn Met Ile
            580                 585                 590

Arg Thr His Ser Thr His Pro Asp Asp Glu Asp Asp Gly Pro Tyr Lys
        595                 600                 605

Trp Ile Ser Pro Gly Asp Thr Lys Val Met Val Glu His Gly Glu Leu
    610                 615                 620

Val Met Gly Ile Leu Cys Lys Lys Ser Leu Gly Thr Ser Ala Gly Ser
625                 630                 635                 640

Leu Leu His Ile Cys Met Leu Glu Leu Gly His Glu Val Cys Gly Arg
                645                 650                 655

Phe Tyr Gly Asn Ile Gln Thr Val Ile Asn Asn Trp Leu Leu Leu Glu
            660                 665                 670

Gly His Ser Ile Gly Ile Gly Asp Thr Ile Ala Asp Pro Gln Thr Tyr
        675                 680                 685

Thr Glu Ile Gln Arg Ala Ile Arg Lys Ala Lys Glu Asp Val Ile Glu
    690                 695                 700

Val Ile Gln Lys Ala His Asn Met Glu Leu Glu Pro Thr Pro Gly Asn
705                 710                 715                 720

Thr Leu Arg Gln Thr Phe Glu Asn Gln Val Asn Arg Ile Leu Asn Asp
                725                 730                 735

Ala Arg Asp Lys Thr Gly Gly Ser Ala Lys Lys Ser Leu Thr Glu Tyr
            740                 745                 750

Asn Asn Leu Lys Ala Met Val Val Ser Gly Ser Lys Gly Ser Asn Ile
        755                 760                 765

Asn Ile Ser Gln Val Ile Ala Cys Val Gly Gln Gln Asn Val Glu Gly
    770                 775                 780

Lys Arg Ile Pro Phe Gly Phe Arg Lys Arg Thr Leu Pro His Phe Ile
785                 790                 795                 800

Lys Asp Asp Tyr Gly Pro Glu Ser Arg Gly Phe Val Glu Asn Ser Tyr
                805                 810                 815

Leu Ala Gly Leu Thr Pro Ser Glu Phe Tyr Phe His Ala Met Gly Gly
```

820 825 830

Arg Glu Gly Leu Ile Asp Thr Ala Val Lys Thr Ala Glu Thr Gly Tyr
835 840 845

Ile Gln Arg Arg Leu Ile Lys Ala Met Glu Ser Val Met Val His Tyr
850 855 860

Asp Gly Thr Val Arg Asn Ser Val Gly Gln Leu Ile Gln Leu Arg Tyr
865 870 875 880

Gly Glu Asp Gly Leu Cys Gly Glu Met Val Glu Phe Gln Tyr Leu Ala
885 890 895

Thr Val Lys Leu Ser Asn Lys Ala Phe Glu Arg Lys Phe Arg Phe Asp
900 905 910

Pro Ser Asn Glu Arg Tyr Leu Arg Arg Val Phe Asn Glu Glu Val Ile
915 920 925

Lys Gln Leu Met Gly Ser Gly Glu Val Ile Ser Glu Leu Glu Arg Glu
930 935 940

Trp Glu Gln Leu Gln Lys Asp Arg Glu Ala Leu Arg Gln Ile Phe Pro
945 950 955 960

Ser Gly Glu Ser Lys Val Val Leu Pro Cys Asn Leu Gln Arg Met Ile
965 970 975

Trp Asn Val Gln Lys Ile Phe His Ile Asn Lys Arg Ala Pro Thr Asp
980 985 990

Leu Ser Pro Leu Arg Val Ile Gln Gly Val Arg Glu Leu Leu Arg Lys
995 1000 1005

Cys Val Ile Val Ala Gly Glu Asp Arg Leu Ser Lys Gln Ala Asn
1010 1015 1020

Glu Asn Ala Thr Leu Leu Phe Gln Cys Leu Val Arg Ser Thr Leu
1025 1030 1035

Cys Thr Lys Cys Val Ser Glu Glu Phe Arg Leu Ser Thr Glu Ala
1040 1045 1050

Phe Glu Trp Leu Ile Gly Glu Ile Glu Thr Arg Phe Gln Gln Ala
1055 1060 1065

Gln Ala Asn Pro Gly Glu Met Val Gly Ala Leu Ala Ala Gln Ser
1070 1075 1080

Leu Gly Glu Pro Ala Thr Gln Met Thr Leu Asn Thr Phe His Phe
1085 1090 1095

Ala Gly Val Ser Ser Lys Asn Val Thr Leu Gly Val Pro Arg Leu
1100 1105 1110

Lys Glu Ile Ile Asn Ile Ser Lys Lys Pro Lys Ala Pro Ser Leu
1115 1120 1125

Thr Val Phe Leu Thr Gly Ala Ala Ala Arg Asp Ala Glu Lys Ala
1130 1135 1140

Lys Asn Val Leu Cys Arg Leu Glu His Thr Thr Leu Arg Lys Val
1145 1150 1155

Thr Ala Asn Thr Ala Ile Tyr Tyr Asp Pro Asp Pro Gln Asn Thr
1160 1165 1170

Val Ile Pro Glu Asp Gln Glu Phe Val Asn Val Tyr Tyr Glu Met
1175 1180 1185

Pro Asp Phe Asp Pro Thr Arg Ile Ser Pro Trp Leu Leu Arg Ile
1190 1195 1200

Glu Leu Asp Arg Lys Arg Met Thr Asp Lys Lys Leu Thr Met Glu
1205 1210 1215

Gln Ile Ala Glu Lys Ile Asn Ala Gly Phe Gly Asp Asp Leu Asn
1220 1225 1230

Cys Ile Phe Asn Asp Asp Asn Ala Glu Lys Leu Val Leu Arg Ile
1235 1240 1245

Arg Ile Met Asn Ser Asp Asp Gly Lys Phe Gly Glu Gly Ala Asp
1250 1255 1260

Glu Asp Val Asp Lys Met Asp Asp Asp Met Phe Leu Arg Cys Ile
1265 1270 1275

Glu Ala Asn Met Leu Ser Asp Met Thr Leu Gln Gly Ile Glu Ala
1280 1285 1290

Ile Ser Lys Val Tyr Met His Leu Pro Gln Thr Asp Ser Lys Lys
1295 1300 1305

Arg Ile Val Ile Thr Glu Thr Gly Glu Phe Lys Ala Ile Ala Glu
1310 1315 1320

Trp Leu Leu Glu Thr Asp Gly Thr Ser Met Met Lys Val Leu Ser
1325 1330 1335

Glu Arg Asp Val Asp Pro Val Arg Thr Phe Ser Asn Asp Ile Cys
1340 1345 1350

Glu Ile Phe Ser Val Leu Gly Ile Glu Ala Val Arg Lys Ser Val
1355 1360 1365

Glu Lys Glu Met Asn Ala Val Leu Ser Phe Tyr Gly Leu Tyr Val
1370 1375 1380

Asn Tyr Arg His Leu Ala Leu Leu Cys Asp Val Met Thr Ala Lys
1385 1390 1395

Gly His Leu Met Ala Ile Thr Arg His Gly Ile Asn Arg Gln Asp
1400 1405 1410

Thr Gly Ala Leu Met Arg Cys Ser Phe Glu Glu Thr Val Asp Val
1415 1420 1425

Leu Met Asp Ala Ala Ser His Ala Glu Val Asp Pro Met Arg Gly
1430 1435 1440

Val Ser Glu Asn Ile Ile Leu Gly Gln Leu Pro Arg Met Gly Thr
1445 1450 1455

Gly Cys Phe Asp Leu Leu Leu Asp Ala Glu Lys Cys Lys Met Gly
1460 1465 1470

Ile Ala Ile Pro Gln Ala His Ser Ser Asp Leu Met Ala Ser Gly
1475 1480 1485

Met Phe Phe Gly Leu Ala Ala Thr Pro Ser Ser Met Ser Pro Gly
1490 1495 1500

Gly Ala Met Thr Pro Trp Asn Gln Ala Ala Thr Pro Tyr Val Gly
1505 1510 1515

Ser Ile Trp Ser Pro Gln Asn Leu Met Gly Ser Gly Met Thr Pro
1520 1525 1530

Gly Gly Ala Ala Phe Ser Pro Ser Ala Ala Ser Asp Ala Ser Gly
1535 1540 1545

Met Ser Pro Ala Tyr Gly Gly Trp Ser Pro Thr Pro Gln Ser Pro
1550 1555 1560

Ala Met Ser Pro Tyr Met Ala Ser Pro His Gly Gln Ser Pro Ser
1565 1570 1575

Tyr Ser Pro Ser Ser Pro Ala Phe Gln Pro Thr Ser Pro Ser Met
1580 1585 1590

Thr Pro Thr Ser Pro Gly Tyr Ser Pro Ser Ser Pro Gly Tyr Ser
1595 1600 1605

Pro Thr Ser Leu Asn Tyr Ser Pro Thr Ser Pro Ser Tyr Ser Pro
1610 1615 1620

Thr Ser Gln Ser Tyr Ser Pro Thr Ser Pro Ser Tyr Ser Pro Thr
1625 1630 1635

Ser Pro Asn Tyr Ser Pro Thr Ser Pro Ser Tyr Ser Pro Thr Ser
1640 1645 1650

Pro Asn Tyr Ser Pro Thr Ser Pro Asn Tyr Ser Pro Thr Ser Pro
1655 1660 1665

Ser Tyr Pro Ser Thr Ser Pro Gly Tyr Ser Pro Thr Ser Arg Ser
1670 1675 1680

Tyr Ser Pro Thr Ser Pro Ser Tyr Ser Gly Thr Ser Pro Ser Tyr
1685 1690 1695

Ser Pro Thr Ser Pro Ser Tyr Ser Pro Thr Ser Pro Ser Tyr Ser
1700 1705 1710

Pro Ser Ser Pro Asn Tyr Ser Pro Thr Ser Pro Asn Tyr Ser Pro
1715 1720 1725

Thr Ser Pro Asn Tyr Ser Pro Ser Ser Pro Arg Tyr Thr Pro Gly
1730 1735 1740

Ser Pro Ser Phe Ser Pro Ser Ser Asn Ser Tyr Ser Pro Thr Ser
1745 1750 1755

Pro Gln Tyr Ser Pro Thr Ser Pro Ser Tyr Ser Pro Ser Ser Pro
1760 1765 1770

Lys Tyr Ser Pro Thr Ser Pro Asn Tyr Ser Pro Thr Ser Pro Ser

1775 1780 1785

Phe Ser Gly Gly Ser Pro Gln Tyr Ser Pro Thr Ser Pro Lys Tyr
1790 1795 1800

Ser Pro Thr Ser Pro Asn Tyr Thr Leu Ser Ser Pro Gln His Thr
1805 1810 1815

Pro Thr Gly Ser Ser Arg Tyr Ser Pro Ser Thr Ser Ser Tyr Ser
1820 1825 1830

Pro Asn Ser Pro Asn Tyr Ser Pro Thr Ser Pro Gln Tyr Ser Ile
1835 1840 1845

His Ser Thr Lys Tyr Ser Pro Ala Ser Pro Thr Phe Thr Pro Thr
1850 1855 1860

Ser Pro Ser Phe Ser Pro Ala Ser Pro Ala Tyr Ser Pro Gln Pro
1865 1870 1875

Met Tyr Ser Pro Ser Ser Pro Asn Tyr Ser Pro Thr Ser Pro Ser
1880 1885 1890

Gln Asp Thr Asp
1895

<210> 5
<211> 490
<212> DNA
<213> Diabrotica virgifera

<400> 5
ctgaggtcat aattagaggt ggtgaacttc ttgttggggt tctagataaa actcactacg 60

gttctactcc ttatggttta gtacactgta tttatgagtt atatgggggt acctatgcaa 120

tcagattact ttcctcgttg acaaaacttt tcatgagatt tttgcaacaa gaagggttta 180

cacttggagt acatgatata cttacagtag aaagagctga tgttaggaga agggaaatta 240

taaaagactg tagacaagta ggaaaagaag ccgtaactaa agctttagat gtacctttag 300

acactcctga tgctgaagtt gttgaaacaa tagaaaaact aagtgctgct gatcccaaaa 360

ttagagctac aatcgacagg tcctacaagt cttcgatgga tatttttacc aatgaaatta 420

atagaacttg tttgcctgct ggtctggttt gtaaatttcc tgaaaataat cttcaattga 480

tggtacaatc 490

<210> 6
<211> 498
<212> DNA

<213> Diabrotica virgifera

<400> 6
gttataaggt agaaagacac atcagagacg gcgatctagt aatcttcaac cgtcaaccaa 60

ccctccacaa gatgagtatg atgggccaca gagtcaaagt cttaccctgg tcgacgttcc 120

gtatgaatct ctcgtgcacc tctccctaca acgccgattt tgacggcgac gaaatgaacc 180

tccatgtgcc ccaaagtatg gaaactcgag ctgaagtcga aaacctccac atcactccca 240

ggcaaatcat tactccgcaa gctaaccaac ccgtcatggg tattgtacaa gatacgttga 300

cagctgttag gaagatgaca aaaagggatg tattcatcga gaaggaacaa atgatgaata 360

tattgatgtt cttgccaatt tgggatggta aaatgccccg tccagccatc ctcaaaccca 420

aaccgttgtg gacaggaaaa cagatatttt ccctgatcat tcctggcaat gtaaatatga 480

tacgtaccca ttctacgc 498

<210> 7
<211> 114
<212> DNA
<213> Diabrotica virgifera

<400> 7
accctccaca agatgagtat gatgggccac agagtcaaag tcttaccctg gtcgacgttc 60

cgtatgaatc tctcgtgcac ctctccctac aacgccgatt ttgacggcga cgaa 114

<210> 8
<211> 106
<212> DNA
<213> Diabrotica virgifera

<400> 8
atgccccgtc cagccatcct caaacccaaa ccgttgtgga caggaaaaca gatattttcc 60

ctgatcattc ctggcaatgt aaatatgata cgtacccatt ctacgc 106

<210> 9
<211> 24
<212> DNA
<213> Artificial Sequence

<220>
<223> T7 promoter sequence

<400> 9
ttaatacgac tcactatagg gaga 24

<210> 10
<211> 503
<212> DNA
<213> Artificial Sequence

<220>
<223> Partial YFP coding sequence

<400> 10
caccatgggc tccagcggcg ccctgctgtt ccacggcaag atcccctacg tggtggagat 60

ggagggcaat gtggatggcc acaccttcag catccgcggc aagggctacg gcgatgccag 120

cgtgggcaag gtggatgccc agttcatctg caccaccggc gatgtgcccg tgccctggag 180

caccctggtg accaccctga cctacggcgc ccagtgcttc gccaagtacg gccccgagct 240

gaaggatttc tacaagagct gcatgcccga tggctacgtg caggagcgca ccatcacctt 300

cgagggcgat ggcaatttca agacccgcgc cgaggtgacc ttcgagaatg gcagcgtgta 360

caatcgcgtg aagctgaatg gccagggctt caagaaggat ggccacgtgc tgggcaagaa 420

tctggagttc aatttcaccc cccactgcct gtacatctgg ggcgatcagg ccaatcacgg 480

cctgaagagc gccttcaaga tct 503

<210> 11
<211> 48
<212> DNA
<213> Artificial Sequence

<220>
<223> Primer Dvv-Rpi-1-1_For

<400> 11
ttaatacgac tcactatagg gagactgagg tcataattag aggtggtg 48

<210> 12
<211> 53
<212> DNA
<213> Artificial Sequence

<220>
<223> Primer Dvv-Rpi-1-1_Rev

<400> 12
ttaatacgac tcactatagg gagagattgt accatcaatt gaagattatt ttc 53

<210> 13
<211> 49
<212> DNA
<213> Artificial Sequence

<220>
<223> Primer Dvv-Rpi-1-2_For

<400> 13
ttaatacgac tcactatagg gagagttata aggtagaaag acacatcag 49

<210> 14
<211> 44
<212> DNA
<213> Artificial Sequence

<220>

<223> Primer Dvv-RPi-1-2_Rev

<400> 14
ttaatacgac tcactatagg gagagcgtag aatgggtacg tatc 44

<210> 15
<211> 48
<212> DNA
<213> Artificial Sequence

<220>
<223> Primer Dvv-Rpi-1-2 v1_For

<400> 15
ttaatacgac tcactatagg gagaaccctc cacaagatga gtatgatg 48

<210> 16
<211> 44
<212> DNA
<213> Artificial Sequence

<220>
<223> Primer Dvv-Rpi-1-2 v2_Rev

<400> 16
ttaatacgac tcactatagg gagattcgtc gccgtcaaaa tcgg 44

<210> 17
<211> 49
<212> DNA
<213> Artificial Sequence

<220>
<223> Primer Dvv-Rpi-1-2 v2_For

<400> 17
ttaatacgac tcactatagg gagaatgccc cgtccagcca tcctcaaac 49

<210> 18
<211> 44
<212> DNA
<213> Artificial Sequence

<220>
<223> Primer Dvv-Rpi-1-2 v2_Rev

<400> 18
ttaatacgac tcactatagg gagagcgtag aatgggtacg tatc 44

<210> 19
<211> 218
<212> DNA
<213> Diabrotica virgifera

<400> 19
tagctctgat gacagagccc atcgagtttc aagccaaaca gttgcataaa gctatcagcg 60

gattgggaac tgatgaaagt acaatmgtmg aaattttaag tgtmcacaac aacgatgaga 120

ttataagaat ttcccaggcc tatgaaggat tgtaccaacg mtcattggaa tctgatatca 180

aaggagatac ctcaggaaca ttaaaaaaga attattag 218

<210> 20
<211> 424
<212> DNA
<213> Diabrotica virgifera

<220>
<221> misc_feature
<222> (393)..(395)
<223> n is a, c, g, or t

<400> 20
ttgttacaag ctggagaact tctctttgct ggaaccgaag agtcagtatt taatgctgta 60

ttctgtcaaa gaaataaacc acaattgaat ttgatattcg acaaatatga agaaattgtt 120

gggcatccca ttgaaaaagc cattgaaaac gagttttcag gaaatgctaa acaagccatg 180

ttacacctta tccagagcgt aagagatcaa gttgcatatt tggtaaccag gctgcatgat 240

tcaatggcag gcgtcggtac tgacgataga actttaatca gaattgttgt ttcgagatct 300

gaaatcgatc tagaggaaat caaacaatgc tatgaagaaa tctacagtaa aaccttggct 360

gataggatag cggatgacac atctggcgac tannnaaaag ccttattagc cgttgttggt 420

taag 424

<210> 21
<211> 397
<212> DNA
<213> Diabrotica virgifera

<400> 21
agatgttggc tgcatctaga gaattacaca agttcttcca tgattgcaag gatgtactga 60

gcagaatagt ggaaaaacag gtatccatgt ctgatgaatt gggaagggac gcaggagctg 120

tcaatgccct tcaacgcaaa caccagaact tcctccaaga cctacaaaca ctccaatcga 180

acgtccaaca aatccaagaa gaatcagcta aacttcaagc tagctatgcc ggtgatagag 240

ctaaagaaat caccaacagg gagcaggaag tggtagcagc ctgggcagcc ttgcagatcg 300

cttgcgatca gagacacgga aaattgagcg atactggtga tctattcaaa ttctttaact 360

tggtacgaac gttgatgcag tggatggacg aatggac 397

<210> 22
<211> 490
<212> DNA
<213> Diabrotica virgifera

<400> 22
gcagatgaac accagcgaga aaccaagaga tgttagtggt gttgaattgt tgatgaacaa 60

ccatcagaca ctcaaggctg agatcgaagc cagagaagac aactttacgg cttgtatttc 120

tttaggaaag gaattgttga gccgtaatca ctatgctagt gctgatatta aggataaatt 180

ggtcgcgttg acgaatcaaa ggaatgctgt actacagagg tgggaagaaa gatgggagaa 240

cttgcaactc atcctcgagg tataccaatt cgccagagat gcggccgtcg ccgaagcatg 300

gttgatcgca caagaacctt acttgatgag ccaagaacta ggacacacca ttgacgacgt 360

tgaaaacttg ataaagaaac acgaagcgtt cgaaaaatcg gcagcggcgc aagaagagag 420

attcagtgct ttggagagac tgacgacgtt cgaattgaga gaaataaaga ggaaacaaga 480

agctgcccag 490

<210> 23
<211> 330
<212> DNA
<213> Diabrotica virgifera

<400> 23
agtgaaatgt tagcaaatat aacatccaag tttcgtaatt gtacttgctc agttagaaaa 60

tattctgtag tttcactatc ttcaaccgaa aatagaataa atgtagaacc tcgcgaactt 120

gcctttcctc caaaatatca agaacctcga caagtttggt tggagagttt agatacgata 180

gacgacaaaa aattgggtat tcttgagctg catcctgatg tttttgctac taatccaaga 240

atagatatta tacatcaaaa tgttagatgg caaagtttat atagatatgt aagctatgct 300

catacaaagt caagatttga agtgagaggt 330

<210> 24
<211> 320
<212> DNA
<213> Diabrotica virgifera

<400> 24
caaagtcaag atttgaagtg agaggtggag gtcgaaaacc gtggccgcaa aagggattgg 60

gacgtgctcg acatggttca attagaagtc cactttggag aggtggagga gttgttcatg 120

gaccaaaatc tccaacccct catttttaca tgattccatt ctacacccgt ttgctgggtt 180

tgactagcgc actttcagta aaatttgccc aagatgactt gcacgttgtg gatagtctag 240

atctgccaac tgacgaacaa agttatatag aagagctggt caaaagccgc ttttgggggt 300

ccttcttgtt ttatttgtag 320

<210> 25
<211> 47
<212> DNA
<213> Artificial Sequence

<220>
<223> Primer YFP-F_T7

<400> 25
ttaatacgac tcactatagg gagacaccat gggctccagc ggcgccc 47

<210> 26
<211> 23
<212> DNA
<213> Artificial Sequence

<220>
<223> Primer YFP-R

<400> 26
agatcttgaa ggcgctcttc agg 23

<210> 27
<211> 23
<212> DNA
<213> Artificial Sequence

<220>
<223> Primer YFP-F

<400> 27
caccatgggc tccagcggcg ccc 23

<210> 28
<211> 47
<212> DNA
<213> Artificial Sequence

<220>
<223> Primer YFP-R_T7

<400> 28
ttaatacgac tcactatagg gagaagatct tgaaggcgct cttcagg 47

<210> 29
<211> 46
<212> DNA
<213> Artificial Sequence

<220>
<223> Primer Ann-F1_T7

<400> 29
ttaatacgac tcactatagg gagagctcca acagtggttc cttatc 46

<210> 30
<211> 29
<212> DNA
<213> Artificial Sequence

<220>
<223> Primer Ann-R1

<400> 30
ctaataattc ttttttaatg ttcctgagg 29

<210> 31
<211> 22
<212> DNA
<213> Artificial Sequence

<220>
<223> Primer Ann-F1

<400> 31
gctccaacag tggttcctta tc 22

<210> 32
<211> 53
<212> DNA
<213> Artificial Sequence

<220>
<223> Primer Ann-R1_T7

<400> 32
ttaatacgac tcactatagg gagactaata attctttttt aatgttcctg agg 53

<210> 33
<211> 48
<212> DNA
<213> Artificial Sequence

<220>
<223> Primer Ann-F2_T7

<400> 33
ttaatacgac tcactatagg gagattgtta caagctggag aacttctc 48

<210> 34
<211> 24
<212> DNA
<213> Artificial Sequence

<220>
<223> Primer Ann-R2

<400> 34
cttaaccaac aacggctaat aagg 24

<210> 35
<211> 24
<212> DNA
<213> Artificial Sequence

<220>
<223> Primer Ann-F2

<400> 35
ttgttacaag ctggagaact tctc 24

<210> 36

<211> 48
<212> DNA
<213> Artificial Sequence

<220>
<223> Primer Ann-R2T7

<400> 36
ttaatacgac tcactatagg gagacttaac caacaacggc taataagg 48

<210> 37
<211> 47
<212> DNA
<213> Artificial Sequence

<220>
<223> Primer Betasp2-F1_T7

<400> 37
ttaatacgac tcactatagg gagaagatgt tggctgcatc tagagaa 47

<210> 38
<211> 22
<212> DNA
<213> Artificial Sequence

<220>
<223> Primer Betasp2-R1

<400> 38
gtccattcgt ccatccactg ca 22

<210> 39
<211> 23
<212> DNA
<213> Artificial Sequence

<220>
<223> Primer Betasp2-F1

<400> 39
agatgttggc tgcatctaga gaa 23

<210> 40
<211> 46
<212> DNA
<213> Artificial Sequence

<220>
<223> Primer Betasp2-R1_T7

<400> 40
ttaatacgac tcactatagg gagagtccat tcgtccatcc actgca 46

<210> 41
<211> 46
<212> DNA
<213> Artificial Sequence

<220>
<223>  Primer Betasp2-F2_T7

<400>  41
ttaatacgac tcactatagg gagagcagat gaacaccagc gagaaa                    46

<210>  42
<211>  22
<212>  DNA
<213>  Artificial Sequence

<220>
<223>  Primer Betasp2-R2

<400>  42
ctgggcagct tcttgtttcc tc                                              22

<210>  43
<211>  22
<212>  DNA
<213>  Artificial Sequence

<220>
<223>  Primer Betasp2-F2

<400>  43
gcagatgaac accagcgaga aa                                              22

<210>  44
<211>  46
<212>  DNA
<213>  Artificial Sequence

<220>
<223>  Primer Betasp2-R2_T7

<400>  44
ttaatacgac tcactatagg gagactgggc agcttcttgt ttcctc                    46

<210>  45
<211>  51
<212>  DNA
<213>  Artificial Sequence

<220>
<223>  Primer L4-F1_T7

<400>  45
ttaatacgac tcactatagg gagaagtgaa atgttagcaa atataacatc c              51

<210>  46
<211>  26
<212>  DNA
<213>  Artificial Sequence

<220>
<223>  Primer L4-R1

<400> 46
acctctcact tcaaatcttg actttg 26

<210> 47
<211> 27
<212> DNA
<213> Artificial Sequence

<220>
<223> Primer L4-F1

<400> 47
agtgaaatgt tagcaaatat aacatcc 27

<210> 48
<211> 50
<212> DNA
<213> Artificial Sequence

<220>
<223> Primer L4-R1_T7

<400> 48
ttaatacgac tcactatagg gagaacctct cacttcaaat cttgactttg 50

<210> 49
<211> 50
<212> DNA
<213> Artificial Sequence

<220>
<223> Primer L4-F2_T7

<400> 49
ttaatacgac tcactatagg gagacaaagt caagatttga agtgagaggt 50

<210> 50
<211> 25
<212> DNA
<213> Artificial Sequence

<220>
<223> Primer L4-R2

<400> 50
ctacaaataa aacaagaagg acccc 25

<210> 51
<211> 26
<212> DNA
<213> Artificial Sequence

<220>
<223> Primer L4-F2

<400> 51
caaagtcaag atttgaagtg agaggt 26

<210> 52
<211> 49
<212> DNA
<213> Artificial Sequence

<220>
<223> Primer L4-R2_T7

<400> 52
ttaatacgac tcactatagg gagactacaa ataaaacaag aaggacccc 49

<210> 53
<211> 1150
<212> DNA
<213> Zea mays

<400> 53
caacggggca gcactgcact gcactgcaac tgcgaatttc cgtcagcttg gagcggtcca 60

agcgccctgc gaagcaaact acgccgatgg cttcggcggc ggcgtgggag ggtccgacgg 120

ccgcggagct gaagacagcg ggggcggagg tgattcccgg cggcgtgcga gtgaaggggt 180

gggtcatcca gtcccacaaa ggccctatcc tcaacgccgc ctctctgcaa cgctttgaag 240

atgaacttca aacaacacat ttacctgaga tggtttttgg agagagtttc ttgtcacttc 300

aacatacaca aactggcatc aaatttcatt ttaatgcgct tgatgcactc aaggcatgga 360

agaaagaggc actgccacct gttgaggttc ctgctgcagc aaaatggaag ttcagaagta 420

agccttctga ccaggttata cttgactacg actatacatt tacgacacca tattgtggga 480

gtgatgctgt ggttgtgaac tctggcactc cacaaacaag tttagatgga tgcggcactt 540

tgtgttggga ggatactaat gatcggattg acattgttgc cctttcagca aaagaaccca 600

ttcttttcta cgacgaggtt atcttgtatg aagatgagtt agctgacaat ggtatctcat 660

ttcttactgt gcgagtgagg gtaatgccaa ctggttggtt tctgcttttg cgtttttggc 720

ttagagttga tggtgtactg atgaggttga gagacactcg gttacattgc ctgtttggaa 780

acggcgacgg agccaagcca gtggtacttc gtgagtgctg ctggagggaa gcaacatttg 840

ctactttgtc tgcgaaagga tatccttcgg actctgcagc gtacgcggac ccgaacctta 900

ttgcccataa gcttcctatt gtgacgcaga agacccaaaa gctgaaaaat cctacctgac 960

tgacacaaag gcgccctacc gcgtgtacat catgactgtc ctgtcctatc gttgcctttt 1020

gtgtttgcca catgttgtgg atgtacgttt ctatgacgaa acaccatagt ccatttcgcc 1080

tgggccgaac agagatagct gattgtcatg tcacgtttga attagaccat tccttagccc 1140

tttttccccc 1150

<210> 54
<211> 22

<212> DNA
<213> Artificial Sequence

<220>
<223> Oligonucleotide T20VN

<220>
<221> misc_feature
<222> (22)..(22)
<223> n is a, c, g, or t

<400> 54
tttttttttt tttttttttt vn 22

<210> 55
<211> 23
<212> DNA
<213> Artificial Sequence

<220>
<223> Primer RPI-2v1FWDSet1

<400> 55
cctccacaag atgagtatga tgg 23

<210> 56
<211> 22
<212> DNA
<213> Artificial Sequence

<220>
<223> Primer RPI-2v1REVSet1

<400> 56
gaggtgcacg agagattcat ac 22

<210> 57
<211> 21
<212> DNA
<213> Artificial Sequence

<220>
<223> Primer TIPmxF

<400> 57
tgagggtaat gccaactggt t 21

<210> 58
<211> 24
<212> DNA
<213> Artificial Sequence

<220>
<223> Primer TIPmxR

<400> 58
gcaatgtaac cgagtgtctc tcaa 24

<210> 59
<211> 32
<212> DNA
<213> Artificial Sequence

<220>
<223> Probe HXTIP

<400> 59
tttttggctt agagttgatg gtgtactgat ga 32

<210> 60
<211> 151
<212> DNA
<213> Escherichia coli

<400> 60
gaccgtaagg cttgatgaaa caacgcggcg agctttgatc aacgaccttt tggaaacttc 60

ggcttcccct ggagagagcg agattctccg cgctgtagaa gtcaccattg ttgtgcacga 120

cgacatcatt ccgtggcgtt atccagctaa g 151

<210> 61
<211> 69
<212> DNA
<213> Artificial Sequence

<220>
<223> Partial AAD1 coding region

<400> 61
tgttcggttc cctctaccaa gcacagaacc gtcgcttcag caacacctca gtcaaggtga 60

tggatgttg 69

<210> 62
<211> 4233
<212> DNA
<213> Zea mays

<400> 62
agcctggtgt ttccggagga gacagacatg atccctgccg ttgctgatcc gacgacgctg 60

gacggcgggg gcgcgcgcag gccgttgctc ccggagacgg accctcgggg gcgtgctgcc 120

gccggcgccg agcagaagcg gccgccggct acgccgaccg ttctcaccgc cgtcgtctcc 180

gccgtgctcc tgctcgtcct cgtggcggtc acagtcctcg cgtcgcagca cgtcgacggg 240

caggctgggg gcgttcccgc gggcgaagat gccgtcgtcg tcgaggtggc cgcctcccgt 300

ggcgtggctg agggcgtgtc ggagaagtcc acggccccgc tcctcggctc cggcgcgctc 360

caggacttct cctggaccaa cgcgatgctg gcgtggcagc gcacggcgtt ccacttccag 420

ccccccaaga actggatgaa cggttagttg gacccgtcgc catcggtgac gacgcgcgga 480

tcgttttttt ctttttttcct ctcgttctgg ctctaacttg gttccgcgtt tctgtcacgg 540

acgcctcgtg cacatggcga tacccgatcc gccggccgcg tatatctatc tacctcgacc 600
ggcttctcca gatccgaacg gtaagttgtt ggctccgata cgatcgatca catgtgagct 660
cggcatgctg cttttctgcg cgtgcatgcg gctcctagca ttccacgtcc acgggtcgtg 720
acatcaatgc acgatataat cgtatcggta cagagatatt gtcccatcag ctgctagctt 780
tcgcgtattg atgtcgtgac attttgcacg caggtccgct gtatcacaag ggctggtacc 840
acctcttcta ccagtggaac ccggactccg cggtatgggg caacatcacc tggggccacg 900
ccgtctcgcg cgacctcctc cactggctgc acctaccgct ggccatggtg cccgatcacc 960
cgtacgacgc caacggcgtc tggtccgggt cggcgacgcg cctgcccgac ggccggatcg 1020
tcatgctcta cacgggctcc acggcggagt cgtcggcgca ggtgcagaac ctcgcggagc 1080
cggccgacgc gtccgacccg ctgctgcggg agtgggtcaa gtcggacgcc aacccggtgc 1140
tggtgccgcc gccgggcatc gggccgacgg acttccgcga cccgacgacg gcgtgtcgga 1200
cgccggccgg caacgacacg gcgtggcggg tcgccatcgg gtccaaggac cgggaccacg 1260
cggggctggc gctggtgtac cggacggagg acttcgtgcg gtacgacccg gcgccggcgc 1320
tgatgcacgc cgtgccgggc accggcatgt gggagtgcgt ggacttctac ccggtggccg 1380
cgggatcagg cgccgcggcg ggcagcgggg acgggctgga gacgtccgcg gcgccgggac 1440
ccggggtgaa gcacgtgctc aaggctagcc tcgacgacga caagcacgac tactacgcga 1500
tcggcaccta cgacccggcg acggacacct ggacccccga cagcgcggag gacgacgtcg 1560
ggatcggcct ccggtacgac tatggcaagt actacgcgtc gaagaccttc tacgaccccg 1620
tccttcgccg gcgggtgctc tgggggtggg tcggcgagac cgacagcgag cgcgcggaca 1680
tcctcaaggg ctgggcatcc gtgcaggtac gtctcagggt ttgaggctag catggcttca 1740
atcttgctgg catcgaatca ttaatgggca gatattataa cttgataatc tgggttggtt 1800
gtgtgtggtg gggatggtga cacacgcgcg gtaataatgt agctaagctg gttaaggatg 1860
agtaatgggg ttgcgtataa acgacagctc tgctaccatt acttctgaca cccgattgaa 1920
ggagacaaca gtaggggtag ccggtagggt tcgtcgactt gccttttctt ttttcctttg 1980
ttttgttgtg gatcgtccaa cacaaggaaa ataggatcat ccaacaaaca tggaagtaat 2040
cccgtaaaac atttctcaag gaaccatcta gctagacgag cgtggcatga tccatgcatg 2100
cacaaacact agataggtct ctgcagctgt gatgttcctt tacatatacc accgtccaaa 2160
ctgaatccgg tctgaaaatt gttcaagcag agaggccccg atcctcacac ctgtacacgt 2220
ccctgtacgc gccgtcgtgg tctcccgtga tcctgccccg tcccctccac gcggccacgc 2280
ctgctgcagc gctctgtaca agcgtgcacc acgtgagaat ttccgtctac tcgagcctag 2340
tagttagacg ggaaaacgag aggaagcgca cggtccaagc acaacacttt gcgcgggccc 2400

gtgacttgtc tccggttggc tgagggcgcg cgacagagat gtatggcgcc gcggcgtgtc 2460

ttgtgtcttg tcttgcctat acaccgtagt cagagactgt gtcaaagccg tccaacgaca 2520

atgagctagg aaacgggttg gagagctggg ttcttgcctt gcctcctgtg atgtctttgc 2580

cttgcatagg gggcgcagta tgtagctttg cgttttactt cacgccaaag gatactgctg 2640

atcgtgaatt attattatta tatatatatc gaatatcgat ttcgtcgctc tcgtggggtt 2700

ttattttcca gactcaaact tttcaaaagg cctgtgtttt agttcttttc ttccaattga 2760

gtaggcaagg cgtgtgagtg tgaccaacgc atgcatggat atcgtggtag actggtagag 2820

ctgtcgttac cagcgcgatg cttgtatatg tttgcagtat tttcaaatga atgtctcagc 2880

tagcgtacag ttgaccaagt cgacgtggag ggcgcacaac agacctctga cattattcac 2940

ttttttttta ccatgccgtg cacgtgcagt caatccccag gacggtcctc ctggacacga 3000

agacgggcag caacctgctc cagtggccgg tggtggaggt ggagaacctc cggatgagcg 3060

gcaagagctt cgacggcgtc gcgctggacc gcggatccgt cgtgcccctc gacgtcggca 3120

aggcgacgca ggtgacgccg cacgcagcct gctgcagcga acgaactcgc gcgttgccgg 3180

cccgcggcca gctgacttag tttctctggc tgatcgaccg tgtgcctgcg tgcgtgcagt 3240

tggacatcga ggctgtgttc gaggtggacg cgtcggacgc ggcgggcgtc acggaggccg 3300

acgtgacgtt caactgcagc accagcgcag gcgcggcggg ccgggggcctg ctcggcccgt 3360

tcggccttct cgtgctggcg gacgacgact tgtccgagca gaccgccgtg tacttctacc 3420

tgctcaaggg cacggacggc agcctccaaa ctttcttctg ccaagacgag ctcaggtatg 3480

tatgttatga cttatgacca tgcatgcatg cgcatttctt agctaggctg tgaagcttct 3540

tgttgagttg tttcacagat gcttaccgtc tgctttgttt cgtatttcga ctaggcatcc 3600

aaggcgaacg atctggttaa gagagtatac gggagcttgg tccctgtgct agatggggag 3660

aatctctcgg tcagaatact ggtaagtttt tacagcgcca gccatgcatg tgttggccag 3720

ccagctgctg gtactttgga cactcgttct tctcgcactg ctcattattg cttctgatct 3780

ggatgcacta caaattgaag gttgaccact ccatcgtgga gagctttgct caaggcggga 3840

ggacgtgcat cacgtcgcga gtgtacccca cacgagccat ctacgactcc gcccgcgtct 3900

tcctcttcaa caacgccaca catgctcacg tcaaagcaaa atccgtcaag atctggcagc 3960

tcaactccgc ctacatccgg ccatatccgg caacgacgac ttctctatga ctaaattaag 4020

tgacggacag ataggcgata ttgcatactt gcatcatgaa ctcatttgta caacagtgat 4080

tgtttaattt atttgctgcc ttccttatcc ttcttgtgaa actatatggt acacacatgt 4140

atcattaggt ctagtagtgt tgttgcaaag acacttagac accagaggtt ccaggagtat 4200

cagagataag gtataagagg gagcagggag cag 4233

<210> 63
<211> 20
<212> DNA
<213> Artificial Sequence

<220>
<223> Primer GAAD1-F

<400> 63
tgttcggttc cctctaccaa 20

<210> 64
<211> 22
<212> DNA
<213> Artificial Sequence

<220>
<223> Primer GAAD1-R

<400> 64
caacatccat caccttgact ga 22

<210> 65
<211> 24
<212> DNA
<213> Artificial Sequence

<220>
<223> Probe GAAD1-P (FAM)

<400> 65
cacagaaccg tcgcttcagc aaca 24

<210> 66
<211> 18
<212> DNA
<213> Artificial Sequence

<220>
<223> Primer IVR1-F

<400> 66
tggcggacga cgacttgt 18

<210> 67
<211> 19
<212> DNA
<213> Artificial Sequence

<220>
<223> Primer IVR1-R

<400> 67
aaagtttgga ggctgccgt 19

<210> 68
<211> 26
<212> DNA

<213>  Artificial Sequence

<220>
<223>  Probe IVR1-P (HEX)

<400>  68
cgagcagacc gccgtgtact tctacc                                          26

<210>  69
<211>  19
<212>  DNA
<213>  Artificial Sequence

<220>
<223>  Primer SPC1A

<400>  69
cttagctgga taacgccac                                                  19

<210>  70
<211>  19
<212>  DNA
<213>  Artificial Sequence

<220>
<223>  Primer SPC1S

<400>  70
gaccgtaagg cttgatgaa                                                  19

<210>  71
<211>  21
<212>  DNA
<213>  Artificial Sequence

<220>
<223>  Probe TQSPEC (CY5)

<400>  71
cgagattctc cgcgctgtag a                                               21

<210>  72
<211>  20
<212>  DNA
<213>  Artificial Sequence

<220>
<223>  Primer LoopF

<400>  72
ggaacgagct gcttgcgtat                                                 20

<210>  73
<211>  20
<212>  DNA
<213>  Artificial Sequence

<220>

<223> Primer LoopR

<400> 73
cacggtgcag ctgattgatg 20

<210> 74
<211> 18
<212> DNA
<213> Artificial Sequence

<220>
<223> Probe Loop FAM

<400> 74
tcccttccgt agtcagag 18

<210> 75
<211> 5053
<212> RNA
<213> Diabrotica virgifera

<400> 75
uucaaaugaa gagaagaaga agaagaaucu ucaaagcaug uuuuuguuag guuaaauuuc 60

aaauuuuuua auuugauguu uguuauugug uuuaaaaaca agauuauuaa auaaacggaa 120

acaugaagau ucacuuuaua cccgauaaag ugagcuuuuc acuuuuuacc acagaagaaa 180

ucaagaaaau guguguuacu caaauuauua ccccacuuau guuagauccc uuaggucauc 240

cucuacaagg agguuuauau gaucguaaau uaggaccaua cucagccaaa gauacucuau 300

gugaaucuug uaaccaaaca uucaccaauu guccuggcca uuuuggauac auugaacuuc 360

cuuuaccagu aguaaauccu cuguuucaua aaauuauugg aaccauuaua aaaauaucau 420

guuugucaug uuuccacaua caacuaccaa cucauauaaa aaaaguuuua uguauucaaa 480

ugaaacuuuu aaauugugga cugauuacag aagccuuauc aguagaaaca gcauuagcgg 540

aauuaauuuc gaaauaugaa aaguuugaga auauuccaac ugaaaguaua aaaagugua u 600

uacgauauga ggaauuagcu gaugaaacau uaaagaaucu agaagguaaa aauguaacca 660

gccaaaacac cgaaacacuc cgcaacaacu uugugucuaa aauguugaaa gagguaaaau 720

cuagacaacu auguauauuu uguaaaaaca gaauuaauag gauucaggca cuaaagaaca 780

gaauuauuuu aacaaaaaag aaaggugauc uagaugauuc uaacguggu u augggacaaa 840

aagugacagg uauggagucu caauacauua cgccggaaga gucaaggcaa uauuuaagaa 900

auauauggag acaagaaaaa gaauuucuuc aacaacuugu aucuguacuc gcaaaugucg 960

acugugaaca cccaacugau gcauuuuauu uugaagugau uccuguucca ccaccaaaug 1020

uuagaccugu uaacuucgug aaugguagaa uauuagagaa uaaacaaucg auugguuaca 1080

aaaauaucau acaaaauguc auucuuuuaa aaacuauaau acaaguagua caaaguaaga 1140

gcgauauuaa uagcuuguca ucuguggaag cuaagagugc cuauagcauu gccagaggaa 1200

auucuccugu agaaaaauua aauuauugcu gggaagaauu gcagagugac guaaacgggu 1260
uacuugauaa ugaaaauguu cgacaagagg gacagggauu gaaacagauc auugaaaaga 1320
aagaaggugu uauacguaug uguaugaugg guaaacgugu uaauuuuucc gcuagaucag 1380
uuauaacucc ugaccccaac cuuaauaucg acgaaaucgg aaucccggaa gaauuugcaa 1440
agaagcugac uuauccagua gcuguaacuc cuuggaaugu ugaagaacua aggaaaauga 1500
uuuuaaaugg accuaauguu cacccagggg caauuaugau agaaaacaau gggauucuaa 1560
aacgaauuaa ucccuauaau gaaguucaac aaaaaagcau auugaaaugu uuguuaacuc 1620
cugaagccac uaaaggacca aaggacaag gaauucaaau cgugcauagg cauuuaugca 1680
acggugacgu ucuguuacuu aaucgccaac caacuuugca caaaccuagu auaauggcgc 1740
acacagcgcg aauuuuaaag ggagaaaaaa cucuucgauu gcauuaugcu aauuguaaag 1800
ccuauaaugc ugauuuugac ggggaugaaa ugaacgcaca uuacccccaa aaugaacuug 1860
caagaaguga aggcuauaau auuguuaacg uuucuaacca auaucucguu ccaaaagaug 1920
gcacuccucu cagugguuua auucaggacc acaugauuuc uggugugcga cugucuuuaa 1980
gaggaagauu uuuugauaaa caagauuaug agcaucuagu uuaccaagcu cucucguuca 2040
aaacaggucg cauaaagcuc uuaccaccaa caauuauaaa accacaagug uuauggucug 2100
ggaaacaaau uuuaucuaca guuauaauua acgucauacc cgacgaaaga gaauuaauca 2160
aucuuacguc aacagcuaag auuucuucca aagcuuggca gagaagaccc ucgagaagau 2220
ggagagcagg ugguacuaua uuucuugaug acaaggucau gagugaagcu gaggucauaa 2280
uuagaggugg ugaacuucuu guuggggguuc uagauaaaac ucacuacggu ucuacuccuu 2340
augguuuagu acacuguauu uaugaguuau augggguac cuaugcaauc agauuacuuu 2400
ccucguugac aaaacuuuuc augagauuuu ugcaacaaga aggguuuaca cuuggaguac 2460
augauauacu uacaguagaa agagcugaug uuaggagaag ggaaauuaua aaagacugua 2520
gacaaguagg aaaagaagcc guaacuaaag cuuuagaugu accuuuagac acuccugaug 2580
cugaaguugu ugaaacaaua gaaaaacuaa gugcugcuga ucccaaaauu agagcuacaa 2640
ucgacagguc cuacaagucu ucgauggaua uuuuuaccaa ugaaauuaau agaacuuguu 2700
ugccugcugg ucugguuugu aaauuuccug aaaauaaucu ucaauugaug guacaaucug 2760
gagcgaaagg uucaacagua aauacuaugc aaauuuccug ucuucuuggu caaauagaau 2820
uggaaggaaa acggccaccu guaaugauau ccggaaaauc ucuaccuagu uuuccaucau 2880
ucgaguuuac uccaagggcg ggaggauuua ucgauggacg auucaugacu gguauccaac 2940
cgcaagaauu cuucuuccau uguauggcag gacgugaagg ucuuauugau acagcuguua 3000
aaacuagucg uaguggauau uuacaaagau gucucaucaa acauuuggaa ggucuacgug 3060

uugguuauga uaugaccgug agaaacagug auaaaagugu aauacaguuu uuguauggag 3120

aggauggaau ggauauuuca aaagcucagu uuuucaauga aaaacagaug agcuucuugg 3180

ccgaaaauau caagguguug gguaauucug auacgcuuaa acaguugaag aaugaagaag 3240

aucaagaggc uguaaaagaa cauggaaaag cgguaaaaga auggaagaaa caucauggga 3300

auccauuaaa ucacagaagg aauaguccau uuucccuguu ugcuaaauau guucaaaaua 3360

ggacuggaga uaacaaccuu uuaaccaagg agaaguuaau gaaacuuugg uaugaaaugg 3420

acaaggacau aaaaacaaac uucaccgacc agugcgagaa augcccagau cccauagaag 3480

ccguguauca accugaugca aauuuuggag cgauaaauga aacagugcag aaacuuauca 3540

agaacuauaa aggauucgac aauaagaaaa guaagaaaaa auuugaagau gucauaaaau 3600

ugaaaguaau ggaaucgaug uguucugcug gagaaccagu uggacuucuu gcggcacaau 3660

caauaggaga accaucuacc cagaugacac ucaauacuuu ccauuuugcu ggaagaggug 3720

aaaugaacgu uacucucggu auuccccguu uaagagaaau cuuaaugaug gcuucaaaga 3780

auauaaaaac gccaucgaug gagauuccau ucuugcaggu uccagauuua gaauauaagg 3840

cgaacgaguu aaggaaacuu cugacucgag uggugguagc cgacguuuua gaaacuauug 3900

auguuacugu ugaacuucaa uuuaaaccca uuaggcaaua uaaguauacc uugaaauucc 3960

aauuuuuacc gaagaaauau uacaguaugg auuauugugu gaaucccaca aaaauacuaa 4020

ggcauaugaa ggggaaauau uuuggugaaa uguuugcguc caucaagaaa gucaguaaaa 4080

uuaauucaaa cauaguaaug auggaggaag aaagaaacaa gaaacguaca acuaauaacg 4140

aagaagauga agaucgacca gaaacaaaug aaagagaagg ugacaaucaa auugauuccu 4200

cagaugacga aguggaagau aaugaagaug cuaagcagag ucauaaguac caagaaacaa 4260

gggaugauuu agaaccagaa gaagaagaga aagaaaaauc ugacgaugag gaugaugaaa 4320

gcgauaacga aacccaagcg aaccaaaaag aaacugacaa ucaagaacaa gauaaugaag 4380

uaguugauag uuacaauuuu gcacaaaguu auuaugaaga ccaacaaaaa caauuguggu 4440

gugaaauaac auuugguuug cccuugucgu ucaaaaaauu ggaucuuacu gcaauuuuaa 4500

aggagacugc cggcaaaucu guucuuuggg aaacgcccca aauuaaaaga gccauuacuu 4560

augugaagga ugauaaauua augcuuagaa cggaugguau uaauauuguu gaaauguuua 4620

aauacaauac ccuuuuagac uugccacaac uuuauuguaa ugauauccau aaaguggcag 4680

aaacauacgg cauugaagca gcaucuaaag uaauaguaaa ggagguuaaa gacguauuua 4740

auguguacgg aauuaaagua gauccucguc auuugucccu aguagccgac uacaugacau 4800

uuaaugguac auuugaacca cucagcagaa gaggaaugga aaacagcgcu uccccucugc 4860

aacagauguc auuugaauca ucuuuaguau uuuuaaggaa ugcagcaauu agaggccgag 4920

aagaugauuu acaaaacccu ucgaguaguc uuauguuagg aaaaccaugu ggaaccggca 4980

caggaagcuu uacccuuuua cauaaguccu uuguaacaug uuaauaaaua aauuguuaua 5040

gauaaaaaaa aaa 5053

<210> 76
<211> 6927
<212> RNA
<213> Diabrotica virgifera

<400> 76
ugcucgaccu guagauucuu guaacggauu ucggagaguu cgauucguug ucgagccuuc 60

aaaauggcua ccaacgauag uaaagcuccg uugaggacag uuaaaagagu gcaauuugga 120

auacuuaguc cagaugaaau uagacgaaug ucagucacag aagggggcau ccgcuuccca 180

gaaaccaugg aagcaggccg ccccaaacua ugcggucuua uggaccccag acaaggugac 240

auagacagaa gcucaagaug ccagacaugu gccggaaaua ugacagaaug uccuggacau 300

uucggacaua ucgagcuggc aaaaccaguu uuccacguag gauucguaac aaaaacaaua 360

aagaucuuga gaugcguuug cuucuuuugc aguaaauuau uagucagucc aaauaauccg 420

aaaauuaaag aaguuguaau gaaaucaaag ggacagccac guaaaagauu agcuuucguu 480

uaugaucugu guaaagguaa aaauauuugu gaaggguggag augaaaugga uguggguaaa 540

gaaagcgaag aucccaauaa aaaagcaggc cauggugguu guggucgaua ucaaccaaau 600

aucagacgug ccgguuuaga uuuaacagca gaauggaaac acgucaauga agacacacaa 660

gaaaagaaaa ucgcacuauc ugccgaacgu gucugggaaa uccuaaaaca uaucacagau 720

gaagaauguu ucauucuugg uauggauccc aaauuugcua gaccagauug gaugauagua 780

acgguacuuc cuguuccucc ccuagcagua cgaccugcug uaguuaugca cggaucugca 840

aggaaucagg augauaucac ucacaaauug gccgacauua ucaaggcgaa uaacgaauua 900

cagaagaacg agucugcagg ugcagccgcu cauauaauca cagaaaauau uaagauguug 960

caauuucacg ucgccacuuu aguugacaac gauaugccgg gaaugccgag agcaaugcaa 1020

aaaucuggaa aaccccuaaa agcuaucaaa gcucggcuga aagguaaaga aggaaggauu 1080

cgagguaacc uuaugggaaa gcguguggac uuuucugcac guacugucau cacaccagau 1140

cccaauuuac guaucgacca aguaggagug ccuagaagua uugcucaaaa caugacguuu 1200

ccagaaaucg ucacaccuuu caauuuugac aaaauguugg aauugguaca gagagguaau 1260

ucucaguauc caggagcuaa guauaucauc agagacaaug gagagaggau ugauuuacgu 1320

uuccacccaa aaccgucaga uuuacauuug caguguggdu auaagguaga aagacacauc 1380

agagacggcg aucuaguaau cuucaaccgu caaccaaccc uccacaagau gaguaugaug 1440

ggccacagag ucaaagucuu acccuggucg acguuccgua ugaaucucuc gugcaccucu 1500

cccuacaacg ccgauuuuga cggcgacgaa augaaccucc augugcccca aaguauggaa 1560

acucgagcug aagucgaaaa ccuccacauc acucccaggc aaaucauuac uccgcaagcu 1620
aaccaacccg ucauggguau uguacaagau acguugacag cuguuaggaa gaugacaaaa 1680
agggauguau ucaucgagaa ggaacaaaug augaauauau ugauguucuu gccaauuugg 1740
gaugguaaaa ugccccgucc agccauccuc aaacccaaac cguuguggac aggaaaacag 1800
auauuuuccc ugaucauucc uggcaaugua aauaugauac guacccauuc uacgcaucca 1860
gacgacgagg acgacggucc cuauaaaugg auaucgccag gagauacgaa aguuauggua 1920
gaacauggag aauuggucau ggguauauug uguaagaaaa gucuuggaac aucagcaggu 1980
ucccugcugc auauuuguau guuggaauua ggacacgaag ugugugguag auuuuauggu 2040
aacauucaaa cuguaaucaa caacugguug uuguuagaag gucacagcau cgguauugga 2100
gacaccauug ccgauccuca gacuuacaca gaaauucaga gagccaucag gaaagccaaa 2160
gaagauguaa uagaagucau ccagaaagcu cacaacaugg aacuggaacc gacucccggu 2220
aauacguugc gucagacuuu cgaaaaucaa guaaacagaa uucuaaacga cgcucgugac 2280
aaaacuggug guuccgcuaa gaaaucuuug acugaauaca auaaccuaaa ggcuaugguc 2340
guaucgggau ccaagggauc caacauuaau auuucccagg uuauugcuug cgugggucaa 2400
cagaacguag aagguaaacg uauuccauuu ggcuucagaa aacgcacguu gccgcacuuc 2460
aucaaggacg auuacggucc ugaauccaga gguuucguag aaaauucgua ucuugccggu 2520
cucacuccuu cggaguucua uuuccacgcu augggagguc gugaaggucu uaucgauacu 2580
gcuguaaaaa cugccgaaac ugguuacauc caacgucguc ugauaaaggc uauggagagu 2640
guaaugguac acuacgacgg uaccguaaga aauucuguag gacaacuuau ccagcugaga 2700
uacggugaag acggacucug uggagagaug guagaguuuc aauauuuagc aacagucaaa 2760
uuaaguaaca aggcguuuga gagaaaauuc agauuugauc caaguaauga aagguauuug 2820
agaagaguuu ucaaugaaga aguuaucaag caacugaugg guucagggga agucauuucc 2880
gaacuugaga gagaauggga acaacuccag aaagacagag aagccuuaag acaaaucuuc 2940
ccuagcggag aaucuaaagu aguacucccc uguaacuuac aacguaugau cuggaaugua 3000
caaaaaauuu uccacauaaa caaacgagcc ccgacagacc uguccccguu aagaguuauc 3060
caaggcguuc gagaauuacu caggaaaugc gucaucguag cuggcgagga ucgucugucc 3120
aaacaagcca acgaaaacgc aacguuacuc uuccaguguc uagucagauc gacccucugc 3180
accaaaugcg uuucugaaga auucaggcuc agcaccgaag ccuucgagug guugauagga 3240
gaaaucgaga cgagguucca acaagcccaa gccaauccug gagaaauggu gggcgcucug 3300
gccgcgcagu cacugggaga acccgcuacu cagaugacac ugaacacuuu ccauuuugcu 3360
gguguauccu ccaagaacgu aacccugggu guaccuagau uaaaggaaau uauuaauauu 3420

uccaagaaac ccaaggcucc aucucuaacc guguuuuuaa cuggugcggc ugcuagagau 3480
gcggaaaaag cgaagaaugu guuaugcaga cuugaacaca ccacucuucg uaaaguaacc 3540
gccaacaccg ccaucuauua cgauccugac ccacaaaaua ccgucauucc ugaggaucag 3600
gaguucguua acgucuacua ugaaaugccc gauuucgauc cuacccguau aucgccgugg 3660
uugcuucgua ucgaacugga cagaaagaga augacagaua agaaacuaac uauggaacaa 3720
auugcugaaa agaucaacgc ugggguucggg gacgauuuga auuguauuuu caacgacgac 3780
aaugcugaaa aguuggugcu gcguaucaga aucaugaaca gcgacgaugg aaaauucgga 3840
gaaggugcug augaggacgu agacaaaaug gaugacgaca uguuuuugag augcaucgaa 3900
gcgaacaugc ugagcgauau gaccuugcaa gguauagaag cgauuuccaa gguauacaug 3960
cacuugccac agacugacuc gaaaaaaagg aucgucauca cugaaacagg cgaauuuaag 4020
gccaucgcag aauggcuauu ggaaacugac gguaccagca ugaugaaagu acugucagaa 4080
agagacgucg auccggucag gacguuuucu aacgacauuu gugaaauauu uucgguacuu 4140
gguaucgagg cugugcguaa gucuguagag aaagaaauga acgcuguccu uucauucuac 4200
ggucuguacg uaaacuaucg ccaucuugcc uugcuuugug acguaaugac agccaaaggu 4260
cacuuaaugg ccaucacccg ucacgguauc aacagacaag acacuggagc ucugaugagg 4320
uguuccuucg aggaaacugu agauguauug auggacgcug ccagucaugc ggaggucgac 4380
ccaaugagag gaguaucuga aaacauuauc cucggucaac uaccaagaau gggcacaggc 4440
ugcuucgauc uuuugcugga cgccgaaaaa uguaaaaugg gaauugccau accucaagcg 4500
cacagcagcg aucuaauggc uucaggaaug uucuuuggau uagccgcuac acccagcagu 4560
augaguccag gugguggcuau gaccccaugg aaucaagcag cuacaccaua cguuggcagu 4620
aucuggucuc cacagaauuu aaugggcagu ggaaugacac cagguggugc cgcuuucucc 4680
ccaucagcug cgucagaugc aucaggaaug ucaccagcuu auggcgguug gucaccaaca 4740
ccacaaucuc cugcaauguc gccauauaug gcuucuccac auggacaauc gccuuccuac 4800
aguccaucaa guccagcguu ccaaccuacu ucaccaucca ugacgccgac cucuccugga 4860
uauucuccca guucuccugg uuauucaccu accagucuca auuacagucc aacgaguccc 4920
aguuauucac ccacuucuca gaguuacucc ccaaccucac cuaguuacuc accgacuucu 4980
ccaaauuauu caccuacuuc cccaagcuac aguccaacau ccccuaacua uucaccaaca 5040
ucucccaacu auucacccac uucaccuagu uauccuucaa cuucgccagg uuacagcccc 5100
acuucacgca gcuacucacc cacaucuccu aguuacucag gaacuucgcc cucuuauuca 5160
ccaacuucgc caaguuacuc cccuacuucu ccuaguuauu cgccgucguc uccuaauuac 5220
ucucccacuu cuccaaauua caguccccacu ucuccuaauu acucaccguc cucuccuagg 5280
uacacgcccg guucuccuag uuuuucccca aguucgaaca guuacucucc cacaucuccu 5340

caauauucuc caacaucucc aaguuauucg ccuucuucgc ccaaauauuc accaacuucc 5400

cccaauuauu cgccaacauc uccaucauuu ucuggaggaa guccacaaua uucacccaca 5460

ucaccgaaau acucuccaac cucgcccaau uacacucugu cgaguccgca gcacacucca 5520

acagguagca gucgauauuc accgucaacu ucgaguuauu cuccuaauuc gcccaauuau 5580

ucaccgacgu cuccacaaua cuccauccac aguacaaaau auuccccugc aaguccuaca 5640

uucacaccca ccaguccuag uuucucuccc gcuucacccg cauauucgcc ucaaccuaug 5700

uauucaccuu cuucuccuaa uuauucuccc acuaguccca gucaagacac ugacuaaaua 5760

uaaucauaag auuguagugg uuaguuguau uuuauacaua gauuuuaauu cagaauuuaa 5820

uauuauuuuu uacuauuuac cagggacauu uuuaaaguug uaaaaacacu uacauuuguu 5880

ccaacggauu uuugcacaaa cguaacgaag uuaaaucaaa acauuacaac ugaaacauac 5940

gucgguaugu acugucaaug ugaucauuag gaaauggcua uuaucccgga ggacguauuu 6000

uauaaaguua uuuuauugaa guguuugauc uuuuuucacu auugaggaga uuuauggacu 6060

caacauuaaa cagcuugaac aucauaccga cuacuacuaa uauaaagaua aauauagaac 6120

gguaagaaau agauuaaaaa aaaauacaau aaguuaaaca guaaucauaa aaauaaauac 6180

guuuccguuc gacagaacua uagccagauu cuuguaguau aaugaaaauu uguagguuaa 6240

aaauauuacu ugucacauua gcuuaaaaau aaaaaauuac cggaaguaau caaauaagag 6300

agcaacaguu agucguucua acaauuaugu uugaaaauaa aaauuacaau gaguuauaca 6360

aacgaagacu acaaguuuaa auaguaugaa aaacuauuug uaaacacaac aaaugcgcau 6420

ugaaauuuau uuaucguacu uaacuuauuu gccuuacaaa aauaauacuc cgcgaguauu 6480

uuuuaugaac uguaaaacua aaaaguugua caguucacac aaaaacaucg aaaaauuuug 6540

uuuuuguaug uuucuauuau uaaaaaaaua cuuuuuaucu uucaccuuau agguacuauu 6600

ugacucuaug acauuuucuc uacauuucuu uaaaucuguu cuauuuauua uguacaugaa 6660

ucuauaagca caaauaauau acauaaucau uuugauaaaa aaucauaguu uuaaauaaaa 6720

cagauuucaa cacaauauuc auaagucuac uuuuuuaaaa auuuauagag acaaaggcca 6780

uuuuucagaa acagauuaaa caaaaaucac uauaaauuau uuugaguaug uugaauaagu 6840

uuauauugcu ucuacaauuu uuaaauauaa aauuauaaca uuagcagagg aacaacgaga 6900

auuaaggucg ggaagaucau gcaccga 6927

<210> 77
<211> 490
<212> RNA
<213> Diabrotica virgifera

<400> 77
cugaggucau aauuagaggu ggugaacuuc uuguuggggu ucuagauaaa acucacuacg 60

guucuacucc uuaugguuua guacacugua uuuaugaguu auaugggggu accuaugcaa 120

ucagauuacu uuccucguug acaaaacuuu ucaugagauu uuugcaacaa gaaggguuua 180

cacuuggagu acaugauaua cuuacaguag aaagagcuga uguuaggaga agggaaauua 240

uaaaagacug uagacaagua ggaaaagaag ccguaacuaa agcuuuagau guaccuuuag 300

acacuccuga ugcugaaguu guugaaacaa uagaaaaacu aagugcugcu gaucccaaaa 360

uuagagcuac aaucgacagg uccuacaagu cuucgaugga uauuuuuacc aaugaaauua 420

auagaacuug uuugccugcu ggucugguuu guaaauuucc ugaaaauaau cuucaauuga 480

ugguacaauc 490

<210> 78
<211> 498
<212> RNA
<213> Diabrotica virgifera

<400> 78
guuauaaggu agaaagacac aucagagacg gcgaucuagu aaucuucaac cgucaaccaa 60

cccuccacaa gaugaguaug augggccaca gagucaaagu cuuacccugg ucgacguucc 120

guaugaaucu cucgugcacc ucucccuaca acgccgauuu ugacggcgac gaaaugaacc 180

uccaugugcc ccaaaguaug gaaacucgag cugaagucga aaccuccacac aucacuccca 240

ggcaaaucau uacuccgcaa gcuaaccaac ccgucauggg uauuguacaa gauacguuga 300

cagcuguuag gaagaugaca aaaagggaug uauucaucga gaaggaacaa augaugaaua 360

uauugauguu cuugccaauu ugggauggua aaaugccccg uccagccauc cucaaaccca 420

aaccguugug gacaggaaaa cagauauuuu cccugaucau uccuggcaau guaaauauga 480

uacguaccca uucuacgc 498

<210> 79
<211> 114
<212> RNA
<213> Diabrotica virgifera

<400> 79
acccuccaca agaugaguau gaugggccac agagucaaag ucuuacccug gucgacguuc 60

cguaugaauc ucucgugcac cucucccuac aacgccgauu uugacggcga cgaa 114

<210> 80
<211> 106
<212> RNA
<213> Diabrotica virgifera

<400> 80
augccccguc cagccauccu caaacccaaa ccguugugga caggaaaaca gauauuuucc 60

cugaucauuc cuggcaaugu aaauaugaua cguacccauu cuacgc 106

<210> 81
<211> 401
<212> DNA
<213> Artificial Sequence

<220>
<223> DNA encoding Diabrotica rpI-2 v1 dsRNA

<400> 81
acccctccaca agatgagtat gatgggccac agagtcaaag tcttaccctg gtcgacgttc 60

cgtatgaatc tctcgtgcac ctctccctac aacgccgatt ttgacggcga cgaagaagct 120

agtaccagtc atcacgctgg agcgcacata taggccctcc atcagaaagt cattgtgtat 180

atctctcata gggaacgagc tgcttgcgta tttcccttcc gtagtcagag tcatcaatca 240

gctgcaccgt gtcgtaaagc gggacgttcg caagctcgtc cgcggtattc gtcgccgtca 300

aaatcggcgt tgtagggaga ggtgcacgag agattcatac ggaacgtcga ccagggtaag 360

actttgactc tgtggcccat catactcatc ttgtggaggg t 401

<210> 82
<211> 25
<212> DNA
<213> Artificial Sequence

<220>
<223> Probe RPI-2v1 PRB Set 1

<400> 82
aaagtcttac cctggtcgac gttcc 25

<210> 83
<211> 173
<212> DNA
<213> Artificial Sequence

<220>
<223> dsRNA loop polynucleotide

<400> 83
gaagctagta ccagtcatca cgctggagcg cacatatagg ccctccatca gaaagtcatt 60

gtgtatatct ctcataggga acgagctgct tgcgtatttc ccttccgtag tcagagtcat 120

caatcagctg caccgtgtcg taaagcggga cgttcgcaag ctcgtccgcg gta 173

<210> 84
<211> 401
<212> RNA
<213> Artificial Sequence

<220>
<223> rpI-2 dsRNA

<400> 84

```
acccuccaca agaugaguau gaugggccac agagucaaag ucuuacccug gucgacguuc    60
cguaugaauc ucucgugcac cucucccuac aacgccgauu uugacggcga cgaagaagcu   120
aguaccaguc aucacgcugg agcgcacaua uaggcccucc aucagaaagu cauuguguau   180
aucucucaua gggaacgagc ugcuugcgua uuucccuucc guagucagag ucaucaauca   240
gcugcaccgu gucguaaagc gggacguucg caagcucguc cgcgguauuc gucgccguca   300
aaaucggcgu uguagggaga ggugcacgag agauucauac ggaacgucga ccaggguaag   360
acuuugacuc uguggcccau cauacucauc uuguggaggg u                       401
```